(19) Europäisches Patentamt
European Patent Office
Office européen des brevets

(11) EP 0 456 063 B1

(12) **EUROPÄISCHE PATENTSCHRIFT**

(45) Veröffentlichungstag und Bekanntmachung des Hinweises auf die Patenterteilung:
**22.01.1997 Patentblatt 1997/04**

(21) Anmeldenummer: **91106870.8**

(22) Anmeldetag: **27.04.1991**

(51) Int Cl.$^6$: **C07D 207/38**, C07D 209/54, C07D 207/408, C07D 403/12, C07D 207/404, C07D 405/12, A01N 43/36

(54) **1-H-3-Aryl-pyrrolidin-2,4-dion-Derivate**

1-H-3-aryl-pyrrolidin-2,4-dion-derivatives

Dérivés de 1-H-3-aryl-pyrrolidine-2,4-dione

(84) Benannte Vertragsstaaten:
**BE CH DE ES FR GB GR IT LI NL**

(30) Priorität: **10.05.1990 DE 4014941**
**08.03.1991 DE 4107394**

(43) Veröffentlichungstag der Anmeldung:
**13.11.1991 Patentblatt 1991/46**

(73) Patentinhaber: **BAYER AG**
**51368 Leverkusen (DE)**

(72) Erfinder:
- **Krauskopf, Birgit, Dr.**
  **W-5060 Bergisch Gladbach 1 (DE)**
- **Lürssen, Klaus, Dr.**
  **W-5060 Bergisch Gladbach (DE)**
- **Santel, Hans-Joachim, Dr.**
  **W-5090 Leverkusen 1 (DE)**
- **Schmidt, Robert R., Dr.**
  **W-5060 Bergisch Gladbach (DE)**
- **Wachendorff-Neumann, Ulrike, Dr.**
  **W-4019 Monheim (DE)**
- **Fischer, Reiner, Dr.**
  **W-4019 Monheim 2 (DE)**
- **Erdelen, Christoph, Dr.**
  **W-5653 Leichlingen 1 (DE)**

(56) Entgegenhaltungen:
**EP-A- 355 599**
**EP-A- 521 334**
**EP-A- 0 377 893**
**EP-A- 0 415 185**
**EP-A- 0 423 482**
**WO-A-88/04652**
**DE-A- 2 361 084**
**US-A- 3 272 842**
**US-A- 4 632 698**

Bemerkungen:
Die Akte enthält technische Angaben, die nach dem Eingang der Anmeldung eingereicht wurden und die nicht in dieser Patentschrift enthalten sind.

EP 0 456 063 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue 3-Aryl-pyrrolidin-2,4-dion-Derivate, mehrere Verfahren zu ihrer Herstellung und ihre Verwendung als Insektizide, Akarizide und Herbizide.

Von 3-Acyl-pyrrolidin-2,4-dionen sind pharmazeutische Eigenschaften vorbeschrieben (S. Suzuki et. al. Chem. Pharm. Bull. 15 1120 (1967)). Weiterhin wurden N-Phenylpyrrolidin-2,4-dione von R. Schmierer und H. Mildenberger Liebigs Ann. Chem. 1985 1095 synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide. insektizide oder akarizide Wirkung bekannt geworden ist.

In DE-A 3 525 109 werden ähnlich strukturierte 1-H-3-Arylpyrrolidin-2,4-dione offenbart, die als Zwischenprodukte für Farbstoffsynthesen verwendet wurden.

Aus EP-A 0 355 599 sind 3-Aryl-pyrrolidin-2,4-dione mit ähnlicher Struktur bekannt, die herbizide, fungizide, insektizide und akarizide Eigenschaften aufweisen.

Die Wirksamkeit und die Wirkungsbreite dieser bekannten Verbindungen ist jedoch nicht immer zufriedenstellend.

Es wurden nun neue 3-Aryl-pyrrolidin-2,4-dion-Derivate gefunden, die durch die Formel (I) dargestellt sind,

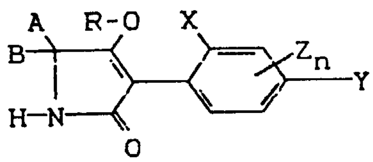

(I)

in welcher

X für $C_1$-$C_6$-Alkyl, Halogen, $C_1$-$C_6$-Alkoxy steht,

Y für Wasserstoff, $C_1$-$C_6$-Alkyl, Halogen, $C_1$-$C_6$-Alkoxy, $C_1$-$C_3$-Halogenalkyl steht,

Z für $C_1$-$C_6$-Alkyl, Halogen, $C_1$-$C_6$-Alkoxy steht,

n für eine Zahl von 0-3 steht,

R für Wasserstoff (Ia) oder für die Gruppen der Formel

| | | | | |
|---|---|---|---|---|
| $-CO-R^1$ | oder | $-CO-O-R^2$ | oder | $E^{\ominus}$ |
| (Ib) | | (Ic) | | (Id) |

steht, in welchen

$R^1$ für gegebenenfalls durch Halogen substituiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxy-$C_2$-$C_8$-alkyl und Cycloalkyl mit 3-8 Ringatomen, das durch Sauerstoff und/oder Schwefel unterbrochen sein kann, steht,
für gegebenenfalls durch Halogen, Nitro, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl;
für gegebenenfalls durch Halogen, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl-$C_1$-$C_6$-alkyl steht,
für gegebenenfalls durch Halogen und $C_1$-$C_6$-Alkyl substituiertes Phenoxy-$C_1$-$C_6$-alkyl steht,

$R^2$ für gegebenenfalls durch Halogen substituiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,
für gegebenenfalls durch Halogen, Nitro, $C_1$-$C_6$-Alkyl, $C_1$-$C_6$-Alkoxy, $C_1$-$C_6$-Halogenalkyl substituiertes Phenyl steht,

A für Wasserstoff oder gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann, steht,

B für Wasserstoff, geradkettiges oder verzweigtes $C_1$-$C_{12}$-Alkyl, $C_1$-$C_8$-Alkoxyalkyl steht,

oder worin

A und B gemeinsam mit dem Kohlenstoffatom, an das sie gebunden sind, einen 3 bis 8-gliedrigen Ring bilden,

$E^{\oplus}$ für ein Metallionenäquivalent oder ein Ammoniumion steht,
sowie die enantiomerenreinen Formen von Verbindungen der Formel (I).

Im folgenden seien die folgenden Untergruppen definiert:

(Ia): Verbindungen der Formel (I) worin R = Wasserstoff,
(Ib): Verbindungen der Formel (I) worin R = $COR^1$,
(Ic): Verbindungen der Formel (I) worin R = $COOR^2$.
(Id): Verbindungen der Formel (I) worin R = $E^{\oplus}$ für ein Metallionäquivalent oder ein Ammoniumion steht.

Weiterhin wurde gefunden, daß man 3-Aryl-pyrrolidin-2,4-dione bzw. deren Enole der Formel (Ia)

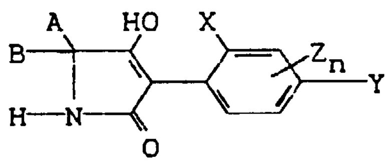

(Ia)

in welcher A, B, C, X, Y, Z und n die oben angegebene Bedeutung haben,
erhält, wenn man

(A)

N-Acylaminosäureester der Formel (II)

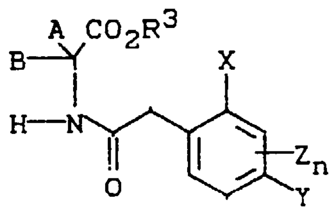

(II)

in welcher
A, B, X, Y, Z und n die oben angegebene Bedeutung haben
und

$R^3$ für Alkyl steht,

in Gegenwart eines Verdünnungsmittels und in Gegenwart einer Base intramolekular kondensiert.

(B)

Außerdem wurde gefunden, daß man Verbindungen der Formel (Ib)

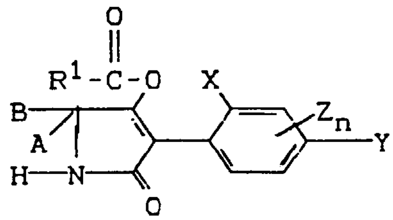

(Ib)

in welcher A, B, X, Y, Z, $R^1$ und n die oben angegebene Bedeutung haben, erhält, wenn man Verbindungen der Formel (Ia),

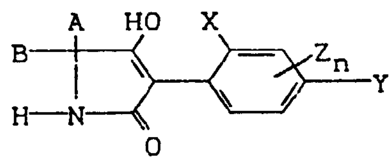

(Ia)

in welcher
A, B, X, Y, Z und n die oben angegebene Bedeutung haben,

α) mit Säurehalogeniden der allgemeinen Formel (III)

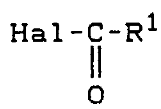

(III)

in welcher

$R^1$ die oben angegebene Bedeutung hat

und

Hal für Halogen, insbesondere Chlor und Brom steht,

gegebenenfalls in Gegenwart eines Verdünnungsmittels und gegebenenfalls in Gegenwart eines Säurebindemittels,
oder

β) mit Carbonsäureanhydriden der allgemeinen Formel (IV)

$$R^1\text{-CO-O-CO-}R^1 \qquad (IV)$$

in welcher

$R^1$ die oben angegebene Bedeutung hat,

gegebenenfalls in Gegenwart eines Verdünnungsmittels und gegebenenfalls in Geaenwart eines Säurebindemittels,

umsetzt.

(C)

Ferner wurde gefunden, daß man Verbindungen der Formel (Ic)

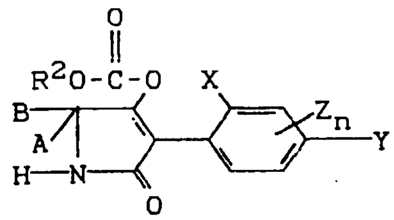

(Ic)

in welcher
A, B, C, X, Y, Z, $R^2$ und n die oben angegebene Bedeutung haben,
erhält, wenn man Verbindungen der Formel (Ia)

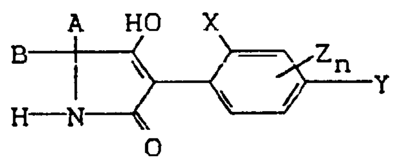

(Ia)

in welcher
A, B, X, Y, Z und n die oben angegebene Bedeutung haben,
mit Chlorameisensäureester der allgemeinen Formel (V)

$$R^2\text{-O-CO-Cl} \qquad (V)$$

in welcher

$R^2$ die oben angegebene Bedeutung hat,

gegebenenfals in Gegenwart eines Verdünnungsmittels und gegebenenfalls in Gegenwart eines Säurebindemittels umsetzt.

D)

Weiterhin wurde gefunden, daß man Verbindungen der Formel (I)

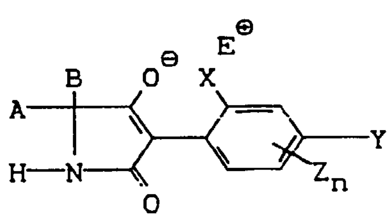

(Id)

in welcher X, Y, Z, A, B und n die oben angegebene Bedeutung haben,

erhält, wenn man Verbindungen der Formel (Ia)

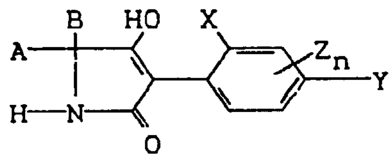

(Ia)

in welcher X, Y, Z, A, B und n die oben angegebene Bedeutung haben,
mit Metallhydroxiden oder Aminen der allgemeinen Formeln (VI) und (VII)

$Me_sOH_t$ (VI) 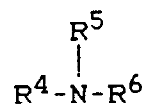 (VII)

in welchen

Me für ein- oder zweiwertige Metallionen,

s und t für die Zahl 1 und 2 und

$R^4$, $R^5$ und $R^6$ unabhängig voneinander für Wasserstoff und Alkyl

stehen,
gegebenenfalls in Gegenwart eines Verdünnungsmittels, umsetzt.

Überraschenderweise wurde gefunden, daß die neuen 3-Arylpyrrolidin-2,4-dion-Derivate der Formel (I) sich durch hervorragende insektizide, akarizide und herbizide Wirkungen auszeichnen.

Bevorzugt sind 3-Aryl-pyrrolidin-2,4-dion-Derivate der Formel (I), in welcher

X für $C_1$-$C_4$-Alkyl, Halogen, $C_1$-$C_4$-Alkoxy steht,

Y für Wasserstoff, $C_1$-$C_6$-Alkyl, Halogen, $C_1$-$C_4$-Alkoxy, $C_1$-$C_2$-Halogenalkyl steht,

Z für $C_1$-$C_4$-Alkyl, Halogen, $C_1$-$C_4$-Alkoxy steht,

n für eine Zahl von 0-3 steht,

R für Wasserstoff (Ia) oder für die Gruppen der Formel

$-CO-R^1$ oder $-CO-O-R^2$ oder $E^{\ominus}$
(Ib) (Ic) (Id)

steht, in welchen

$R^1$ für gegebenenfalls durch Halogen substituiertes $C_1$-$C_{16}$-Alkyl, $C_2$-$C_{16}$-Alkenyl, $C_1$-$C_6$-Alkoxy-$C_2$-$C_6$-alkyl, $C_1$-$C_6$-Alkylthio-$C_2$-$C_6$-alkyl, $C_1$-$C_6$-Polyalkoxy-$C_2$-$C_6$-alkyl und Cycloalkyl mit 3-7 Ringatomen, das durch 1-2 Sauerstoff- und/oder Schwefelatome unterbrochen sein kann steht,
für gegebenenfalls durch Halogen, Nitro, $C_1$-$C_4$-Alkyl, $C_1$-$C_4$-Alkoxy, $C_1$-$C_3$-Halogenalkyl, $C_1$-$C_3$-Halogenalkoxy substituiertes Phenyl steht,
für gegebenenfalls durch Halogen, $C_1$-$C_4$-Alkyl, $C_1$-$C_4$-Alkoxy, $C_1$-$C_3$-Halogenalkyl, $C_1$-$C_3$-Halogenalkoxy

substituiertes Phenyl-$C_1$-$C_4$-alkyl steht,
gegebenenfalls für durch Halogen und $C_1$-$C_4$-Alkyl substituiertes Phenoxy-$C_1$-$C_5$-alkyl steht,

$R^2$ für gegebenenfalls durch Halogen substituiertes $C_1$-$C_{16}$-Alkyl, $C_2$-$C_{16}$-Alkenyl, $C_1$-$C_{16}$-Alkoxy-$C_2$-$C_6$-alkyl, $C_1$-$C_6$-Polyalkoxy-$C_2$-$C_6$-alkyl steht,
für gegebenenfalls durch Halogen, Nitro, $C_1$-$C_4$-Alkyl, $C_1$-$C_3$-Alkoxy, $C_1$-$C_3$-Halogenalkyl substituiertes Phenyl steht,

A für Wasserstoff, gegebenenfalls durch Halogen substituiertes geradkettiges oder verzweigtes $C_1$-$C_{10}$-Alkyl, $C_3$-$C_6$-Alkenyl, $C_3$-$C_6$-Alkinyl, $C_1$-$C_8$-Alkoxy-$C_2$-$C_6$-alkyl, $C_1$-$C_6$-Polyalkoxy-$C_2$-$C_6$-alkyl, $C_1$-$C_8$-Alkylthio-$C_2$-$C_6$-alkyl, Cycloalkyl mit 3-7 Ringatomen, das durch 1-2 Sauerstoff- und/oder Schwefelatomen unterbrochen sein kann, steht,

B für Wasserstoff, geradkettiges oder verzweigtes $C_1$-$C_{10}$-Alkyl, $C_1$-$C_6$-Alkoxyalkyl steht,

oder worin

A und B gemeinsam mit dem Kohlenstoffatom, an das sie gebunden sind, einen 3 bis 7-gliedrigen Ring bilden,

$E^{\oplus}$ für ein Metallionenäquivalent oder ein Ammoniumion steht

sowie die enantiomerenreinen Formen von Verbindungen der Formel (I).
Besonders bevorzugt sind Verbindungen der Formel (I) in welcher

X für Methyl, Ethyl, Propyl, i-Propyl, Fluor, Chlor, Brom, Methoxy und Ethoxy steht,

Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,

Z für Methyl, Ethyl, i-Propyl, Butyl, i-Butyl, tert.-Butyl, Fluor, Chlor, Brom, Methoxy und Ethoxy steht,

n für eine Zahl von 0-3 steht,

R für Wasserstoff (Ia) oder für die Gruppen der Formel

$$-CO-R^1 \quad (Ib) \qquad \text{oder} \qquad -CO-O-R^2 \quad (Ic) \qquad \text{oder} \qquad E^{\ominus} \quad (Id)$$

steht, in welcher

$R^1$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Alkylthio-$C_2$-$C_6$-alkyl, $C_1$-$C_4$-Polyalkoxyl-$C_2$-$C_4$-alkyl und Cycloalkyl mit 3-6 Ringatomen, das durch 1-2 Sauerstoff- und/oder Schwefelatome unterbrochen sein kann steht,
für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-$C_1$-$C_3$-alkyl steht,
für gegebenenfalls durch Fluor, Chlor, Methyl, Ethyl substituiertes Phenoxy-$C_1$-$C_4$-alkyl steht,

$R^2$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Polyalkoxy-$C_2$-$C_6$-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl steht,

A für Wasserstoff, gegebenenfalls durch Halogen substituiertes geradkettiges oder verzweigtes $C_1$-$C_8$-Alkyl, $C_3$-

$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$-$C_4$-alkyl, Cycloalkyl mit 3-6 Ringatomen, das durch 1-2 Sauerstoff- und/oder Schwefelatomen unterbrochen sein kann, steht,

B für Wasserstoff, geradkettiges oder verzweigtes $C_1$-$C_8$-Alkyl, $C_1$-$C_4$-Alkoxyalkyl steht,

oder worin

A und B gemeinsam mit dem Kohlenstoffatom, an das sie gebunden sind, einen 3 bis 6-gliedrigen Ring bilden,

$E^{\oplus}$ für ein Metallionenäquivalent oder ein Ammoniumion steht

sowie die enantiomerenreinen Formen von Verbindungen der Formel I.

Verwendet man gemäß Verfahren (A) N-2,6-Dichlorphenylacetyl-N-methyl-alaninethylester, so kann der Verlauf des erfindungsgemäßen Verfahrens durch folgendes Reaktionsschema wiedergegeben werden:

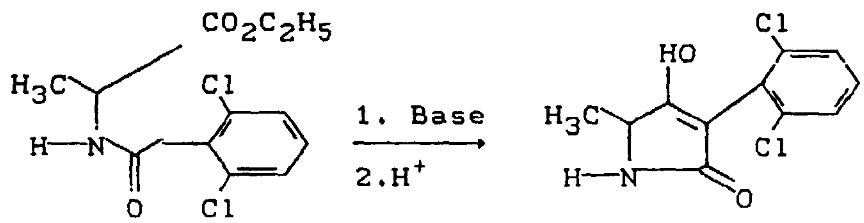


Verwendet man gemäß Verfahren (B) (Variante α) 3-(2,4,6-Trimethylphenyl)-3-isopropyl-pyrrolidin-2,4-dion und Pivaloylchlorid als Ausgangsstoff, so kann der Verlauf des erfindungsgemäßen Verfahrens durch folgendes Reaktionsschema wiedergegeben werden.

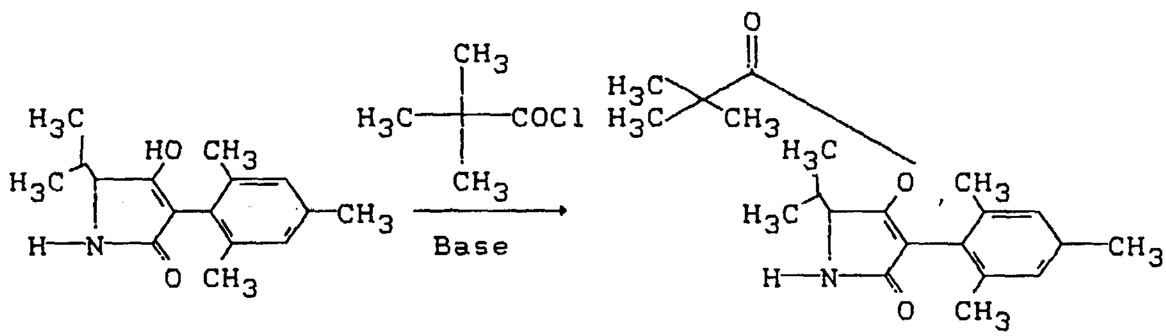


Verwendet man gemäß Verfahren B (Variante β) 3-(2,4,6-Trimethylphenyl)-5-cyclopentyl-pyrrolidin-2,4-dion und Acetanhydrid, so kann der Verlauf des erfindungsgemäßen Verfahrens durch folgendes Reaktionsschema wiedergegeben werden.

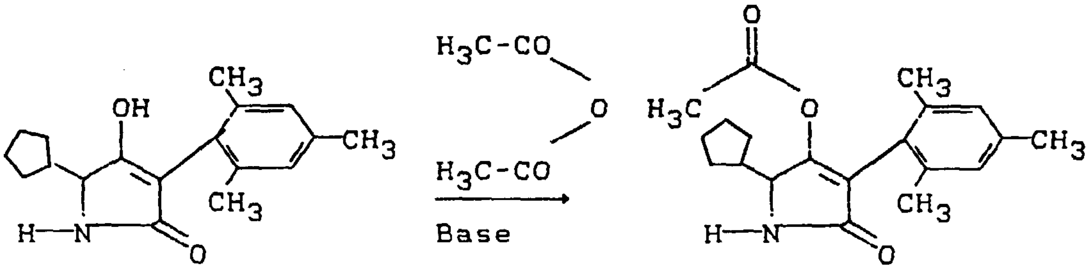

Verwendet man gemäß Verfahren C 3-(2,4-6-Trimethylphenyl)-5-phenyl-pyrrolidin-2,4-dion und Chlorameisensäureethoxyethylester, so kann der Verlauf des erfindungsgemäßen Verfahrens durch folgendes Reaktions schema wiedergegeben werden.

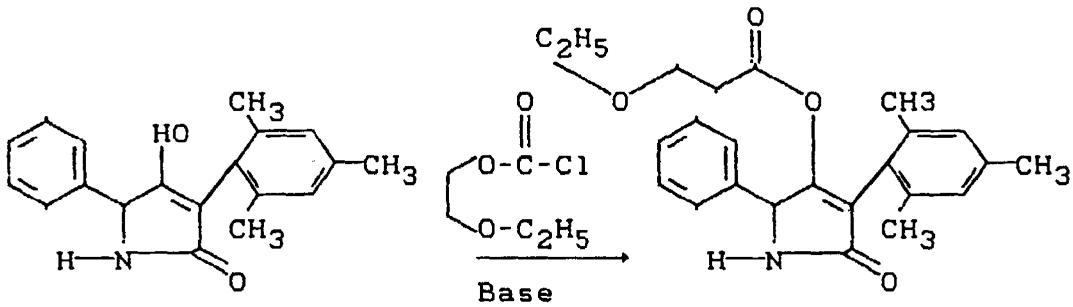


Verwendet man gemäß Verfahren D 3-(2,4-Dichlorphenyl)-5-methyl-pyrrolidin-2,4-dion und Methylamin, so kann der Verlauf des erfindungsgemäßen Verfahrens durch folgendes Reaktionsschema wiedergegeben werden.

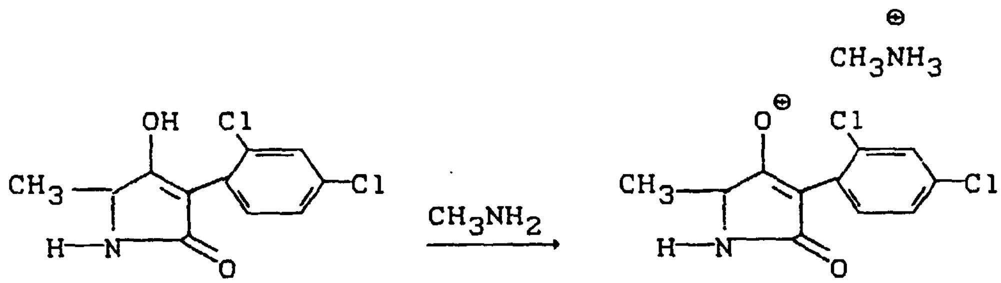


Die bei dem obigen Verfahren (A) als Ausgangsstoffe benötigten Verbindungen der Formel (II)

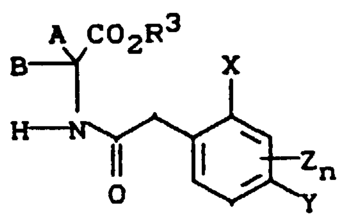

(II)

in welcher
A, B, X, Y, Z, n und $R^3$ die oben angegebene Bedeutung haben sind teilweise bekannt oder lassen sich nach im Prinzip bekannten Methoden in einfacher Weise herstellen. So erhalt man z.B. Acyl-aminosäureester der Formel (II), wenn man
a) Aminosaurederivate der Formel (VIII),

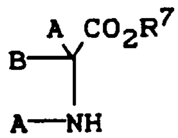

(VIII)

in welcher

$R^7$ für Wasserstoff (VIIIa) und Alkyl (VIIIb) steht

und

A die oben angegebene Bedeutung haben

mit Phenylessigsäurehalogeniden der Formel (IX)

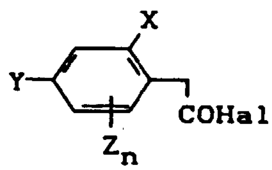

(IX)

in welcher
X, Y, Z und n die oben angegebene Bedeutung haben und

Hal für Chlor oder Brom steht,

acyliert (Chem. Reviews 52 237-416 (1953);
oder wenn man Acylaminosäuren der Formel (IIa),

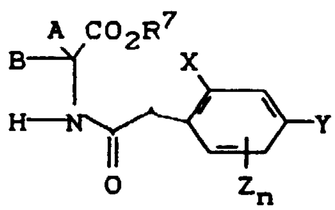

(IIa)

in welcher
A, B, X, Y, Z und n die oben angegebene Bedeutung haben
und

$R^7$ für Wasserstoff steht,

verestert (Chem. Ind. (London) 1568 (1968).

Beispielhaft seien folgende Verbindungen der Formel (II) genannt:

1. N-2,4-Dichlorphenyl-acetyl-glycinethylester
2. N-2,6-Dichlorphenyl-acetyl-glycinethylester
3. N-(2,6-Dichlorphenyl-acetyl)-alanin-ethylester
4. N-(2,6-Dichlorphenyl-acetyl)-valin-ethylester
5. N-(2,6-Dichlorphenyl-acetyl)-leucin-ethylester
6. N-(2,6-Dichlorphenyl-acetyl)-methionin-ethylester
7. N-(2,6-Dichlorphenyl-acetyl)-phenylalanin-ethylester
8. N-(2,6-Dichlorphenyl-acetyl)-tryptophan-ethylester
9. N-(2,6-Dichlorphenyl-acetyl)-isoleucin-ethylester
10. N-(2,4,6-Trimethylphenyl-acetyl)-glycin-methylester
11. N-(2,4,6-Trimethylphenyl-acetyl)-alanin-ethylester
12. N-(2,4,6-Trimethylphenyl-acetyl)-valin-ethylester
13. N-(2,4,6-Trimethylphenyl-acetyl)-leucin-ethylester
14. N-(2,4,6-Trimethylphenyl-acetyl)-isoleucin-ethylester
15. N-(2,4,6-Trimethylphenyl-acetyl)-methionin-ethylester

16. N-(2,4,6-Trimethylphenyl-acetyl)-phenylalanin-ethylester
17. N-(2,4,6-Trimethylphenyl-acetyl)-tryptophan-ethylester
18. N-(2,4,6-Trimethylphenyl-acetyl)-(4-chlorphenyl)-alanin-ethylester
19. N-(2,4,6-Trimethylphenyl-acetyl)-S-methyl-cystein-ethylester
20. N-(2,4,6-Trimethylphenyl-acetyl)-S-benzyl-cystein-ethylester
21. N-(2,4,6-Trimethylphenyl-acetyl)-O-methyl-threonin-ethylester
22. N-(2,4,6-Trimethylphenyl-acetyl)-tert.-butyl-alanin-ethylester
23. N-(2,4,6-Trimethylphenyl-acetyl)-histidin-ethylester
24. N-(2,4,6-Trimethylphenyl-acetyl)-O-methyl-tyrosin-ethylester
25. N-(2,4,6-Trimethylphenyl-acetyl)-1-amino-cyclopropan-carbonsäure-methylester
26. N-(2,4,6-Trimethylphenyl-acetyl)-1-amino-cyclopentan-carbonsäure-methylester
27. N-(2,4,6-Trimethylphenyl-acetyl)-1-amino-cyclohexan-carbonsäure-methylester
28. N-(2,4,6-Trimethylphenyl-acetyl)-amino-isobuttersäure-methylester
29. N-(2,4,6-Trimethylphenyl-acetyl)-2-ethyl-2-amino-buttersäure-methylester
30. N-(2,4,6-Trimethylphenyl-acetyl)-2-methyl-2-amino-buttersäure-methylester
31. N-(2,4,6-Trimethylphenyl-acetyl)-2-methyl-2-amino-valeriansäure-methylester
32. N-(2,4,6-Trimethylphenyl-acetyl)-2,3-dimethyl-2-amino-valeriansäure-methylester

Beispielhaft seien folgende Verbindungen der Formel (IIa) genannt:

1. N-2,4-Dichlorphenyl-acetyl-glycin
2. N-2,6-Dichlorphenyl-acetyl-glycin
3. N-(2,6-Dichlorphenyl-acetyl)-alanin
4. N-(2,6-Dichlorphenyl-acetyl)-valin
5. N-(2,6-Dichlorphenyl-acetyl)-leucin
6. N-(2,6-Dichlorphenyl-acetyl)-methionin
7. N-(2,6-Dichlorphenyl-acetyl)-phenylalanin
8. N-(2,6-Dichlorphenyl-acetyl)-tryptophan
9. N-(2,6-Dichlorphenyl-acetyl)-isoleucin
10. N-(2,4,6-Trimethylphenyl-acetyl-glycin
11. N-(2,4,6-Trimethylphenyl-acetyl)-alanin
12. N-(2,4,6-Trimethylphenyl-acetyl)-valin
13. N-(2,4,6-Trimethylphenyl-acetyl)-leucin
14. N-(2,4,6-Trimethylphenyl-acetyl)-isoleucin
15. N-(2,4,6-Trimethylphenyl-acetyl)-methionin
16. N-(2,4,6-Trimethylphenyl-acetyl)-phenylalanin
17. N-(2,4,6-Trimethylphenyl-acetyl)-tryptophan
18. N-(2,4,6-Trimethylphenyl-acetyl)-14-chlorphenyl)-alanin
19. N-(2,4,6-Trimethylphenyl-acetyl)-S-methyl-cystein
20. N-(2,4,6-Trimethylphenyl-acetyl)-S-benzyl-cystein
21. N-(2,4,6-Trimethylphenyl-acetyl)-O-methyl-threonin
22. N-(2,4,6-Trimethylphenyl-acetyl)-tert.-butyl-alanin
23. N-(2,4,6-Trimethylphenyl-acetyl)-histidin
24. N-(2,4,6-Trimethylphenyl-acetyl)-O-methyl-tyrosin
25. N-(2,4,6-Trimethylphenyl-acetyl)-1-amino-cyclopropancarbonsäure
26. N-(2,4,6-Trimethylphenyl-acetyl)-1-amino-cyclopentancarbonsäure
27. N-(2,4,6-Trimethylphenyl-acetyl)-1-amino-cyclohexancarbonsäure
tancarbonsäure
28. N-(2,4,6-Trimethylphenyl-acetyl)-1-amino-isobuttersäure
29. N-(2,4,6-Trimethylphenyl-acetyl)-2-ethyl-2-amino-buttersäure-methylester
30. N-(2,4,6-Trimethylphenyl-acetyl)-2-methyl-2-amino-buttersäure-methylester
31. N-(2,4,6-Trimethylphenyl-acetyl)-2-methyl-2-amino-valeriansäure-methylester
32. N-(2,4,6-Trimethylphenyl-acetyl)-2,3-dimethyl-2-amino-valeriansäure-methylester

Verbindungen der Formel (IIa) sind beispielsweise aus den Phenylessigsäurehalogeniden der Formel (IX) und Aminosäuren der Formel (VIIIa) nach Schotten-Baumann (Organikum 9. Auflage 446 (1970) VEB Deutscher Verlag der Wissenschaften, Berlin) erhältlich.

Verbindungen der Formel (VIIIa) und (VIIIb) sind bekannt oder aber nach im Prinzip bekannten Literaturverfahren

einfach herstellbar.

Das Verfahren (A) ist dadurch gekennzeichnet, daß Verbindungen der Formel (II) in welcher A, B, X, Y, Z, n und $R^3$ die oben angegebene Bedeutung haben in Gegenwart von Basen einer intramolekularen Kondensation unterwirft.

Als Verdünnungsmittel können bei dem erfindungsgemäßen Verfahren (A) alle üblichen inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glylkoldimethylether und Diglykoldimethylether, außerdem polare Lösungsmittel, wie Dimethylsulfoxid, Sulfolan, Dimethylformamid und N-Methyl-pyrrolidon.

Als Deprotonierungsmittel können bei der Durchführung des erfindungsgemäßen Verfahrens (A) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natriummethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Dimethylsulfoxid und Sulfolan. Wenn die Hydrolysestabilität des Saurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können auch bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäurean-

Adogen 464 = Methyltrialkyl($C_8$-$C_{10}$)ammoniumchlorid
TDA 1 = Tris-(methoxyethoxylethyl)-amin

hydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern der Formel (V) umsetzt.

Verwendet man die entsprechenden Chlorameisensäureester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBC, DBA, Hünig-Base und N,N-Dimethyl-anilin, ferner Erdalkalimetalloxide, wie Magnesium- und Calcium-oxid, außerdem Alkali- und Erdalkali-metall-carbonate, wie Natriumcarbonat, Kaliumcarbonat und Calciumcarbonat.

Als Verdünnungsmittel können bei dem erfindungsgemäßen Verfahren (C) bei Verwendung der Chlorameisensäureester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende (Chlorameisensäureester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Das Verfahren (D) ist dadurch gekennzeichnet, daß man Verbindungen der Formel (Ia) mit Metallhydroxiden (VI) oder Aminen (VII) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (D) wird im allgemeinen unter Normaldruck durchgeführt. Die Reaktionstemperatur liegen im allgemeinen zwischen -20°C und 100°C, vorzugsweise zwischen 0°C und 50°C.

Bei der Durchführung des erfindungsgemäßen Verfahrens (D) werden die Ausgangsstoffe der Formel (Ia) bzw. (VI= oder (VII) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man das Reaktionsgemisch durch Abziehen des Verdünnungsmittels einengt.

Herstellungsbeispiele

Beispiel 1

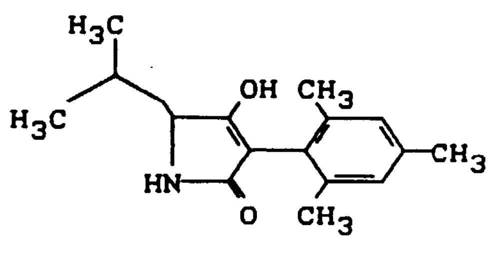

124,9 g (0,428 Mol) N-(2,4,6-Trimethylphenyl-acetyl)-valinmethylester werden in 430 ml abs. Toluol suspendiert. Nach Zugabe von 51,6 g Kalium-tert.-butylat (95 %ig) wird unter DC-Kontrolle unter Rückfluß erhitzt. Man rührt in 500 ml Eiswasser ein, trennt das Toluol ab und tropft die wäßrige Phase bei 0-20°C in 600 ml 1N HCl. Der Niederschlag wird abgesaugt, getrocknet und aus Chloroform/Methyl-tert.-butyl-Ether/n-Hexan umkristallisiert.

Ausbeute: 51,5 g (= 46,4 % d.Th.) der illustrierten Verbindung Fp. 126°C

Beispiel 2

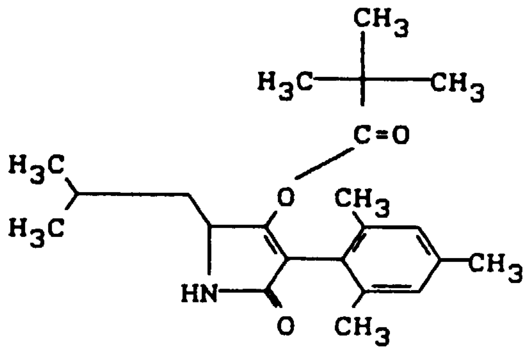

5,46 g (20 mmol) 5-Isobutyl-3-(2,4,6-Trimethylphenyl)-pyrrolidin-2,4-dion werden in 70 ml Methyl-tert.-Butyl-Ether suspendiert und mit 3,4 ml (20 mmol) Hünig-Base versetzt. Bei 0-10°C werden 2,52 ml (20 mmol) Pivaloylchlorid in 5 ml Methyl-tert.-butyl-Ether zugetropft und anschließend unter Dünnschichtchromatographie-Kontrolle weitergerührt. Der Niederschlag wird abgesaugt, nachgewaschen und das Filtrat einrotiert. Nach SC an Kieselgel mit Cyclohexan/ Essigester 1:1 und Kristallisation aus Methyl-tert.-butyl-Ether/n-Hexan erhielt man 2,14 g (29,9 % d.Th.) der illustrierten Verbindung vom Schmp. 154°C.

Beispiel 3

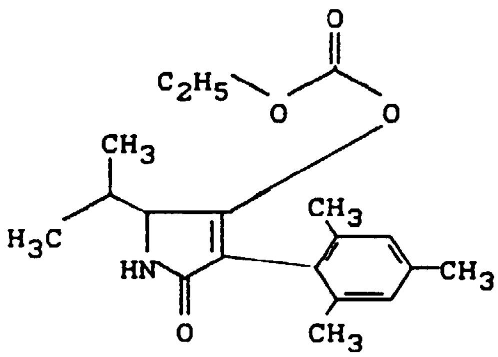

4,19 g (20 mmol) 5-Isopropyl-3-(2,4,6-trimethylphenyl)-pyrrolidin-2,4-dion werden in 70 ml Methyl-tert.-butyl-Ether suspendiert und mit 3,4 ml (20 mmol) HÜnig-Base versetzt. Bei -70°C tropft man 1,92 ml (20 mmol) Chlorameisensäureethylester in 5 ml Methyl-tert.-butyl-Ether zu und läßt auf Raumtemperatur erwärmen. Nach dem Einrotieren wird der Rückstand in Methylenchlorid aufgenommen, mit Wasser gewaschen, getrocknet und erneut einrotiert. Nach Kristallisation aus Methyl-tert.-butyl-Ether/n-Hexan erhält man 2,6 g (= 39,3 % d.Th.) der illustrierten Verbindung vom Schmp. 190°C.

Die folgenden Verbindungen der Tabellen 1, 2 und 3 können in Analogie zu den Beispielen 1, 2 bzw. 3 hergestellt werden.

Tabelle 1

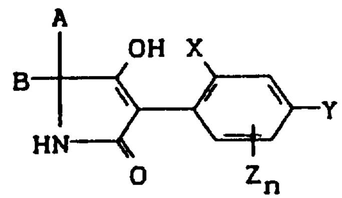

(Ia)

| Bsp.-Nr. | X | Y | $Z_n$ | A | B | Fp° C |
|---|---|---|---|---|---|---|
| 4 | Cl | Cl | H | H | H | |
| 5 | Cl | Cl | H | $CH_3$ | H | |
| 6 | Cl | Cl | H | $CH(CH_3)_2$ | H | |
| 7 | Cl | Cl | H | $CH_3$ | $CH_3$ | |
| 8 | Cl | Cl | H | $C_2H_5$ | $CH_3$ | |
| 9 | Cl | Cl | H | $C_2H_5$ | $C_2H_5$ | |
| 10 | Cl | Cl | H | $C_3H_7$ | $CH_3$ | |
| 11 | Cl | Cl | H | $i-C_3H_7$ | $CH_3$ | |
| 12 | Cl | Cl | H | $-(CH_2)_2-$ | | |
| 13 | Cl | Cl | H | $-(CH_2)_4-$ | | |
| 14 | Cl | Cl | H | $-(CH_2)_5-$ | | |
| 15 | Cl | Cl | H | $C_2H_5$ | H | |
| 16 | Cl | Cl | H | $C(CH_3)_3$ | H | |
| 17 | Cl | Cl | H | $CH_2CH(CH_3)_2$ | H | |

<u>Tabelle 1</u> (Fortsetzung)

| Bsp.-Nr. | X | Y | $Z_n$ | A | B | Fp° C |
|---|---|---|---|---|---|---|
| 18 | Cl | Cl | H | 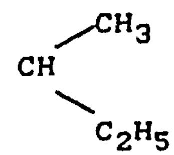 | H | |
| 19 | Cl | Cl | H | $CH_2-CH_2-S-CH_3$ | H | |
| 20 | Cl | Cl | H | $CH_2-S-CH_3$ | H | |
| 21 | Cl | Cl | H | $CH_2-S-CH_2-C_6H_5$ | H | |
| 22 | Cl | Cl | H | $CH_2-C_6H_5$ | | |
| 23 [1)] | Cl | Cl | H | 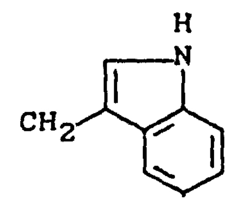 | H | |
| 24 [1)] | Cl | Cl | H | 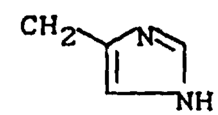 | H | |

**Tabelle 1** (Fortsetzung)

| Bsp.-Nr. | X | Y | $Z_n$ | A | B | Fp° C |
|---|---|---|---|---|---|---|
| 25 | Cl | H | 6-Cl | H | H | |
| 26 | Cl | H | 6-Cl | $CH_3$ | H | |
| 27 | Cl | H | 6-Cl | $CH(CH_3)_2$ | H | |
| 28 | $CH_3$ | $CH_3$ | H | H | H | |
| 29 | $CH_3$ | $CH_3$ | H | $CH_3$ | H | |
| 30 | $CH_3$ | $CH_3$ | H | $CH(CH_3)_2$ | H | |
| 31 | $CH_3$ | $CH_3$ | 6-$CH_3$ | H | H | |
| 32 | $CH_3$ | $CH_3$ | 6-$CH_3$ | $CH_3$ | H | > 230 |
| 33 | $CH_3$ | $CH_3$ | 6-$CH_3$ | $CH_3$ | $CH_3$ | 223 |
| 34 | $CH_3$ | $CH_3$ | 6-$CH_3$ | $C_2H_5$ | $CH_3$ | |
| 35 | $CH_3$ | $CH_3$ | 6-$CH_3$ | $C_2H_5$ | $C_2H_5$ | |
| 36 | $CH_3$ | $CH_3$ | 6-$CH_3$ | $C_3H_7$ | $CH_3$ | |
| 37 | $CH_3$ | $CH_3$ | 6-$CH_3$ | i-$C_3H_7$ | $CH_3$ | |
| 38 | $CH_3$ | $CH_3$ | 6-$CH_3$ | $-(CH_2)_2-$ | | 225 |
| 39 | $CH_3$ | $CH_3$ | 6-$CH_3$ | $-(CH_2)_4-$ | | |
| 40 | $CH_3$ | $CH_3$ | 6-$CH_3$ | $-(CH_2)_5-$ | | |

**Tabelle 1** (Fortsetzung)

| Bsp.-Nr. | X | Y | $Z_n$ | A | B | Fp° C |
|---|---|---|---|---|---|---|
| 41 | $CH_3$ | $CH_3$ | 6-$CH_3$ | $C_2H_5$ | H | |
| 42 | $CH_3$ | $CH_3$ | 6-$CH_3$ | $C(CH_3)_3$ | H | |
| 43 | $CH_3$ | $CH_3$ | 6-$CH_3$ | $CH_2CH(CH_3)_2$ | H | > 220 |
| 44 | $CH_3$ | $CH_3$ | 6-$CH_3$ | 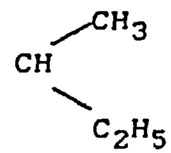 | H | |
| 45 | $CH_3$ | $CH_3$ | 6-$CH_3$ | $CH_2-CH_2-S-CH_3$ | H | |
| 46 | $CH_3$ | $CH_3$ | 6-$CH_3$ | $CH_2-S-CH_3$ | H | |
| 47 | $CH_3$ | $CH_3$ | 6-$CH_3$ | $CH_2-S-CH_2-C_6H_5$ | H | |
| 48 | $CH_3$ | $CH_3$ | 6-$CH_3$ | $CH_2-C_6H_5$ | | |
| 49[1)] | $CH_3$ | $CH_3$ | 6-$CH_3$ | $CH_2$ 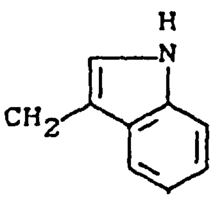 | H | |
| 50[1)] | $CH_3$ | $CH_3$ | 6-$CH_3$ | $CH_2$ 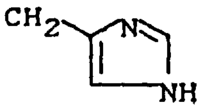 | H | |

**Tabelle 2**

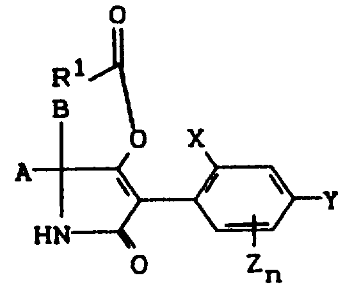

(Ib)

| Bsp.-Nr. | X | Y | $Z_n$ | A | B | $R^1$ | Fp° C |
|---|---|---|---|---|---|---|---|
| 51 | Cl | Cl | H | H | H | $CH_3$ | |
| 52 | Cl | Cl | H | $CH_3$ | H | $CH_3$ | |
| 53 | Cl | Cl | H | $CH_3$ | H | $C(CH_3)_3$ | |
| 54 | Cl | Cl | H | $CH_3$ | $CH_3$ | $CH_3$ | |
| 55 | Cl | Cl | H | $CH_3$ | $CH_3$ | $(CH_3)_2CH-$ | |
| 56 | Cl | Cl | H | $CH_3$ | $CH_3$ | $(CH_3)_3C-$ | |
| 57 | Cl | Cl | H | $CH_3$ | $CH_3$ | $CH_3-(CH_2)_3-$ | |
| 58 | Cl | Cl | H | $CH_3$ | $CH_3$ | $C_2H_5-C(CH_3)_2-$ | |
| 59 | Cl | Cl | H | $CH_3$ | $CH_3$ | $(CH_3)_3C-CH_2-$ | |
| 60 | Cl | Cl | H | $CH_3$ | $CH_3$ | $(CH_3)_2CH-C(CH_3)_2-$ | |
| 61 | Cl | Cl | H | $CH_3$ | $CH_3$ | $CH_2=CH-(CH_2)_8-$ | |

Tabelle 2 (Fortsetzung)

| Bsp.-Nr. | X | Y | $Z_n$ | A | B | $R^1$ | Fp° C |
|---|---|---|---|---|---|---|---|
| 62 | Cl | Cl | H | $CH_3$ | $CH_3$ | 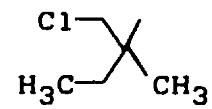 | |
| 63 | Cl | Cl | H | $CH_3$ | $CH_3$ | $C_4H_9-CH-C_2H_5$ | |
| 64 | Cl | Cl | H | $CH_3$ | $CH_3$ | 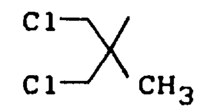 | |
| 65 | Cl | Cl | H | $CH_3$ | $CH_3$ | 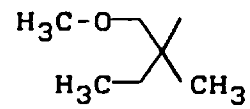 | |
| 66 | Cl | Cl | H | $CH_3$ | $CH_3$ | 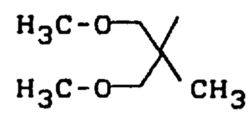 | |
| 67 | Cl | Cl | H | $CH_3$ | $CH_3$ | 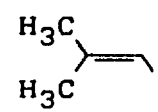 | |

Tabelle 2 (Fortsetzung)

| Bsp.-Nr. | X | Y | $Z_n$ | A | B | $R^1$ | Fp° C |
|---|---|---|---|---|---|---|---|
| 68 | Cl | Cl | H | $CH_3$ | $CH_3$ | $H_3C-S-CH_2-$ | |
| 69[1] | Cl | Cl | H | $CH_3$ | $CH_3$ | 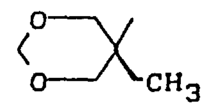 | |
| 70[1] | Cl | Cl | H | $CH_3$ | $CH_3$ | 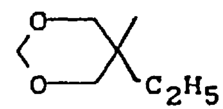 | |
| 71 | Cl | Cl | H | $CH_3$ | $CH_3$ | 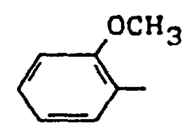 | |
| 72 | Cl | Cl | H | $CH_3$ | $CH_3$ | 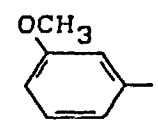 | |
| 73 | Cl | Cl | H | $CH_3$ | $CH_3$ | 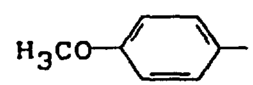 | |

**Tabelle 2** (Fortsetzung)

| Bsp.-Nr. | X | Y | $Z_n$ | A | B | $R^1$ | Fp° C |
|---|---|---|---|---|---|---|---|
| 74 | Cl | Cl | H | $CH_3$ | $CH_3$ | 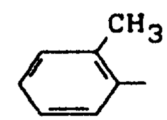 | |
| 75 | Cl | Cl | H | $CH_3$ | $CH_3$ | 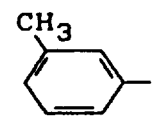 | |
| 76 | Cl | Cl | H | $CH_3$ | $CH_3$ | 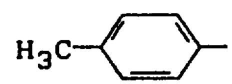 | |
| 77 | Cl | Cl | H | $CH_3$ | $CH_3$ | 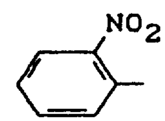 | |
| 78 | Cl | Cl | H | $CH_3$ | $CH_3$ | 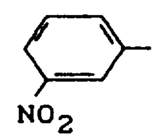 | |

Tabelle 2 (Fortsetzung)

| Bsp.-Nr. | X | Y | $Z_n$ | A | B | $R^1$ | Fp° C |
|---|---|---|---|---|---|---|---|
| 79 | Cl | Cl | H | $CH_3$ | $CH_3$ | 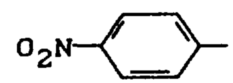 | |
| 80 | Cl | Cl | H | $CH_3$ | $CH_3$ | 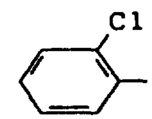 | |
| 81 | Cl | Cl | H | $CH_3$ | $CH_3$ | 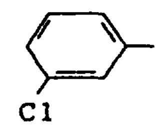 | |
| 82 | Cl | Cl | H | $CH_3$ | $CH_3$ | 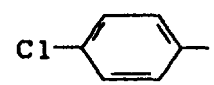 | |
| 83 | Cl | Cl | H | $CH_3$ | $CH_3$ | 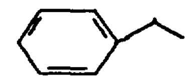 | |

**Tabelle 2** (Fortsetzung)

| Bsp.-Nr. | X | Y | $Z_n$ | A | B | $R^1$ | Fp° C |
|---|---|---|---|---|---|---|---|
| 84 | Cl | Cl | H | $C_2H_5$ | $CH_3$ | $CH_3$ | |
| 85 | Cl | Cl | H | $C_2H_5$ | $CH_3$ | $(CH_3)_2CH-$ | |
| 86 | Cl | Cl | H | $C_2H_5$ | $CH_3$ | $(CH_3)_3C-$ | |
| 87 | Cl | Cl | H | $C_2H_5$ | $CH_3$ | $CH_3-(CH_2)_3-$ | |
| 88 | Cl | Cl | H | $C_2H_5$ | $CH_3$ | $C_2H_5-C(CH_3)_2$ | |
| 89 | Cl | Cl | H | $C_2H_5$ | $CH_3$ | $(CH_3)_3C-CH_2-$ | |
| 90 | Cl | Cl | H | $C_2H_5$ | $CH_3$ | $(CH_3)_2CH-C(CH_3)_2$ | |
| 91 | Cl | Cl | H | $C_2H_5$ | $CH_3$ | $CH_2=CH-(CH_2)_8-$ | |
| 92 | Cl | Cl | H | $C_2H_5$ | $CH_3$ | 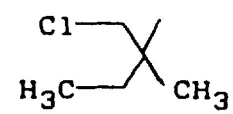 | |
| 93 | Cl | Cl | H | $C_2H_5$ | $CH_3$ | $C_4H_9-CH-C_2H_5$ | |

**Tabelle 2** (Fortsetzung)

| Bsp.-Nr. | X | Y | $Z_n$ | A | B | $R^1$ | Fp° C |
|---|---|---|---|---|---|---|---|
| 94 | Cl | Cl | H | $C_2H_5$ | $CH_3$ | 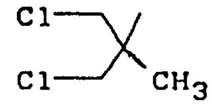 | |
| 95 | Cl | Cl | H | $C_2H_5$ | $CH_3$ | 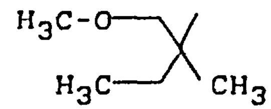 | |
| 96 | Cl | Cl | H | $C_2H_5$ | $CH_3$ | 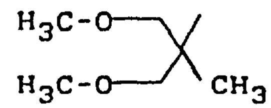 | |
| 97 | Cl | Cl | H | $C_2H_5$ | $CH_3$ | 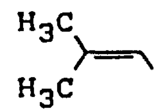 | |
| 98 | Cl | Cl | H | $C_2H_5$ | $CH_3$ | $H_3C-S-CH_2-$ | |
| 99 [1)] | Cl | Cl | H | $C_2H_5$ | $CH_3$ | 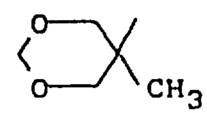 | |
| 100 [1)] | Cl | Cl | H | $C_2H_5$ | $CH_3$ | 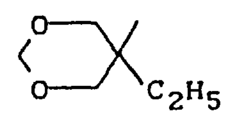 | |
| 101 | Cl | Cl | H | $C_2H_5$ | $CH_3$ | 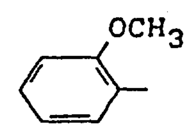 | |

**Tabelle 2** (Fortsetzung)

| Bsp.-Nr. | X | Y | $Z_n$ | A | B | $R^1$ | Fp° C |
|---|---|---|---|---|---|---|---|
| 102 | Cl | Cl | H | $C_2H_5$ | $CH_3$ | 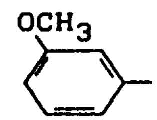 | |
| 103 | Cl | Cl | H | $C_2H_5$ | $CH_3$ | 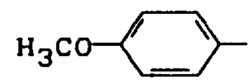 | |
| 104 | Cl | Cl | H | $C_2H_5$ | $CH_3$ | 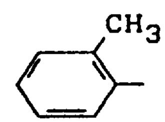 | |
| 105 | Cl | Cl | H | $C_2H_5$ | $CH_3$ | 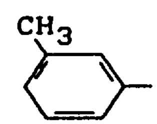 | |
| 106 | Cl | Cl | H | $C_2H_5$ | $CH_3$ | 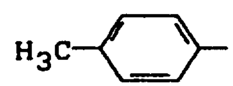 | |
| 107 | Cl | Cl | H | $C_2H_5$ | $CH_3$ | 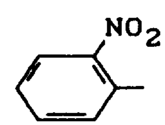 | |

<u>Tabelle 2</u> (Fortsetzung)

| Bsp.-Nr. | X | Y | $Z_n$ | A | B | $R^1$ | Fp° C |
|---|---|---|---|---|---|---|---|
| 108 | Cl | Cl | H | $C_2H_5$ | $CH_3$ | 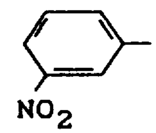 | |
| 109 | Cl | Cl | H | $C_2H_5$ | $CH_3$ | 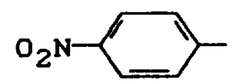 | |
| 110 | Cl | Cl | H | $C_2H_5$ | $CH_3$ | 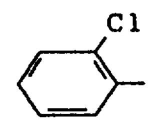 | |
| 111 | Cl | Cl | H | $C_2H_5$ | $CH_3$ | 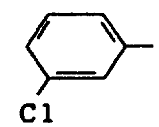 | |
| 112 | Cl | Cl | H | $C_2H_5$ | $CH_3$ | 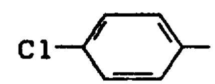 | |
| 113 | Cl | Cl | H | $C_2H_5$ | $CH_3$ | 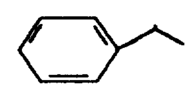 | |

Tabelle 2 (Fortsetzung)

| Bsp.-Nr. | X | Y | $Z_n$ | A | B | $R^1$ | Fp° C |
|---|---|---|---|---|---|---|---|
| 114 | Cl | Cl | H | $C_2H_5$ | $C_2H_5$ | $CH_3$ | |
| 115 | Cl | Cl | H | $C_2H_5$ | $C_2H_5$ | $(CH_3)_2CH-$ | |
| 116 | Cl | Cl | H | $C_2H_5$ | $C_2H_5$ | $(CH_3)_3C-$ | |
| 117 | Cl | Cl | H | $C_2H_5$ | $C_2H_5$ | $CH_3-(CH_2)_3-$ | |
| 118 | Cl | Cl | H | $C_2H_5$ | $C_2H_5$ | $C_2H_5-C(CH_3)_2$ | |
| 119 | Cl | Cl | H | $C_2H_5$ | $C_2H_5$ | $(CH_3)_3C-CH_2-$ | |
| 120 | Cl | Cl | H | $C_2H_5$ | $C_2H_5$ | $(CH_3)_2CH-C(CH_3)_2$ | |
| 121 | Cl | Cl | H | $C_2H_5$ | $C_2H_5$ | $CH_2=CH-(CH_2)_8-$ | |
| 122 | Cl | Cl | H | $C_2H_5$ | $C_2H_5$ | 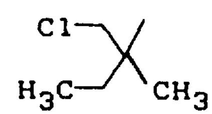 | |
| 123 | Cl | Cl | H | $C_2H_5$ | $C_2H_5$ | $C_4H_9-CH-C_2H_5$ | |

Tabelle 2 (Fortsetzung)

| Bsp.-Nr. | X | Y | $Z_n$ | A | B | $R^1$ | Fp° C |
|---|---|---|---|---|---|---|---|
| 124 | Cl | Cl | H | $C_2H_5$ | $C_2H_5$ | 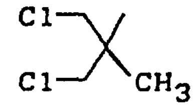 | |
| 125 | Cl | Cl | H | $C_2H_5$ | $C_2H_5$ | 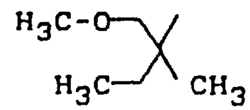 | |
| 126 | Cl | Cl | H | $C_2H_5$ | $C_2H_5$ | 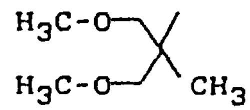 | |
| 127 | Cl | Cl | H | $C_2H_5$ | $C_2H_5$ | 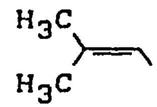 | |
| 128 | Cl | Cl | H | $C_2H_5$ | $C_2H_5$ | $H_3C-S-CH_2-$ | |
| 129 1) | Cl | Cl | H | $C_2H_5$ | $C_2H_5$ | 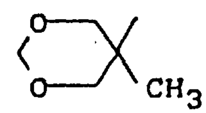 | |
| 130 1) | Cl | Cl | H | $C_2H_5$ | $C_2H_5$ | 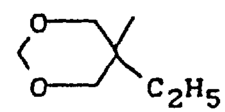 | |

**Tabelle 2** (Fortsetzung)

| Bsp.-Nr. | X | Y | $Z_n$ | A | B | $R^1$ | Fp° C |
|---|---|---|---|---|---|---|---|
| 131 | Cl | Cl | H | $C_2H_5$ | $C_2H_5$ | 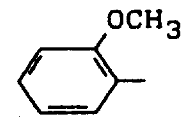 | |
| 132 | Cl | Cl | H | $C_2H_5$ | $C_2H_5$ | 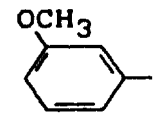 | |
| 133 | Cl | Cl | H | $C_2H_5$ | $C_2H_5$ | 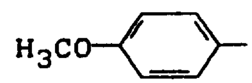 | |
| 134 | Cl | Cl | H | $C_2H_5$ | $C_2H_5$ | 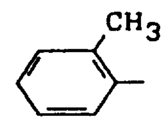 | |
| 135 | Cl | Cl | H | $C_2H_5$ | $C_2H_5$ | 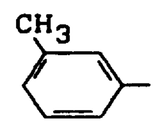 | |
| 136 | Cl | Cl | H | $C_2H_5$ | $C_2H_5$ | 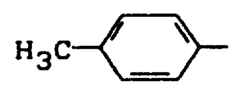 | |

Tabelle 2 (Fortsetzung)

| Bsp.-Nr. | X | Y | $Z_n$ | A | B | $R^1$ | Fp° C |
|---|---|---|---|---|---|---|---|
| 137 | Cl | Cl | H | $C_2H_5$ | $C_2H_5$ | 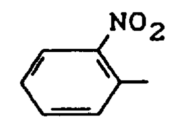 | |
| 138 | Cl | Cl | H | $C_2H_5$ | $C_2H_5$ | 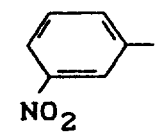 | |
| 139 | Cl | Cl | H | $C_2H_5$ | $C_2H_5$ | 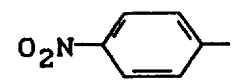 | |
| 140 | Cl | Cl | H | $C_2H_5$ | $C_2H_5$ | 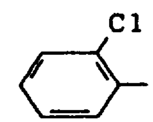 | |
| 141 | Cl | Cl | H | $C_2H_5$ | $C_2H_5$ | 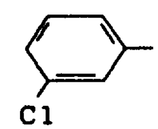 | |
| 142 | Cl | Cl | H | $C_2H_5$ | $C_2H_5$ | 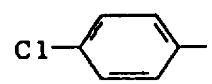 | |
| 143 | Cl | Cl | H | $C_2H_5$ | $C_2H_5$ | 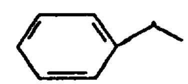 | |

**Tabelle 2** (Fortsetzung)

| Bsp.-Nr. | X | Y | $Z_n$ | A | B | $R^1$ | Fp° C |
|---|---|---|---|---|---|---|---|
| 144 | Cl | Cl | H | $C_3H_7$ | $CH_3$ | $CH_3$ | |
| 145 | Cl | Cl | H | $C_3H_7$ | $CH_3$ | $(CH_3)_2CH-$ | |
| 146 | Cl | Cl | H | $C_3H_7$ | $CH_3$ | $(CH_3)_3C-$ | |
| 147 | Cl | Cl | H | $C_3H_7$ | $CH_3$ | $CH_3-(CH_2)_3-$ | |
| 148 | Cl | Cl | H | $C_3H_7$ | $CH_3$ | $C_2H_5-C(CH_3)_2$ | |
| 149 | Cl | Cl | H | $C_3H_7$ | $CH_3$ | $(CH_3)_3C-CH_2-$ | |
| 150 | Cl | Cl | H | $C_3H_7$ | $CH_3$ | $(CH_3)_2CH-C(CH_3)_2$ | |
| 151 | Cl | Cl | H | $C_3H_7$ | $CH_3$ | $CH_2=CH-(CH_2)_8-$ | |
| 152 | Cl | Cl | H | $C_3H_7$ | $CH_3$ | 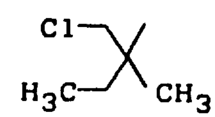 | |
| 153 | Cl | Cl | H | $C_3H_7$ | $CH_3$ | $C_4H_9-CH-C_2H_5$ | |

Tabelle 2 (Fortsetzung)

| Bsp.-Nr. | X | Y | $Z_n$ | A | B | $R^1$ | Fp° C |
|---|---|---|---|---|---|---|---|
| 154 | Cl | Cl | H | $C_3H_7$ | $CH_3$ | 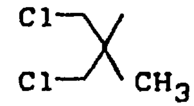 | |
| 155 | Cl | Cl | H | $C_3H_7$ | $CH_3$ | 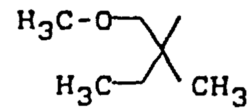 | |
| 156 | Cl | Cl | H | $C_3H_7$ | $CH_3$ | 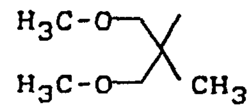 | |
| 157 | Cl | Cl | H | $C_3H_7$ | $CH_3$ | 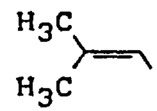 | |
| 158 | Cl | Cl | H | $C_3H_7$ | $CH_3$ | $H_3C-S-CH_2-$ | |
| 159[1)] | Cl | Cl | H | $C_3H_7$ | $CH_3$ | 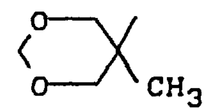 | |
| 160[1)] | Cl | Cl | H | $C_3H_7$ | $CH_3$ | 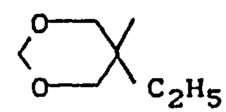 | |
| 161 | Cl | Cl | H | $C_3H_7$ | $CH_3$ | 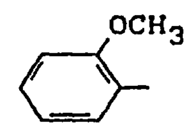 | |

<u>Tabelle 2</u> (Fortsetzung)

| Bsp.-Nr. | X | Y | $Z_n$ | A | B | $R^1$ | Fp° C |
|---|---|---|---|---|---|---|---|
| 162 | Cl | Cl | H | $C_3H_7$ | $CH_3$ | 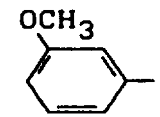 | |
| 163 | Cl | Cl | H | $C_3H_7$ | $CH_3$ | 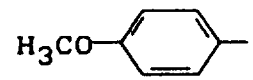 | |
| 164 | Cl | Cl | H | $C_3H_7$ | $CH_3$ | 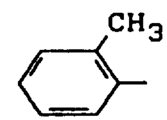 | |
| 165 | Cl | Cl | H | $C_3H_7$ | $CH_3$ | 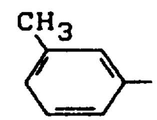 | |
| 166 | Cl | Cl | H | $C_3H_7$ | $CH_3$ | 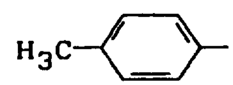 | |
| 167 | Cl | Cl | H | $C_3H_7$ | $CH_3$ | 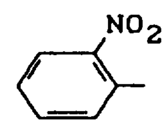 | |
| 168 | Cl | Cl | H | $C_3H_7$ | $CH_3$ | 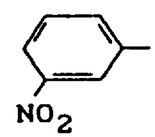 | |

Tabelle 2 (Fortsetzung)

| Bsp.-Nr. | X | Y | $Z_n$ | A | B | $R^1$ | Fp° C |
|---|---|---|---|---|---|---|---|
| 169 | Cl | Cl | H | $C_3H_7$ | $CH_3$ | 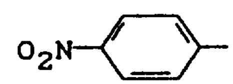 | |
| 170 | Cl | Cl | H | $C_3H_7$ | $CH_3$ | 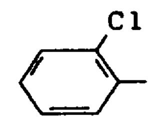 | |
| 171 | Cl | Cl | H | $C_3H_7$ | $CH_3$ | 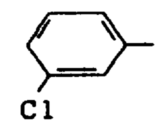 | |
| 172 | Cl | Cl | H | $C_3H_7$ | $CH_3$ | 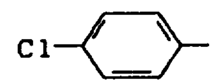 | |
| 173 | Cl | Cl | H | $C_3H_7$ | $CH_3$ | 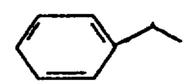 | |
| 174 | Cl | Cl | H | i-$C_3H_7$ | $CH_3$ | $CH_3$ | |
| 175 | Cl | Cl | H | i-$C_3H_7$ | $CH_3$ | $(CH_3)_2CH-$ | |
| 176 | Cl | Cl | H | i-$C_3H_7$ | $CH_3$ | $(CH_3)_3C-$ | |
| 177 | Cl | Cl | H | i-$C_3H_7$ | $CH_3$ | $CH_3-(CH_2)_3-$ | |
| 178 | Cl | Cl | H | i-$C_3H_7$ | $CH_3$ | $C_2H_5-C(CH_3)_2$ | |

<u>Tabelle 2</u> (Fortsetzung)

| Bsp.-Nr. | X | Y | $Z_n$ | A | B | $R^1$ | Fp° C |
|---|---|---|---|---|---|---|---|
| 179 | Cl | Cl | H | i-$C_3H_7$ | $CH_3$ | $(CH_3)_3C-CH_2-$ | |
| 180 | Cl | Cl | H | i-$C_3H_7$ | $CH_3$ | $(CH_3)_2CH-C(CH_3)_2$ | |
| 181 | Cl | Cl | H | i-$C_3H_7$ | $CH_3$ | $CH_2=CH-(CH_2)_8-$ | |
| 182 | Cl | Cl | H | i-$C_3H_7$ | $CH_3$ | 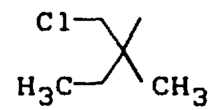 | |
| 183 | Cl | Cl | H | i-$C_3H_7$ | $CH_3$ | $C_4H_9-CH-C_2H_5$ | |
| 184 | Cl | Cl | H | i-$C_3H_7$ | $CH_3$ | 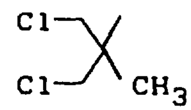 | |
| 185 | Cl | Cl | H | i-$C_3H_7$ | $CH_3$ | 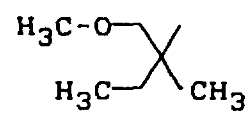 | |
| 186 | Cl | Cl | H | i-$C_3H_7$ | $CH_3$ | 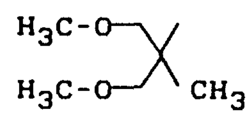 | |
| 187 | Cl | Cl | H | i-$C_3H_7$ | $CH_3$ | 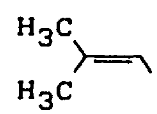 | |

<u>Tabelle 2</u> (Fortsetzung)

| Bsp.-Nr. | X | Y | $Z_n$ | A | B | $R^1$ | Fp° C |
|---|---|---|---|---|---|---|---|
| 188 | Cl | Cl | H | $i-C_3H_7$ | $CH_3$ | $H_3C-S-CH_2-$ | |
| 189[1)] | Cl | Cl | H | $i-C_3H_7$ | $CH_3$ | 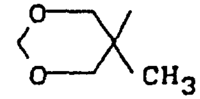 | |
| 190[1)] | Cl | Cl | H | $i-C_3H_7$ | $CH_3$ | 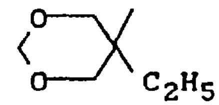 | |
| 191 | Cl | Cl | H | $i-C_3H_7$ | $CH_3$ | 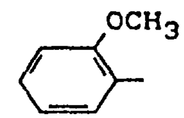 | |
| 192 | Cl | Cl | H | $i-C_3H_7$ | $CH_3$ | 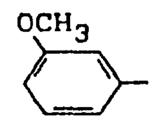 | |
| 193 | Cl | Cl | H | $i-C_3H_7$ | $CH_3$ | 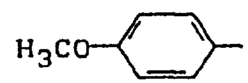 | |
| 194 | Cl | Cl | H | $i-C_3H_7$ | $CH_3$ | 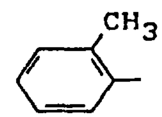 | |
| 195 | Cl | Cl | H | $i-C_3H_7$ | $CH_3$ | 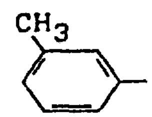 | |

**Tabelle 2** (Fortsetzung)

| Bsp.-Nr. | X | Y | $Z_n$ | A | B | $R^1$ | Fp° C |
|---|---|---|---|---|---|---|---|
| 196 | Cl | Cl | H | $i-C_3H_7$ | $CH_3$ | 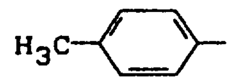 | |
| 197 | Cl | Cl | H | $i-C_3H_7$ | $CH_3$ | 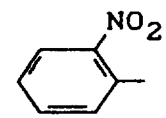 | |
| 198 | Cl | Cl | H | $i-C_3H_7$ | $CH_3$ | 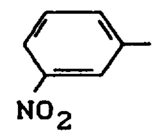 | |
| 199 | Cl | Cl | H | $i-C_3H_7$ | $CH_3$ | 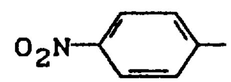 | |
| 200 | Cl | Cl | H | $i-C_3H_7$ | $CH_3$ | 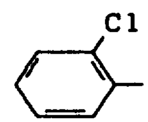 | |
| 201 | Cl | Cl | H | $i-C_3H_7$ | $CH_3$ | 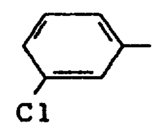 | |
| 202 | Cl | Cl | H | $i-C_3H_7$ | $CH_3$ | 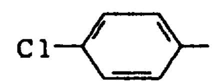 | |

Tabelle 2 (Fortsetzung)

| Bsp.-Nr. | X | Y | $Z_n$ | A | B | $R^1$ | Fp° C |
|---|---|---|---|---|---|---|---|
| 203 | Cl | Cl | H | $i-C_3H_7$ | $CH_3$ | 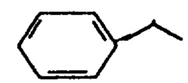 | |
| 204 | Cl | Cl | H | $-(CH_2)_4-$ | | $CH_3$ | |
| 205 | Cl | Cl | H | $-(CH_2)_4-$ | | $(CH_3)_2CH-$ | |
| 206 | Cl | Cl | H | $-(CH_2)_4-$ | | $(CH_3)_3C-$ | |
| 207 | Cl | Cl | H | $-(CH_2)_4-$ | | $CH_3-(CH_2)_3-$ | |
| 208 | Cl | Cl | H | $-(CH_2)_4-$ | | $C_2H_5-C(CH_3)_2$ | |
| 209 | Cl | Cl | H | $-(CH_2)_4-$ | | $(CH_3)_3C-CH_2-$ | |
| 210 | Cl | Cl | H | $-(CH_2)_4-$ | | $(CH_3)_2CH-C(CH_3)_2$ | |
| 211 | Cl | Cl | H | $-(CH_2)_4-$ | | $CH_2=CH-(CH_2)_8-$ | |
| 212 | Cl | Cl | H | $-(CH_2)_4-$ | | 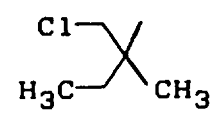 | |
| 213 | Cl | Cl | H | $-(CH_2)_4-$ | | $C_4H_9-CH-C_2H_5$ | |

Tabelle 2 (Fortsetzung)

| Bsp.-Nr. | X | Y | $Z_n$ | A | B | $R^1$ | Fp° C |
|---|---|---|---|---|---|---|---|
| 214 | Cl | Cl | H | $-(CH_2)_4-$ | | 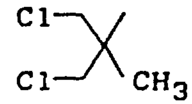 | |
| 215 | Cl | Cl | H | $-(CH_2)_4-$ | | 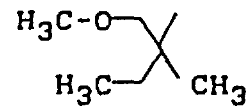 | |
| 216 | Cl | Cl | H | $-(CH_2)_4-$ | | 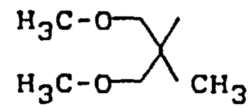 | |
| 217 | Cl | Cl | H | $-(CH_2)_4-$ | | 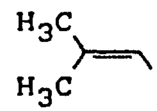 | |
| 218 | Cl | Cl | H | $-(CH_2)_4-$ | | $H_3C-S-CH_2-$ | |
| 219[1] | Cl | Cl | H | $-(CH_2)_4-$ | | 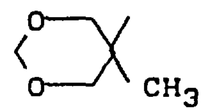 | |
| 220[1] | Cl | Cl | H | $-(CH_2)_4-$ | | 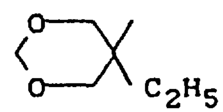 | |

Tabelle 2 (Fortsetzung)

| Bsp.-Nr. | X | Y | $Z_n$ | A | B | $R^1$ | Fp° C |
|---|---|---|---|---|---|---|---|
| 221 | Cl | Cl | H | $-(CH_2)_4-$ | | 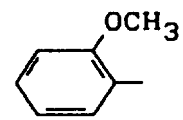 | |
| 222 | Cl | Cl | H | $-(CH_2)_4-$ | | 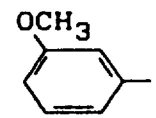 | |
| 223 | Cl | Cl | H | $-(CH_2)_4-$ | | 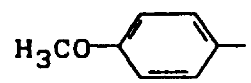 | |
| 224 | Cl | Cl | H | $-(CH_2)_4-$ | | 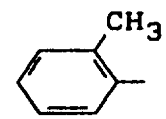 | |
| 225 | Cl | Cl | H | $-(CH_2)_4-$ | | 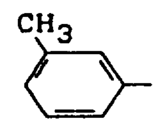 | |
| 226 | Cl | Cl | H | $-(CH_2)_4-$ | | 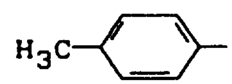 | |

**Tabelle 2** (Fortsetzung)

| Bsp.-Nr. | X | Y | $Z_n$ | A | B | $R^1$ | Fp° C |
|---|---|---|---|---|---|---|---|
| 227 | Cl | Cl | H | $-(CH_2)_4-$ | | 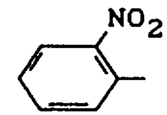 | |
| 228 | Cl | Cl | H | $-(CH_2)_4-$ | | 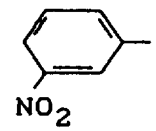 | |
| 229 | Cl | Cl | H | $-(CH_2)_4-$ | | 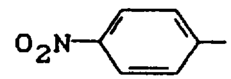 | |
| 230 | Cl | Cl | H | $-(CH_2)_4-$ | | 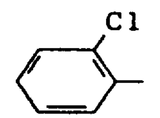 | |
| 231 | Cl | Cl | H | $-(CH_2)_4-$ | | 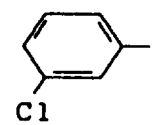 | |
| 232 | Cl | Cl | H | $-(CH_2)_4-$ | | 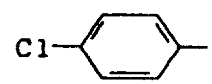 | |
| 233 | Cl | Cl | H | $-(CH_2)_4-$ | | 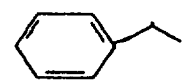 | |

**Tabelle 2** (Fortsetzung)

| Bsp.-Nr. | X | Y | $Z_n$ | A | B | $R^1$ | Fp° C |
|---|---|---|---|---|---|---|---|
| 234 | Cl | Cl | H | $-(CH_2)_5-$ | | $CH_3$ | |
| 235 | Cl | Cl | H | $-(CH_2)_5-$ | | $(CH_3)_2CH-$ | |
| 236 | Cl | Cl | H | $-(CH_2)_5-$ | | $(CH_3)_3C-$ | |
| 237 | Cl | Cl | H | $-(CH_2)_5-$ | | $CH_3-(CH_2)_3-$ | |
| 238 | Cl | Cl | H | $-(CH_2)_5-$ | | $C_2H_5-C(CH_3)_2$ | |
| 239 | Cl | Cl | H | $-(CH_2)_5-$ | | $(CH_3)_3C-CH_2-$ | |
| 240 | Cl | Cl | H | $-(CH_2)_5-$ | | $(CH_3)_2CH-C(CH_3)_2$ | |
| 241 | Cl | Cl | H | $-(CH_2)_5-$ | | $CH_2=CH-(CH_2)_8-$ | |
| 242 | Cl | Cl | H | $-(CH_2)_5-$ | | 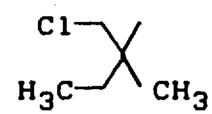 | |
| 243 | Cl | Cl | H | $-(CH_2)_5-$ | | $C_4H_9-CH-C_2H_5$ | |
| 244 | Cl | Cl | H | $-(CH_2)_5-$ | | 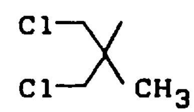 | |

Tabelle 2 (Fortsetzung)

| Bsp.-Nr. | X | Y | $Z_n$ | A | B | $R^1$ | Fp° C |
|---|---|---|---|---|---|---|---|
| 245 | Cl | Cl | H | $-(CH_2)_5-$ | | 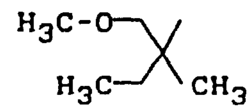 | |
| 246 | Cl | Cl | H | $-(CH_2)_5-$ | | 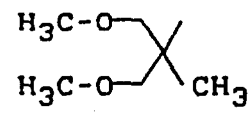 | |
| 247 | Cl | Cl | H | $-(CH_2)_5-$ | | 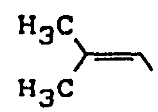 | |
| 248 | Cl | Cl | H | $-(CH_2)_5-$ | | $H_3C-S-CH_2-$ | |
| 249 1) | Cl | Cl | H | $-(C_2H_2)_5$ | | 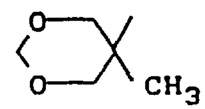 | |
| 250 1) | Cl | Cl | H | $-(CH_2)_5-$ | | 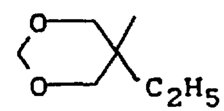 | |
| 251 | Cl | Cl | H | $-(CH_2)_5-$ | | 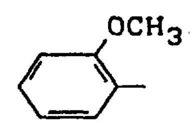 | |

<u>Tabelle 2</u> (Fortsetzung)

| Bsp.-Nr. | X | Y | $Z_n$ | A | B | $R^1$ | Fp° C |
|---|---|---|---|---|---|---|---|
| 252 | Cl | Cl | H | $-(CH_2)_5-$ | | 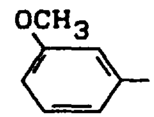 | |
| 253 | Cl | Cl | H | $-(CH_2)_5-$ | | 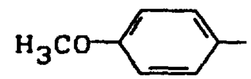 | |
| 254 | Cl | Cl | H | $-(CH_2)_5-$ | | 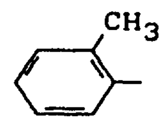 | |
| 255 | Cl | Cl | H | $-(CH_2)_5-$ | | 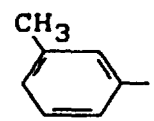 | |
| 256 | Cl | Cl | H | $-(CH_2)_5-$ | | 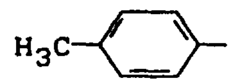 | |
| 257 | Cl | Cl | H | $-(CH_2)_5-$ | | 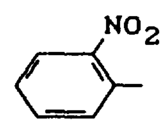 | |
| 258 | Cl | Cl | H | $-(CH_2)_5-$ | | 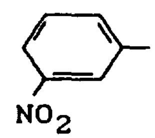 | |

<u>Tabelle 2</u> (Fortsetzung)

| Bsp.-Nr. | X | Y | $Z_n$ | A | B | $R^1$ | Fp° C |
|---|---|---|---|---|---|---|---|
| 259 | Cl | Cl | H | $-(CH_2)_5-$ | | 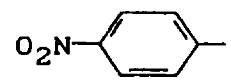 | |
| 260 | Cl | Cl | H | $-(CH_2)_5-$ | | 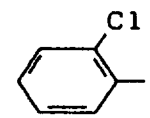 | |
| 261 | Cl | Cl | H | $-(CH_2)_5-$ | | 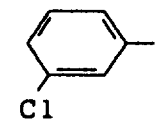 | |
| 262 | Cl | Cl | H | $-(CH_2)_5-$ | | 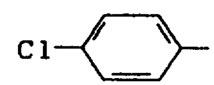 | |
| 263 | Cl | Cl | H | $-(CH_2)_5-$ | | 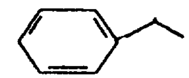 | |
| 264 | Cl | H | 6-Cl | H | H | $CH_3$ | |
| 265 | Cl | H | 6-Cl | H | H | $C(CH_3)_3$ | |
| 266 | Cl | H | 6-Cl | $CH_3$ | H | $CH_3$ | |
| 267 | Cl | H | 6-Cl | $CH_3$ | H | $C(CH_3)_3$ | |
| 268 | $CH_3$ | $CH_3$ | H | H | H | $CH_3$ | |
| 269 | $CH_3$ | $CH_3$ | H | H | H | $C(CH_3)_3$ | |

Tabelle 2 (Fortsetzung)

| Bsp.-Nr. | X | Y | $Z_n$ | A | B | $R^1$ | Fp° C |
|---|---|---|---|---|---|---|---|
| 270 | $CH_3$ | $CH_3$ | H | $CH_3$ | H | $CH_3$ | |
| 271 | $CH_3$ | $CH_3$ | H | $CH_3$ | H | $C(CH_3)_3$ | |
| 272 | $CH_3$ | $CH_3$ | 6-$CH_3$ | H | H | $CH_3$ | |
| 273 | $CH_3$ | $CH_3$ | 6-$CH_3$ | H | H | $CH(CH_3)_2$ | |
| 274 | $CH_3$ | $CH_3$ | 6-$CH_3$ | H | H | $C(CH_3)_3$ | |
| 275 | $CH_3$ | $CH_3$ | 6-$CH_3$ | H | H | $C(CH_3)_2CH_2Cl$ | |
| 276 | $CH_3$ | $CH_3$ | 6-$CH_3$ | H | H | $C(CH_3)_2CH_2$-O-$CH_3$ | |
| 277 | $CH_3$ | $CH_3$ | 6-$CH_3$ | H | H | $CH_2$-S-$CH_3$ | |
| 278 1) | $CH_3$ | $CH_3$ | 6-$CH_3$ | H | H | 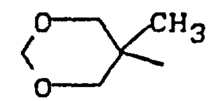 | |
| 279 | $CH_3$ | $CH_3$ | 6-$CH_3$ | H | H | 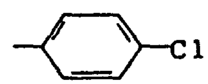 | |
| 280 | $CH_3$ | $CH_3$ | 6-$CH_3$ | H | H | 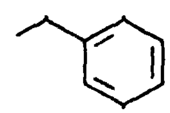 | |
| 281 | $CH_3$ | $CH_3$ | 6-$CH_3$ | $CH_3$ | H | $CH_3$ | 132 |
| 282 | $CH_3$ | $CH_3$ | 6-$CH_3$ | $CH_3$ | H | $CH(CH_3)_2$ | |
| 283 | $CH_3$ | $CH_3$ | 6-$CH_3$ | $CH_3$ | H | $C(CH_3)_3$ | 152 |

<u>Tabelle 2</u> (Fortsetzung)

| Bsp.-Nr. | X | Y | $Z_n$ | A | B | $R^1$ | Fp° C |
|---|---|---|---|---|---|---|---|
| 284 | $CH_3$ | $CH_3$ | 6-$CH_3$ | $CH_3$ | H | $C(CH_3)_2CH_2Cl$ | |
| 285 | $CH_3$ | $CH_3$ | 6-$CH_3$ | $CH_3$ | H | $C(CH_3)_2CH_2$-O-$CH_3$ | |
| 286 | $CH_3$ | $CH_3$ | 6-$CH_3$ | $CH_3$ | H | $CH_2$-S-$CH_3$ | |
| 287 1) | $CH_3$ | $CH_3$ | 6-$CH_3$ | $CH_3$ | H | 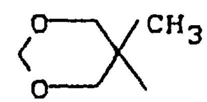 | |
| 288 | $CH_3$ | $CH_3$ | 6-$CH_3$ | $CH_3$ | H | 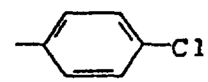 | |
| 289 | $CH_3$ | $CH_3$ | 6-$CH_3$ | $CH_3$ | H | 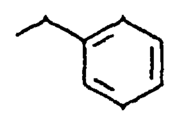 | |
| 290 | $CH_3$ | $CH_3$ | 6-$CH_3$ | $CH(CH_3)_2$ | H | $CH_3$ | 188 |
| 291 | $CH_3$ | $CH_3$ | 6-$CH_3$ | $CH(CH_3)_2$ | H | $CH(CH_3)_2$ | |
| 292 | $CH_3$ | $CH_3$ | 6-$CH_3$ | $CH(CH_3)_2$ | H | $C(CH_3)_3$ | 213 |
| 293 | $CH_3$ | $CH_3$ | 6-$CH_3$ | $CH(CH_3)_2$ | H | $C(CH_3)_2CH_2Cl$ | |
| 294 | $CH_3$ | $CH_3$ | 6-$CH_3$ | $CH(CH_3)_2$ | H | $C(CH_3)_2CH_2$-O-$CH_3$ | |
| 295 | $CH_3$ | $CH_3$ | 6-$CH_3$ | $CH(CH_3)_2$ | H | $CH_2$-S-$CH_3$ | |

Tabelle 2 (Fortsetzung)

| Bsp.-Nr. | X | Y | $Z_n$ | A | B | $R^1$ | Fp° |
|---|---|---|---|---|---|---|---|
| 296 | $CH_3$ | $CH_3$ | $6-CH_3$ | $CH(CH_3)_2$ | H | 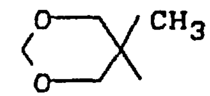 | |
| 297 | $CH_3$ | $CH_3$ | $6-CH_3$ | $CH(CH_3)_2$ | H | 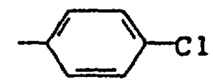 | |
| 298 | $CH_3$ | $CH_3$ | $6-CH_3$ | $CH(CH_3)_2$ | H | 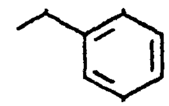 | |
| 299 | $CH_3$ | $CH_3$ | $6-CH_3$ | $CH_2CH(CH_3)_2$ | H | $CH_3$ | 169 |
| 300 | $CH_3$ | $CH_3$ | $6-CH_3$ | $CH_2CH(CH_3)_2$ | H | $C_2H_5$ | |
| 301 | $CH_3$ | $CH_3$ | $6-CH_3$ | $CH_2CH(CH_3)_2$ | H | $CH(CH_3)_2$ | |
| 302 | $CH_3$ | $CH_3$ | $6-CH_3$ | $CH_2CH(CH_3)_2$ | H | $C(CH_3)_2CH_2Cl$ | |
| 303 | $CH_3$ | $CH_3$ | $6-CH_3$ | $CH_2CH(CH_3)_2$ | H | $C(CH_3)_2CH_2-O-CH_3$ | |
| 304 | $CH_3$ | $CH_3$ | $6-CH_3$ | $CH_2CH(CH_3)_2$ | H | $CH_2-S-CH_3$ | |
| 305 1) | $CH_3$ | $CH_3$ | $6-CH_3$ | $CH_2CH(CH_3)_2$ | H | 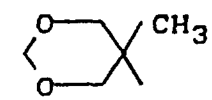 | |
| 306 | $CH_3$ | $CH_3$ | $6-CH_3$ | $CH_2CH(CH_3)_2$ | H | 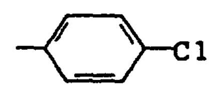 | |

Tabelle 2 (Fortsetzung)

| Bsp.-Nr. | X | Y | $Z_n$ | A | B | $R^1$ | Fp° |
|---|---|---|---|---|---|---|---|
| 307 | $CH_3$ | $CH_3$ | $6-CH_3$ | $CH_2CH(CH_3)_2$ | H | 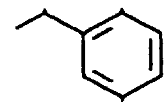 | |
| 308 | $CH_3$ | $CH_3$ | $6-CH_3$ | 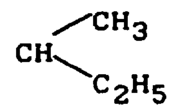 | H | $CH_3$ | 184 |
| 309 | $CH_3$ | $CH_3$ | $6-CH_3$ | 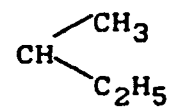 | H | $CH(CH_3)_2$ | |
| 310 | $CH_3$ | $CH_3$ | $6-CH_3$ | 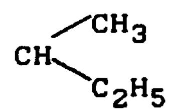 | H | $C(CH_3)_3$ | |
| 311 | $CH_3$ | $CH_3$ | $6-CH_3$ | 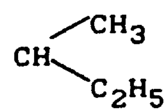 | H | $C(CH_3)_2CH_2Cl$ | |
| 312 | $CH_3$ | $CH_3$ | $6-CH_3$ | 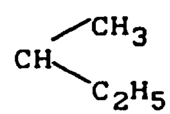 | H | $C(CH_3)_2CH_2-O-CH_3$ | |
| 313 | $CH_3$ | $CH_3$ | $6-CH_3$ | 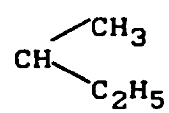 | H | $CH_2-S-CH_3$ | |

**Tabelle 2** (Fortsetzung)

| Bsp.-Nr. | X | Y | $Z_n$ | A | B | $R^1$ | Fp° |
|---|---|---|---|---|---|---|---|
| 314[1] | $CH_3$ | $CH_3$ | $6-CH_3$ | 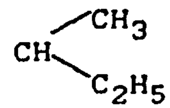 | H | 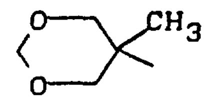 | |
| 315 | $CH_3$ | $CH_3$ | $6-CH_3$ | 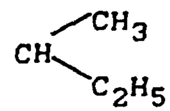 | H | 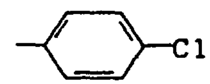 | |
| 316 | $CH_3$ | $CH_3$ | $6-CH_3$ | 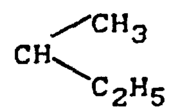 | H | 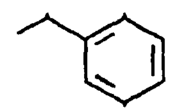 | |
| 317 | $CH_3$ | $CH_3$ | $6-CH_3$ | $-(CH_2)_2SCH_3$ | H | $CH_3$ | |
| 318 | $CH_3$ | $CH_3$ | $6-CH_3$ | $-(CH_2)_2SCH_3$ | H | $CH(CH_3)_2$ | |
| 319 | $CH_3$ | $CH_3$ | $6-CH_3$ | $-(CH_2)_2SCH_3$ | H | $C(CH_3)_2$ | |
| 320 | $CH_3$ | $CH_3$ | $6-CH_3$ | $-(CH_2)_2SCH_3$ | H | $C(CH_3)_2CH_2Cl$ | |
| 321 | $CH_3$ | $CH_3$ | $6-CH_3$ | $-(CH_2)_2SCH_3$ | H | $C(CH_3)_2CH_2-O-CH_3$ | |
| 322 | $CH_3$ | $CH_3$ | $6-CH_3$ | $-(CH_2)_2SCH_3$ | H | $CH_2-S-CH_3$ | |

**Tabelle 2** (Fortsetzung)

| Bsp.-Nr. | X | Y | $Z_n$ | A | B | $R^1$ | Fp° |
|---|---|---|---|---|---|---|---|
| 323[1] | $CH_3$ | $CH_3$ | 6-$CH_3$ | $-(CH_2)_2SCH_3$ | H | 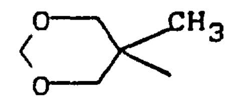 | |
| 324 | $CH_3$ | $CH_3$ | 6-$CH_3$ | $-(CH_2)_2SCH_3$ | H | 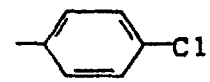 | |
| 325 | $CH_3$ | $CH_3$ | 6-$CH_3$ | $-(CH_2)_2SCH_3$ | H | 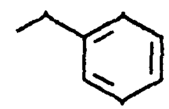 | |
| 326 | $CH_3$ | $CH_3$ | 6-$CH_3$ | $-(CH_2)_2-$ | | $CH_3$ | 94 |
| 327 | $CH_3$ | $CH_3$ | 6-$CH_3$ | $-(CH_2)_2-$ | | $-C(CH_3)_3$ | 95 |
| 328 | $CH_3$ | $CH_3$ | 6-$CH_3$ | $CH_3$ | $CH_3$ | $CH_3$ | 216 |
| 329 | $CH_3$ | $CH_3$ | 6-$CH_3$ | $CH_3$ | $CH_3$ | $(CH_3)_2CH-$ | |
| 330 | $CH_3$ | $CH_3$ | 6-$CH_3$ | $CH_3$ | $CH_3$ | $(CH_3)_3C-$ | $> 230$ |
| 331 | $CH_3$ | $CH_3$ | 6-$CH_3$ | $CH_3$ | $CH_3$ | $CH_3-(CH_2)_3-$ | |
| 332 | $CH_3$ | $CH_3$ | 6-$CH_3$ | $CH_3$ | $CH_3$ | $C_2H_5-C(CH_3)_2$ | 183 |
| 333 | $CH_3$ | $CH_3$ | 6-$CH_3$ | $CH_3$ | $CH_3$ | $(CH_3)_3C-CH_2-$ | 175 |
| 334 | $CH_3$ | $CH_3$ | 6-$CH_3$ | $CH_3$ | $CH_3$ | $(CH_3)_2CH-C(CH_3)_2$ | |

**Tabelle 2** (Fortsetzung)

| Bsp.-Nr. | X | Y | $Z_n$ | A | B | $R^1$ | Fp° |
|---|---|---|---|---|---|---|---|
| 335 | $CH_3$ | $CH_3$ | 6-$CH_3$ | $CH_3$ | $CH_3$ | $CH_2=CH-(CH_2)_8-$ | |
| 336 | $CH_3$ | $CH_3$ | 6-$CH_3$ | $CH_3$ | $CH_3$ | 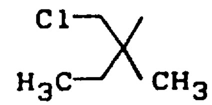 | |
| 337 | $CH_3$ | $CH_3$ | 6-$CH_3$ | $CH_3$ | $CH_3$ | $C_4H_9-CH-C_2H_5$ | |
| 338 | $CH_3$ | $CH_3$ | 6-$CH_3$ | $CH_3$ | $CH_3$ | 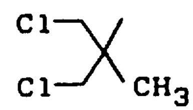 | |
| 339 | $CH_3$ | $CH_3$ | 6-$CH_3$ | $CH_3$ | $CH_3$ | 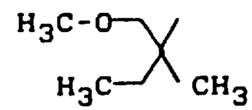 | |
| 340 | $CH_3$ | $CH_3$ | 6-$CH_3$ | $CH_3$ | $CH_3$ | 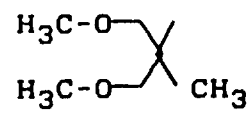 | |
| 341 | $CH_3$ | $CH_3$ | 6-$CH_3$ | $CH_3$ | $CH_3$ | 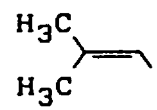 | |
| 342 | $CH_3$ | $CH_3$ | 6-$CH_3$ | $CH_3$ | $CH_3$ | $H_3C-S-CH_2-$ | |

Tabelle 2 (Fortsetzung)

| Bsp.-Nr. | X | Y | $Z_n$ | A | B | $R^1$ | Fp° |
|---|---|---|---|---|---|---|---|
| 343 [1)] | $CH_3$ | $CH_3$ | 6-$CH_3$ | $CH_3$ | $CH_3$ | 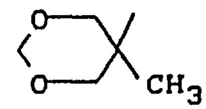 | |
| 344 [1)] | $CH_3$ | $CH_3$ | 6-$CH_3$ | $CH_3$ | $CH_3$ | 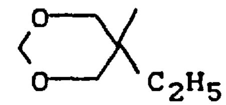 | |
| 345 | $CH_3$ | $CH_3$ | 6-$CH_3$ | $CH_3$ | $CH_3$ | 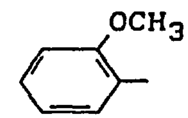 | |
| 346 | $CH_3$ | $CH_3$ | 6-$CH_3$ | $CH_3$ | $CH_3$ | 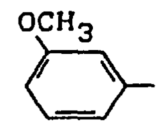 | |
| 347 | $CH_3$ | $CH_3$ | 6-$CH_3$ | $CH_3$ | $CH_3$ | 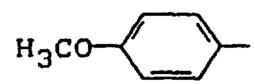 | |
| 348 | $CH_3$ | $CH_3$ | 6-$CH_3$ | $CH_3$ | $CH_3$ | 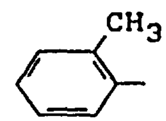 | |
| 349 | $CH_3$ | $CH_3$ | 6-$CH_3$ | $CH_3$ | $CH_3$ | 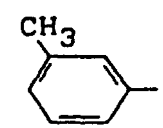 | |

<u>Tabelle 2</u> (Fortsetzung)

| Bsp.-Nr. | X | Y | $Z_n$ | A | B | $R^1$ | Fp° |
|---|---|---|---|---|---|---|---|
| 350 | $CH_3$ | $CH_3$ | 6-$CH_3$ | $CH_3$ | $CH_3$ | 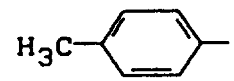 | |
| 351 | $CH_3$ | $CH_3$ | 6-$CH_3$ | $CH_3$ | $CH_3$ | 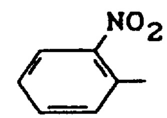 | |
| 352 | $CH_3$ | $CH_3$ | 6-$CH_3$ | $CH_3$ | $CH_3$ | 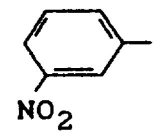 | |
| 353 | $CH_3$ | $CH_3$ | 6-$CH_3$ | $CH_3$ | $CH_3$ | 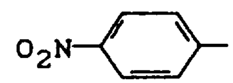 | |
| 354 | $CH_3$ | $CH_3$ | 6-$CH_3$ | $CH_3$ | $CH_3$ | 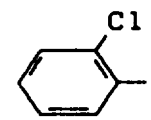 | |
| 355 | $CH_3$ | $CH_3$ | 6-$CH_3$ | $CH_3$ | $CH_3$ | 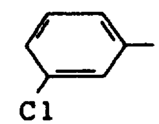 | |
| 356 | $CH_3$ | $CH_3$ | 6-$CH_3$ | $CH_3$ | $CH_3$ | 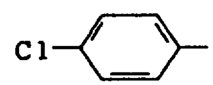 | |

**Tabelle 2** (Fortsetzung)

| Bsp.-Nr. | X | Y | $Z_n$ | A | B | $R^1$ | Fp° |
|---|---|---|---|---|---|---|---|
| 357 | $CH_3$ | $CH_3$ | 6-$CH_3$ | $CH_3$ | $CH_3$ | 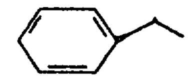 | |
| 358 | $CH_3$ | $CH_3$ | 6-$CH_3$ | $C_2H_5$ | $CH_3$ | $CH_3$ | |
| 359 | $CH_3$ | $CH_3$ | 6-$CH_3$ | $C_2H_5$ | $CH_3$ | $(CH_3)_2CH-$ | |
| 360 | $CH_3$ | $CH_3$ | 6-$CH_3$ | $C_2H_5$ | $CH_3$ | $(CH_3)_3C-$ | |
| 361 | $CH_3$ | $CH_3$ | 6-$CH_3$ | $C_2H_5$ | $CH_3$ | $CH_3-(CH_2)_3-$ | |
| 362 | $CH_3$ | $CH_3$ | 6-$CH_3$ | $C_2H_5$ | $CH_3$ | $C_2H_5-C(CH_3)_2$ | |
| 363 | $CH_3$ | $CH_3$ | 6-$CH_3$ | $C_2H_5$ | $CH_3$ | $(CH_3)_3C-CH_2-$ | |
| 364 | $CH_3$ | $CH_3$ | 6-$CH_3$ | $C_2H_5$ | $CH_3$ | $(CH_3)_2CH-C(CH_3)_2$ | |
| 365 | $CH_3$ | $CH_3$ | 6-$CH_3$ | $C_2H_5$ | $CH_3$ | $CH_2=CH-(CH_2)_8-$ | |
| 366 | $CH_3$ | $CH_3$ | 6-$CH_3$ | $C_2H_5$ | $CH_3$ | 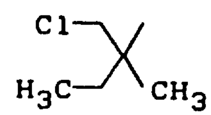 | |
| 367 | $CH_3$ | $CH_3$ | 6-$CH_3$ | $C_2H_5$ | $CH_3$ | $C_4H_9-CH-C_2H_5$ | |

Tabelle 2 (Fortsetzung)

| Bsp.-Nr. | X | Y | $Z_n$ | A | B | $R^1$ | Fp° |
|---|---|---|---|---|---|---|---|
| 368 | $CH_3$ | $CH_3$ | 6-$CH_3$ | $C_2H_5$ | $CH_3$ | 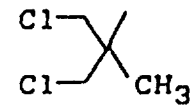 | |
| 369 | $CH_3$ | $CH_3$ | 6-$CH_3$ | $C_2H_5$ | $CH_3$ | 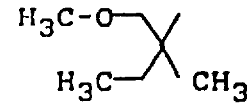 | |
| 370 | $CH_3$ | $CH_3$ | 6-$CH_3$ | $C_2H_5$ | $CH_3$ | 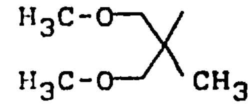 | |
| 371 | $CH_3$ | $CH_3$ | 6-$CH_3$ | $C_2H_5$ | $CH_3$ | 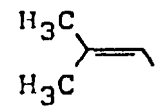 | |
| 372 | $CH_3$ | $CH_3$ | 6-$CH_3$ | $C_2H_5$ | $CH_3$ | $H_3C-S-CH_2-$ | |
| 373 [1)] | $CH_3$ | $CH_3$ | 6-$CH_3$ | $C_2H_5$ | $CH_3$ | 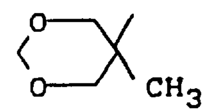 | |
| 374 [1)] | $CH_3$ | $CH_3$ | 6-$CH_3$ | $C_2H_5$ | $CH_3$ | 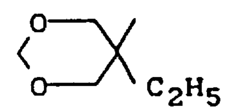 | |
| 375 | $CH_3$ | $CH_3$ | 6-$CH_3$ | $C_2H_5$ | $CH_3$ | 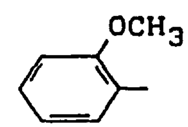 | |

Tabelle 2 (Fortsetzung)

| Bsp.-Nr. | X | Y | $Z_n$ | A | B | $R^1$ | Fp° |
|---|---|---|---|---|---|---|---|
| 376 | $CH_3$ | $CH_3$ | 6-$CH_3$ | $C_2H_5$ | $CH_3$ | 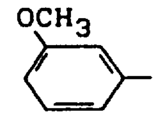 | |
| 377 | $CH_3$ | $CH_3$ | 6-$CH_3$ | $C_2H_5$ | $CH_3$ | 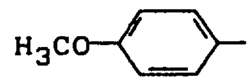 | |
| 378 | $CH_3$ | $CH_3$ | 6-$CH_3$ | $C_2H_5$ | $CH_3$ | 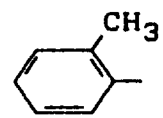 | |
| 379 | $CH_3$ | $CH_3$ | 6-$CH_3$ | $C_2H_5$ | $CH_3$ | 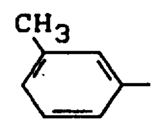 | |
| 380 | $CH_3$ | $CH_3$ | 6-$CH_3$ | $C_2H_5$ | $CH_3$ | 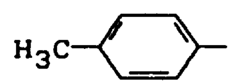 | |
| 381 | $CH_3$ | $CH_3$ | 6-$CH_3$ | $C_2H_5$ | $CH_3$ | 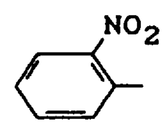 | |
| 382 | $CH_3$ | $CH_3$ | 6-$CH_3$ | $C_2H_5$ | $CH_3$ | 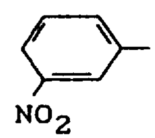 | |

Tabelle 2 (Fortsetzung)

| Bsp.-Nr. | X | Y | $Z_n$ | A | B | $R^1$ | Fp° |
|---|---|---|---|---|---|---|---|
| 383 | $CH_3$ | $CH_3$ | $6-CH_3$ | $C_2H_5$ | $CH_3$ | 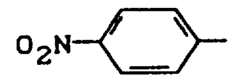 | |
| 384 | $CH_3$ | $CH_3$ | $6-CH_3$ | $C_2H_5$ | $CH_3$ | 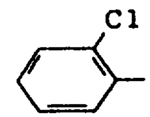 | |
| 385 | $CH_3$ | $CH_3$ | $6-CH_3$ | $C_2H_5$ | $CH_3$ | 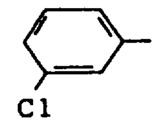 | |
| 386 | $CH_3$ | $CH_3$ | $6-CH_3$ | $C_2H_5$ | $CH_3$ | 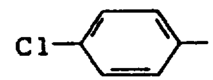 | |
| 387 | $CH_3$ | $CH_3$ | $6-CH_3$ | $C_2H_5$ | $CH_3$ | 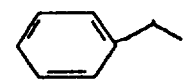 | |
| 388 | $CH_3$ | $CH_3$ | $6-CH_3$ | $C_2H_5$ | $C_2H_5$ | $CH_3$ | |
| 389 | $CH_3$ | $CH_3$ | $6-CH_3$ | $C_2H_5$ | $C_2H_5$ | $(CH_3)_2CH-$ | |
| 390 | $CH_3$ | $CH_3$ | $6-CH_3$ | $C_2H_5$ | $C_2H_5$ | $(CH_3)_3C-$ | |
| 391 | $CH_3$ | $CH_3$ | $6-CH_3$ | $C_2H_5$ | $C_2H_5$ | $CH_3-(CH_2)_3-$ | |
| 392 | $CH_3$ | $CH_3$ | $6-CH_3$ | $C_2H_5$ | $C_2H_5$ | $C_2H_5-C(CH_3)_2$ | |

**Tabelle 2** (Fortsetzung)

| Bsp.-Nr. | X | Y | $Z_n$ | A | B | $R^1$ | Fp° |
|---|---|---|---|---|---|---|---|
| 393 | $CH_3$ | $CH_3$ | $6-CH_3$ | $C_2H_5$ | $C_2H_5$ | $(CH_3)_3C-CH_2-$ | |
| 394 | $CH_3$ | $CH_3$ | $6-CH_3$ | $C_2H_5$ | $C_2H_5$ | $(CH_3)_2CH-C(CH_3)_2$ | |
| 395 | $CH_3$ | $CH_3$ | $6-CH_3$ | $C_2H_5$ | $C_2H_5$ | $CH_2=CH-(CH_2)_8-$ | |
| 396 | $CH_3$ | $CH_3$ | $6-CH_3$ | $C_2H_5$ | $C_2H_5$ | 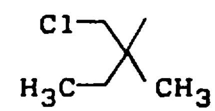 | |
| 397 | $CH_3$ | $CH_3$ | $6-CH_3$ | $C_2H_5$ | $C_2H_5$ | $C_4H_9-CH-C_2H_5$ | |
| 398 | $CH_3$ | $CH_3$ | $6-CH_3$ | $C_2H_5$ | $C_2H_5$ | 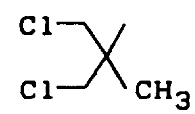 | |
| 399 | $CH_3$ | $CH_3$ | $6-CH_3$ | $C_2H_5$ | $C_2H_5$ | 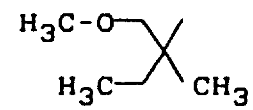 | |
| 400 | $CH_3$ | $CH_3$ | $6-CH_3$ | $C_2H_5$ | $C_2H_5$ | 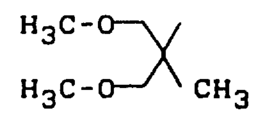 | |
| 401 | $CH_3$ | $CH_3$ | $6-CH_3$ | $C_2H_5$ | $C_2H_5$ | 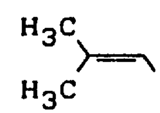 | |

<u>Tabelle 2</u> (Fortsetzung)

| Bsp.-Nr. | X | Y | $Z_n$ | A | B | $R^1$ | Fp⁰ |
|---|---|---|---|---|---|---|---|
| 402 | $CH_3$ | $CH_3$ | 6-$CH_3$ | $C_2H_5$ | $C_2H_5$ | $H_3C-S-CH_2-$ | |
| 403 1) | $CH_3$ | $CH_3$ | 6-$CH_3$ | $C_2H_5$ | $C_2H_5$ | 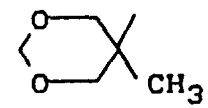 | |
| 404 1) | $CH_3$ | $CH_3$ | 6-$CH_3$ | $C_2H_5$ | $C_2H_5$ | 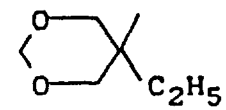 | |
| 405 | $CH_3$ | $CH_3$ | 6-$CH_3$ | $C_2H_5$ | $C_2H_5$ | 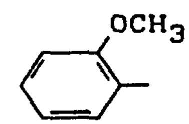 | |
| 406 | $CH_3$ | $CH_3$ | 6-$CH_3$ | $C_2H_5$ | $C_2H_5$ | 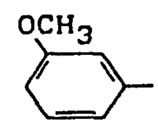 | |
| 407 | $CH_3$ | $CH_3$ | 6-$CH_3$ | $C_2H_5$ | $C_2H_5$ | 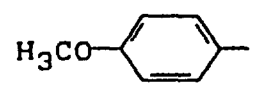 | |
| 408 | $CH_3$ | $CH_3$ | 6-$CH_3$ | $C_2H_5$ | $C_2H_5$ | 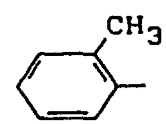 | |

Tabelle 2 (Fortsetzung)

| Bsp.-Nr. | X | Y | $Z_n$ | A | B | $R^1$ | Fp° |
|---|---|---|---|---|---|---|---|
| 409 | $CH_3$ | $CH_3$ | $6-CH_3$ | $C_2H_5$ | $C_2H_5$ | 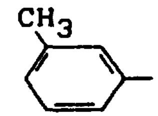 | |
| 410 | $CH_3$ | $CH_3$ | $6-CH_3$ | $C_2H_5$ | $C_2H_5$ | 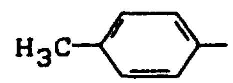 | |
| 411 | $CH_3$ | $CH_3$ | $6-CH_3$ | $C_2H_5$ | $C_2H_5$ | 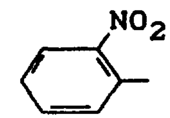 | |
| 412 | $CH_3$ | $CH_3$ | $6-CH_3$ | $C_2H_5$ | $C_2H_5$ | 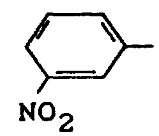 | |
| 413 | $CH_3$ | $CH_3$ | $6-CH_3$ | $C_2H_5$ | $C_2H_5$ | 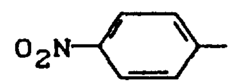 | |
| 414 | $CH_3$ | $CH_3$ | $6-CH_3$ | $C_2H_5$ | $C_2H_5$ | 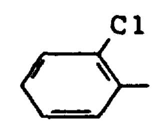 | |
| 415 | $CH_3$ | $CH_3$ | $6-CH_3$ | $C_2H_5$ | $C_2H_5$ | 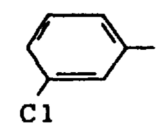 | |

<u>Tabelle 2</u> (Fortsetzung)

| Bsp.-Nr. | X | Y | $Z_n$ | A | B | $R^1$ | Fp° |
|---|---|---|---|---|---|---|---|
| 416 | $CH_3$ | $CH_3$ | 6-$CH_3$ | $C_2H_5$ | $C_2H_5$ | 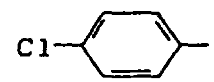 | |
| 417 | $CH_3$ | $CH_3$ | 6-$CH_3$ | $C_2H_5$ | $C_2H_5$ | 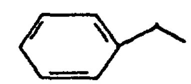 | |
| 418 | $CH_3$ | $CH_3$ | 6-$CH_3$ | $C_3H_7$ | $CH_3$ | $CH_3$ | |
| 419 | $CH_3$ | $CH_3$ | 6-$CH_3$ | $C_3H_7$ | $CH_3$ | $(CH_3)_2CH-$ | |
| 420 | $CH_3$ | $CH_3$ | 6-$CH_3$ | $C_3H_7$ | $CH_3$ | $(CH_3)_3C-$ | |
| 421 | $CH_3$ | $CH_3$ | 6-$CH_3$ | $C_3H_7$ | $CH_3$ | $CH_3-(CH_2)_3-$ | |
| 422 | $CH_3$ | $CH_3$ | 6-$CH_3$ | $C_3H_7$ | $CH_3$ | $C_2H_5-C(CH_3)_2$ | |
| 423 | $CH_3$ | $CH_3$ | 6-$CH_3$ | $C_3H_7$ | $CH_3$ | $(CH_3)_3C-CH_2-$ | |
| 424 | $CH_3$ | $CH_3$ | 6-$CH_3$ | $C_3H_7$ | $CH_3$ | $(CH_3)_2CH-C(CH_3)_2$ | |
| 425 | $CH_3$ | $CH_3$ | 6-$CH_3$ | $C_3H_7$ | $CH_3$ | $CH_2=CH-(CH_2)_8-$ | |
| 426 | $CH_3$ | $CH_3$ | 6-$CH_3$ | $C_3H_7$ | $CH_3$ | 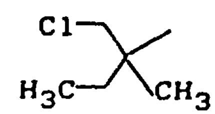 | |

Tabelle 2 (Fortsetzung)

| Bsp.-Nr. | X | Y | $Z_n$ | A | B | $R^1$ | Fp° |
|---|---|---|---|---|---|---|---|
| 427 | $CH_3$ | $CH_3$ | 6-$CH_3$ | $C_3H_7$ | $CH_3$ | $C_4H_9-CH-C_2H_5$ | |
| 428 | $CH_3$ | $CH_3$ | 6-$CH_3$ | $C_3H_7$ | $CH_3$ | 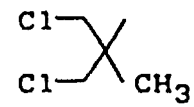 | |
| 429 | $CH_3$ | $CH_3$ | 6-$CH_3$ | $C_3H_7$ | $CH_3$ | 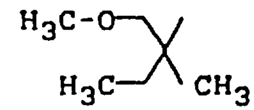 | |
| 430 | $CH_3$ | $CH_3$ | 6-$CH_3$ | $C_3H_7$ | $CH_3$ | 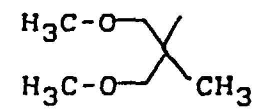 | |
| 431 | $CH_3$ | $CH_3$ | 6-$CH_3$ | $C_3H_7$ | $CH_3$ | 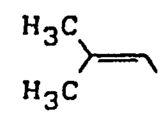 | |
| 432 | $CH_3$ | $CH_3$ | 6-$CH_3$ | $C_3H_7$ | $CH_3$ | $H_3C-S-CH_2-$ | |
| 433 1) | $CH_3$ | $CH_3$ | 6-$CH_3$ | $C_3H_7$ | $CH_3$ | 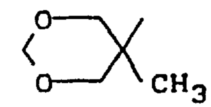 | |
| 434 1) | $CH_3$ | $CH_3$ | 6-$CH_3$ | $C_3H_7$ | $CH_3$ | 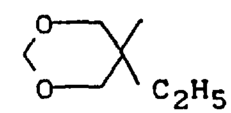 | |

Tabelle 2 (Fortsetzung)

| Bsp.-Nr. | X | Y | $Z_n$ | A | B | $R^1$ | Fp° |
|---|---|---|---|---|---|---|---|
| 435 | $CH_3$ | $CH_3$ | 6-$CH_3$ | $C_3H_7$ | $CH_3$ | 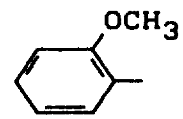 | |
| 436 | $CH_3$ | $CH_3$ | 6-$CH_3$ | $C_3H_7$ | $CH_3$ | 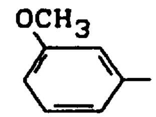 | |
| 437 | $CH_3$ | $CH_3$ | 6-$CH_3$ | $C_3H_7$ | $CH_3$ | 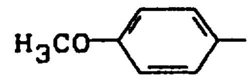 | |
| 438 | $CH_3$ | $CH_3$ | 6-$CH_3$ | $C_3H_7$ | $CH_3$ | 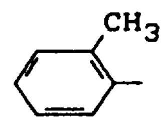 | |
| 439 | $CH_3$ | $CH_3$ | 6-$CH_3$ | $C_3H_7$ | $CH_3$ | 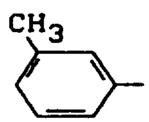 | |
| 440 | $CH_3$ | $CH_3$ | 6-$CH_3$ | $C_3H_7$ | $CH_3$ | 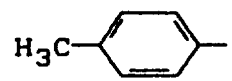 | |
| 441 | $CH_3$ | $CH_3$ | 6-$CH_3$ | $C_3H_7$ | $CH_3$ | 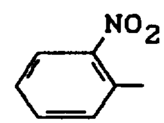 | |

<u>Tabelle 2</u> (Fortsetzung)

| Bsp.-Nr. | X | Y | $Z_n$ | A | B | $R^1$ | Fp° |
|---|---|---|---|---|---|---|---|
| 442 | $CH_3$ | $CH_3$ | 6-$CH_3$ | $C_3H_7$ | $CH_3$ | 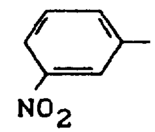 | |
| 443 | $CH_3$ | $CH_3$ | 6-$CH_3$ | $C_3H_7$ | $CH_3$ | 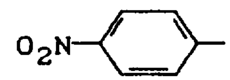 | |
| 444 | $CH_3$ | $CH_3$ | 6-$CH_3$ | $C_3H_7$ | $CH_3$ | 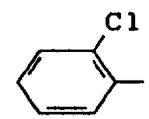 | |
| 445 | $CH_3$ | $CH_3$ | 6-$CH_3$ | $C_3H_7$ | $CH_3$ | 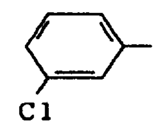 | |
| 446 | $CH_3$ | $CH_3$ | 6-$CH_3$ | $C_3H_7$ | $CH_3$ | 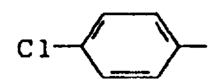 | |
| 447 | $CH_3$ | $CH_3$ | 6-$CH_3$ | $C_3H_7$ | $CH_3$ | 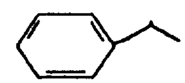 | |
| 448 | $CH_3$ | $CH_3$ | 6-$CH_3$ | i-$C_3H_7$ | $CH_3$ | $CH_3$ | |
| 449 | $CH_3$ | $CH_3$ | 6-$CH_3$ | i-$C_3H_7$ | $CH_3$ | $(CH_3)_2CH-$ | |
| 450 | $CH_3$ | $CH_3$ | 6-$CH_3$ | i-$C_3H_7$ | $CH_3$ | $(CH_3)_3C-$ | |
| 451 | $CH_3$ | $CH_3$ | 6-$CH_3$ | i-$C_3H_7$ | $CH_3$ | $CH_3-(CH_2)_3-$ | |

<u>Tabelle 2</u> (Fortsetzung)

| Bsp.-Nr. | X | Y | $Z_n$ | A | B | $R^1$ | Fp° |
|---|---|---|---|---|---|---|---|
| 452 | $CH_3$ | $CH_3$ | $6-CH_3$ | $i-C_3H_7$ | $CH_3$ | $C_2H_5-C(CH_3)_2$ | |
| 453 | $CH_3$ | $CH_3$ | $6-CH_3$ | $i-C_3H_7$ | $CH_3$ | $(CH_3)_3C-CH_2-$ | |
| 454 | $CH_3$ | $CH_3$ | $6-CH_3$ | $i-C_3H_7$ | $CH_3$ | $(CH_3)_2CH-C(CH_3)_2$ | |
| 455 | $CH_3$ | $CH_3$ | $6-CH_3$ | $i-C_3H_7$ | $CH_3$ | $CH_2=CH-(CH_2)_8-$ | |
| 456 | $CH_3$ | $CH_3$ | $6-CH_3$ | $i-C_3H_7$ | $CH_3$ | 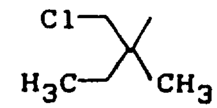 | |
| 457 | $CH_3$ | $CH_3$ | $6-CH_3$ | $i-C_3H_7$ | $CH_3$ | $C_4H_9-CH-C_2H_5$ | |
| 458 | $CH_3$ | $CH_3$ | $6-CH_3$ | $i-C_3H_7$ | $CH_3$ | 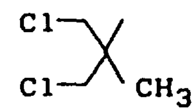 | |
| 459 | $CH_3$ | $CH_3$ | $6-CH_3$ | $i-C_3H_7$ | $CH_3$ | 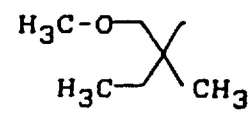 | |
| 460 | $CH_3$ | $CH_3$ | $6-CH_3$ | $i-C_3H_7$ | $CH_3$ | 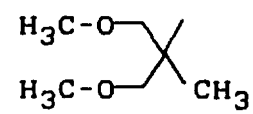 | |

Tabelle 2 (Fortsetzung)

| Bsp.-Nr. | X | Y | $Z_n$ | A | B | $R^1$ | Fp° |
|---|---|---|---|---|---|---|---|
| 461 | $CH_3$ | $CH_3$ | $6\text{-}CH_3$ | $i\text{-}C_3H_7$ | $CH_3$ | 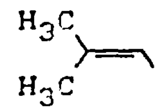 | |
| 462 | $CH_3$ | $CH_3$ | $6\text{-}CH_3$ | $i\text{-}C_3H_7$ | $CH_3$ | $H_3C\text{-}S\text{-}CH_2\text{-}$ | |
| 463 1) | $CH_3$ | $CH_3$ | $6\text{-}CH_3$ | $i\text{-}C_3H_7$ | $CH_3$ | 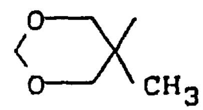 | |
| 464 1) | $CH_3$ | $CH_3$ | $6\text{-}CH_3$ | $i\text{-}C_3H_7$ | $CH_3$ | 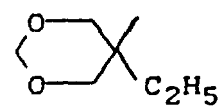 | |
| 465 | $CH_3$ | $CH_3$ | $6\text{-}CH_3$ | $i\text{-}C_3H_7$ | $CH_3$ | 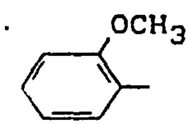 | |
| 466 | $CH_3$ | $CH_3$ | $6\text{-}CH_3$ | $i\text{-}C_3H_7$ | $CH_3$ | 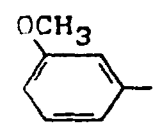 | |
| 467 | $CH_3$ | $CH_3$ | $6\text{-}CH_3$ | $i\text{-}C_3H_7$ | $CH_3$ | 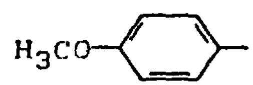 | |

**Tabelle 2** (Fortsetzung)

| Bsp.-Nr. | X | Y | $Z_n$ | A | B | $R^1$ | Fp° |
|---|---|---|---|---|---|---|---|
| 468 | $CH_3$ | $CH_3$ | $6-CH_3$ | $i-C_3H_7$ | H | 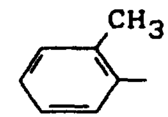 | |
| 469 | $CH_3$ | $CH_3$ | $6-CH_3$ | $i-C_3H_7$ | H | 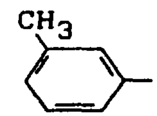 | |
| 470 | $CH_3$ | $CH_3$ | $6-CH_3$ | $i-C_3H_7$ | H | 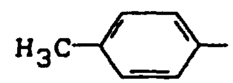 | |
| 471 | $CH_3$ | $CH_3$ | $6-CH_3$ | $i-C_3H_7$ | H | 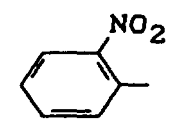 | |
| 472 | $CH_3$ | $CH_3$ | $6-CH_3$ | $i-C_3H_7$ | H | 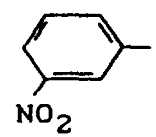 | |
| 473 | $CH_3$ | $CH_3$ | $6-CH_3$ | $i-C_3H_7$ | H | 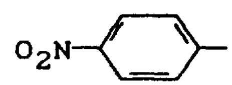 | |
| 474 | $CH_3$ | $CH_3$ | $6-CH_3$ | $i-C_3H_7$ | H | 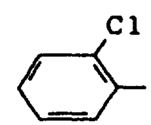 | |

**Tabelle 2** (Fortsetzung)

| Bsp.-Nr. | X | Y | $Z_n$ | A | B | $R^1$ | $Fp^0$ |
|---|---|---|---|---|---|---|---|
| 475 | $CH_3$ | $CH_3$ | $6-CH_3$ | $i-C_3H_7$ | H | 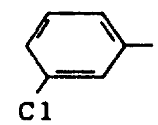 | |
| 476 | $CH_3$ | $CH_3$ | $6-CH_3$ | $i-C_3H_7$ | H | 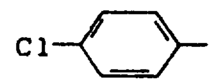 | |
| 477 | $CH_3$ | $CH_3$ | $6-CH_3$ | $i-C_3H_7$ | H | 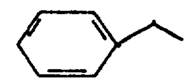 | |
| 478 | $CH_3$ | $CH_3$ | $6-CH_3$ | $-(CH_2)_4-$ | | $CH_3$ | |
| 479 | $CH_3$ | $CH_3$ | $6-CH_3$ | $-(CH_2)_4-$ | | $(CH_3)_2CH-$ | |
| 480 | $CH_3$ | $CH_3$ | $6-CH_3$ | $-(CH_2)_4-$ | | $(CH_3)_3C-$ | |
| 481 | $CH_3$ | $CH_3$ | $6-CH_3$ | $-(CH_2)_4-$ | | $CH_3-(CH_2)_3-$ | |
| 482 | $CH_3$ | $CH_3$ | $6-CH_3$ | $-(CH_2)_4-$ | | $C_2H_5-C(CH_3)_2$ | |
| 483 | $CH_3$ | $CH_3$ | $6-CH_3$ | - $-(CH_2)_4-$ | | $(CH_3)_3C-CH_2-$ | |
| 484 | $CH_3$ | $CH_3$ | $6-CH_3$ | $-(CH_2)_4-$ | | $(CH_3)_2CH-C(CH_3)_2$ | |
| 485 | $CH_3$ | $CH_3$ | $6-CH_3$ | $-(CH_2)_4-$ | | $CH_2=CH-(CH_2)_8-$ | |

Tabelle 2 (Fortsetzung)

| Bsp.-Nr. | X | Y | $Z_n$ | A | B | $R^1$ | Fp° |
|---|---|---|---|---|---|---|---|
| 486 | $CH_3$ | $CH_3$ | 6-$CH_3$ | $-(CH_2)_4-$ | | 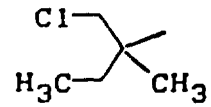 | |
| 487 | $CH_3$ | $CH_3$ | 6-$CH_3$ | $-(CH_2)_4-$ | | $C_4H_9-CH-C_2H_5$ | |
| 488 | $CH_3$ | $CH_3$ | 6-$CH_3$ | $-(CH_2)_4-$ | | 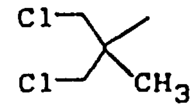 | |
| 489 | $CH_3$ | $CH_3$ | 6-$CH_3$ | $-(CH_2)_4-$ | | 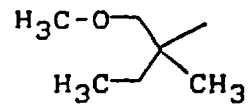 | |
| 490 | $CH_3$ | $CH_3$ | 6-$CH_3$ | $-(CH_2)_4-$ | | 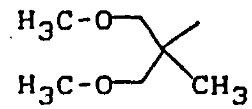 | |
| 491 | $CH_3$ | $CH_3$ | 6-$CH_3$ | $-(CH_2)_4-$ | | 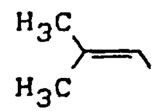 | |
| 492 | $CH_3$ | $CH_3$ | 6-$CH_3$ | $-(CH_2)_4-$ | | $H_3C-S-CH_2-$ | |
| 493[1] | $CH_3$ | $CH_3$ | 6-$CH_3$ | $-(CH_2)_4-$ | | 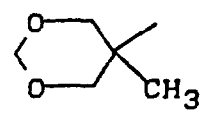 | |
| 494[1] | $CH_3$ | $CH_3$ | 6-$CH_3$ | $-(CH_2)_4-$ | | 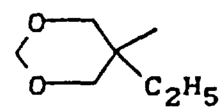 | |

**Tabelle 2** (Fortsetzung)

| Bsp.-Nr. | X | Y | $Z_n$ | A | B | $R^1$ | $Fp^0$ |
|---|---|---|---|---|---|---|---|
| 495 | $CH_3$ | $CH_3$ | $6-CH_3$ | | $-(CH_2)_4-$ | 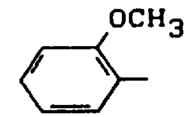 | |
| 496 | $CH_3$ | $CH_3$ | $6-CH_3$ | | $-(CH_2)_4-$ | 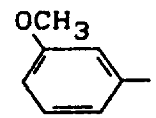 | |
| 497 | $CH_3$ | $CH_3$ | $6-CH_3$ | | $-(CH_2)_4-$ | 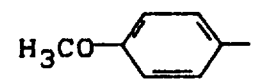 | |
| 498 | $CH_3$ | $CH_3$ | $6-CH_3$ | | $-(CH_2)_4-$ | 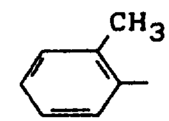 | |
| 499 | $CH_3$ | $CH_3$ | $6-CH_3$ | | $-(CH_2)_4-$ | 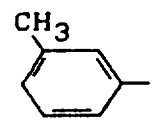 | |
| 500 | $CH_3$ | $CH_3$ | $6-CH_3$ | | $-(CH_2)_4-$ | 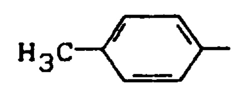 | |
| 501 | $CH_3$ | $CH_3$ | $6-CH_3$ | | $-(CH_2)_4-$ | 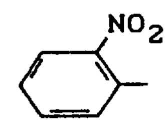 | |

**Tabelle 2** (Fortsetzung)

| Bsp.-Nr. | X | Y | $Z_n$ | A | B | $R^1$ | Fp$^0$ |
|---|---|---|---|---|---|---|---|
| 502 | $CH_3$ | $CH_3$ | $6-CH_3$ | $-(CH_2)_4-$ | | 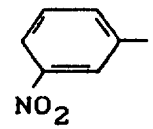 | |
| 503 | $CH_3$ | $CH_3$ | $6-CH_3$ | $-(CH_2)_4-$ | | 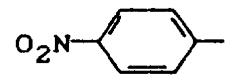 | |
| 504 | $CH_3$ | $CH_3$ | $6-CH_3$ | $-(CH_2)_4-$ | | 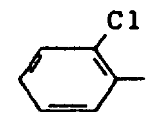 | |
| 505 | $CH_3$ | $CH_3$ | $6-CH_3$ | $-(CH_2)_4-$ | | 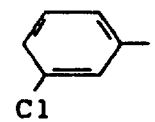 | |
| 506 | $CH_3$ | $CH_3$ | $6-CH_3$ | $-(CH_2)_4-$ | | 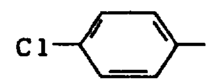 | |
| 507 | $CH_3$ | $CH_3$ | $6-CH_3$ | $-(CH_2)_4-$ | | 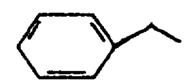 | |
| 508 | $CH_3$ | $CH_3$ | $6-CH_3$ | $-(CH_2)_5-$ | | $CH_3$ | |
| 509 | $CH_3$ | $CH_3$ | $6-CH_3$ | $-(CH_2)_5-$ | | $(CH_3)_2CH-$ | |
| 510 | $CH_3$ | $CH_3$ | $6-CH_3$ | $-(CH_2)_5-$ | | $(CH_3)_3C-$ | |
| 511 | $CH_3$ | $CH_3$ | $6-CH_3$ | $-(CH_2)_5-$ | | $CH_3-(CH_2)_3-$ | |

**Tabelle 2** (Fortsetzung)

| Bsp.-Nr. | X | Y | $Z_n$ | A | B | $R^1$ | $Fp^0$ |
|---|---|---|---|---|---|---|---|
| 512 | $CH_3$ | $CH_3$ | 6-$CH_3$ | $-(CH_2)_5-$ | | $C_2H_5-C(CH_3)_2$ | |
| 513 | $CH_3$ | $CH_3$ | 6-$CH_3$ | $-(CH_2)_5-$ | | $(CH_3)_3C-CH_2-$ | |
| 514 | $CH_3$ | $CH_3$ | 6-$CH_3$ | $-(CH_2)_5-$ | | $(CH_3)_2CH-C(CH_3)_2$ | |
| 515 | $CH_3$ | $CH_3$ | 6-$CH_3$ | $-(CH_2)_5-$ | | $CH_2=CH-(CH_2)_8-$ | |
| 516 | $CH_3$ | $CH_3$ | 6-$CH_3$ | $-(CH_2)_5-$ | | 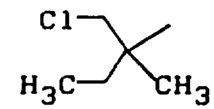 | |
| 517 | $CH_3$ | $CH_3$ | 6-$CH_3$ | $-(CH_2)_5-$ | | $C_4H_9-CH-C_2H_5$ | |
| 518 | $CH_3$ | $CH_3$ | 6-$CH_3$ | $-(CH_2)_5-$ | | 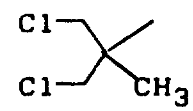 | |
| 519 | $CH_3$ | $CH_3$ | 6-$CH_3$ | $-(CH_2)_5-$ | | 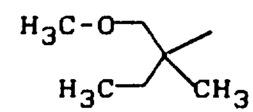 | |
| 520 | $CH_3$ | $CH_3$ | 6-$CH_3$ | $-(CH_2)_5-$ | | 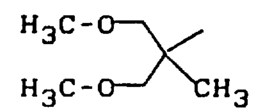 | |

<u>Tabelle 2</u> (Fortsetzung)

| Bsp.-Nr. | X | Y | $Z_n$ | A | B | $R^1$ | Fp° |
|---|---|---|---|---|---|---|---|
| 521 | $CH_3$ | $CH_3$ | 6-$CH_3$ | $-(CH_2)_5-$ | | 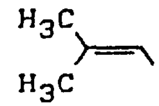 | |
| 522 | $CH_3$ | $CH_3$ | 6-$CH_3$ | $-(CH_2)_5-$ | | $H_3C-S-CH_2-$ | |
| 523[1] | $CH_3$ | $CH_3$ | 6-$CH_3$ | $-(CH_2)_5-$ | | 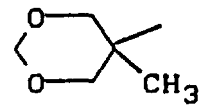 | |
| 524[1] | $CH_3$ | $CH_3$ | 6-$CH_3$ | $-(CH_2)_5-$ | | 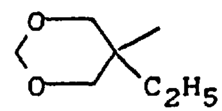 | |
| 525 | $CH_3$ | $CH_3$ | 6-$CH_3$ | $-(CH_2)_5-$ | | 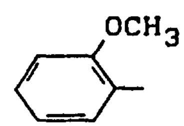 | |
| 526 | $CH_3$ | $CH_3$ | 6-$CH_3$ | $-(CH_2)_5-$ | | 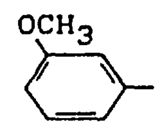 | |
| 527 | $CH_3$ | $CH_3$ | 6-$CH_3$ | $-(CH_2)_5-$ | | 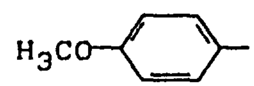 | |

<u>Tabelle 2</u> (Fortsetzung)

| Bsp.-Nr. | X | Y | $Z_n$ | A | B | $R^1$ | Fp° |
|---|---|---|---|---|---|---|---|
| 528 | $CH_3$ | $CH_3$ | 6-$CH_3$ | $-(CH_2)_5-$ | | 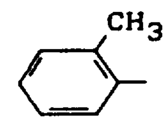 | |
| 529 | $CH_3$ | $CH_3$ | 6-$CH_3$ | $-(CH_2)_5-$ | | 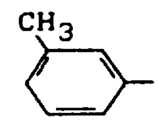 | |
| 530 | $CH_3$ | $CH_3$ | 6-$CH_3$ | $-(CH_2)_5-$ | | 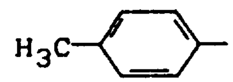 | |
| 531 | $CH_3$ | $CH_3$ | 6-$CH_3$ | $-(CH_2)_5-$ | | 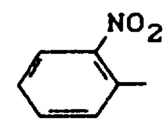 | |
| 532 | $CH_3$ | $CH_3$ | 6-$CH_3$ | $-(CH_2)_5-$ | | 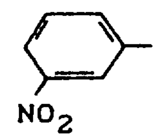 | |
| 533 | $CH_3$ | $CH_3$ | 6-$CH_3$ | $-(CH_2)_5-$ | | 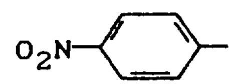 | |

Tabelle 2 (Fortsetzung)

| Bsp.-Nr. | X | Y | $Z_n$ | A | B | $R^1$ | Fp° |
|---|---|---|---|---|---|---|---|
| 534 | $CH_3$ | $CH_3$ | $6-CH_3$ | $-(CH_2)_5-$ | | 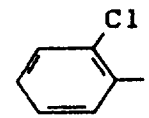 | |
| 535 | $CH_3$ | $CH_3$ | $6-CH_3$ | $-(CH_2)_5-$ | | 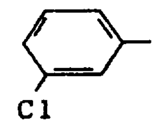 | |
| 536 | $CH_3$ | $CH_3$ | $6-CH_3$ | $-(CH_2)_5-$ | | 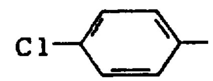 | |
| 537 | $CH_3$ | $CH_3$ | $6-CH_3$ | $-(CH_2)_5-$ | | 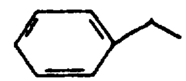 | |

**Tabelle 3**

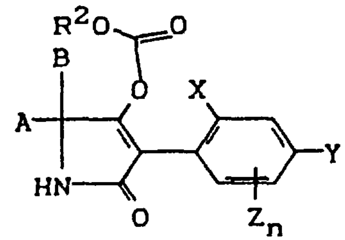

(Ic)

| Bsp.-Nr. | X | Y | $Z_n$ | A | B | $R^2$ | Fp° C |
|---|---|---|---|---|---|---|---|
| 538 | Cl | Cl | H | $CH_3$ | $CH_3$ | $CH_3$ | |
| 539 | Cl | Cl | H | $CH_3$ | $CH_3$ | $C_2H_5$ | |
| 540 | Cl | Cl | H | $CH_3$ | $CH_3$ | $(CH_3)_2CH-$ | |
| 541 | Cl | Cl | H | $CH_3$ | $CH_3$ | $(CH_3)_2CH-CH_2-$ | |
| 542 | Cl | Cl | H | $CH_3$ | $CH_3$ | $C_2H_5-CH-$<br>\|<br>$CH_3$ | |
| 543 | Cl | Cl | H | $CH_3$ | $CH_3$ | $(CH_3)_3C-$ | |
| 544 | Cl | Cl | H | $CH_3$ | $CH_3$ | $(CH_3)_3C-CH_2-$ | |
| 545 1) | Cl | Cl | H | $CH_3$ | $CH_3$ | 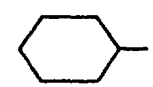 | |
| 546 | Cl | Cl | H | $CH_3$ | $CH_3$ | $C_2H_5O$ 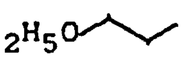 | |
| 547 | Cl | Cl | H | $CH_3$ | $CH_3$ | $C_2H_5O$ 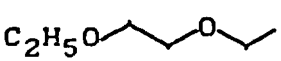 | |

<u>**Tabelle 3**</u> **(Fortsetzung)**

| Bsp.-Nr. | X | Y | $Z_n$ | A | B | $R^2$ | Fp° C |
|---|---|---|---|---|---|---|---|
| 548 | Cl | Cl | H | $CH_3$ | $CH_3$ | 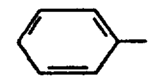 | |
| 549 | Cl | Cl | H | $CH_3$ | $CH_3$ | 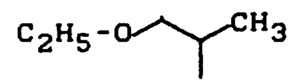 | |
| 550 | Cl | Cl | H | $CH_3$ | $CH_3$ | 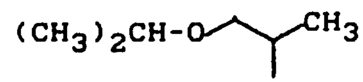 | |
| 551 | Cl | Cl | H | $CH_3$ | $CH_3$ | 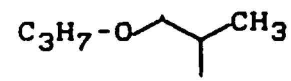 | |
| 552 | Cl | Cl | H | $CH_3$ | $CH_3$ | 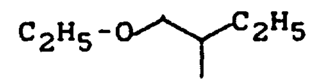 | |
| 553 | Cl | Cl | H | $C_2H_5$ | $CH_3$ | $CH_3$ | |
| 554 | Cl | Cl | H | $C_2H_5$ | $CH_3$ | $C_2H_5$ | |
| 555 | Cl | Cl | H | $C_2H_5$ | $CH_3$ | $(CH_3)_2CH-$ | |
| 556 | Cl | Cl | H | $C_2H_5$ | $CH_3$ | $(CH_3)_2CH-CH_2-$ | |
| 557 | Cl | Cl | H | $C_2H_5$ | $CH_3$ | $C_2H_5-CH-$<br>\|<br>$CH_3$ | |
| 558 | Cl | Cl | H | $C_2H_5$ | $CH_3$ | $(CH_3)_3C-$ | |
| 559 | Cl | Cl | H | $C_2H_5$ | $CH_3$ | $(CH_3)_3C-CH_2-$ | |

<u>Tabelle 3</u> (Fortsetzung)

| Bsp.-Nr. | X | Y | $Z_n$ | A | B | $R^2$ | Fp° C |
|---|---|---|---|---|---|---|---|
| 560 [1] | Cl | Cl | H | $C_2H_5$ | $CH_3$ | 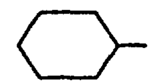 | |
| 561 | Cl | Cl | H | $C_2H_5$ | $CH_3$ | 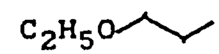 | |
| 562 | Cl | Cl | H | $C_2H_5$ | $CH_3$ | 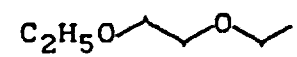 | |
| 563 | Cl | Cl | H | $C_2H_5$ | $CH_3$ | 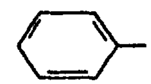 | |
| 564 | Cl | Cl | H | $C_2H_5$ | $CH_3$ | 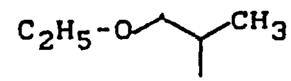 | |
| 565 | Cl | Cl | H | $C_2H_5$ | $CH_3$ | 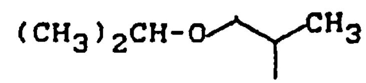 | |
| 566 | Cl | Cl | H | $C_2H_5$ | $CH_3$ | 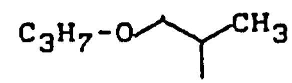 | |
| 567 | Cl | Cl | H | $C_2H_5$ | $CH_3$ | 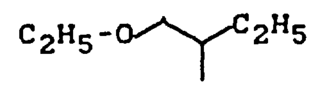 | |
| 568 | Cl | Cl | H | $C_2H_5$ | $C_2H_5$ | $CH_3$ | |
| 569 | Cl | Cl | H | $C_2H_5$ | $C_2H_5$ | $C_2H_5$ | |
| 570 | Cl | Cl | H | $C_2H_5$ | $C_2H_5$ | $(CH_3)_2CH-$ | |
| 571 | Cl | Cl | H | $C_2H_5$ | $C_2H_5$ | $(CH_3)_2CH-CH_2-$ | |

Tabelle 3 (Fortsetzung)

| Bsp.-Nr. | X | Y | $Z_n$ | A | B | $R^2$ | Fp° C |
|---|---|---|---|---|---|---|---|
| 572 | Cl | Cl | H | $C_2H_5$ | $C_2H_5$ | $C_2H_5$-CH-<br>\|<br>$CH_3$ | |
| 573 | Cl | Cl | H | $C_2H_5$ | $C_2H_5$ | $(CH_3)_3C$- | |
| 574 | Cl | Cl | H | $C_2H_5$ | $C_2H_5$ | $(CH_3)_3C-CH_2$- | |
| 575[1] | Cl | Cl | H | $C_2H_5$ | $C_2H_5$ | 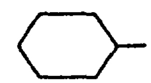 | |
| 576 | Cl | Cl | H | $C_2H_5$ | $C_2H_5$ | 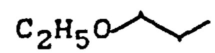 | |
| 577 | Cl | Cl | H | $C_2H_5$ | $C_2H_5$ | 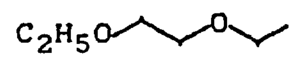 | |
| 578 | Cl | Cl | H | $C_2H_5$ | $C_2H_5$ | 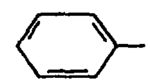 | |
| 579 | Cl | Cl | H | $C_2H_5$ | $C_2H_5$ | 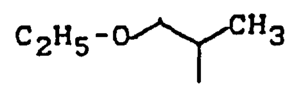 | |
| 580 | Cl | Cl | H | $C_2H_5$ | $C_2H_5$ | 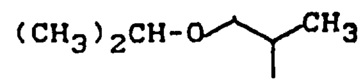 | |
| 581 | Cl | Cl | H | $C_2H_5$ | $C_2H_5$ | 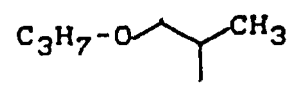 | |
| 582 | Cl | Cl | H | $C_2H_5$ | $C_2H_5$ | 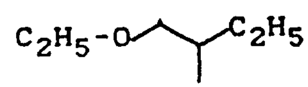 | |

Tabelle 3 (Fortsetzung)

| Bsp.-Nr. | X | Y | $Z_n$ | A | B | $R^2$ | Fp° C |
|---|---|---|---|---|---|---|---|
| 583 | Cl | Cl | H | $C_3H_7$ | $CH_3$ | $CH_3$ | |
| 584 | Cl | Cl | H | $C_3H_7$ | $CH_3$ | $C_2H_5$ | |
| 585 | Cl | Cl | H | $C_3H_7$ | $CH_3$ | $(CH_3)_2CH-$ | |
| 586 | Cl | Cl | H | $C_3H_7$ | $CH_3$ | $(CH_3)_2CH-CH_2-$ | |
| 587 | Cl | Cl | H | $C_3H_7$ | $CH_3$ | $C_2H_5-CH-$<br>$CH_3$ | |
| 588 | Cl | Cl | H | $C_3H_7$ | $CH_3$ | $(CH_3)_3C-$ | |
| 589 | Cl | Cl | H | $C_3H_7$ | $CH_3$ | $(CH_3)_3C-CH_2-$ | |
| 590 1) | Cl | Cl | H | $C_3H_7$ | $CH_3$ | 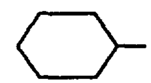 | |
| 591 | Cl | Cl | H | $C_3H_7$ | $CH_3$ | 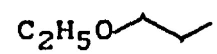 | |
| 592 | Cl | Cl | H | $C_3H_7$ | $CH_3$ | 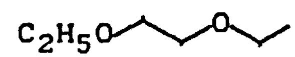 | |
| 593 | Cl | Cl | H | $C_3H_7$ | $CH_3$ | 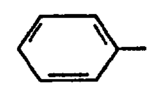 | |
| 594 | Cl | Cl | H | $C_3H_7$ | $CH_3$ | 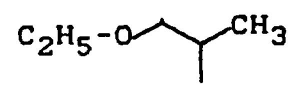 | |

**Tabelle 3** (Fortsetzung)

| Bsp.-Nr. | X | Y | $Z_n$ | A | B | $R^2$ | Fp° C |
|---|---|---|---|---|---|---|---|
| 595 | Cl | Cl | H | $C_3H_7$ | $CH_3$ | 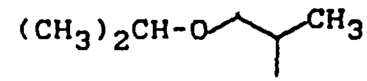 | |
| 596 | Cl | Cl | H | $C_3H_7$ | $CH_3$ | 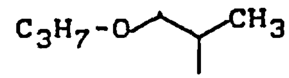 | |
| 597 | Cl | Cl | H | $C_3H_7$ | $CH_3$ | 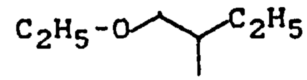 | |
| 598 | Cl | Cl | H | i-$C_3H_7$ | $CH_3$ | $CH_3$ | |
| 699 | Cl | Cl | H | i-$C_3H_7$ | $CH_3$ | $C_2H_5$ | |
| 600 | Cl | Cl | H | i-$C_3H_7$ | $CH_3$ | $(CH_3)_2CH-$ | |
| 601 | Cl | Cl | H | i-$C_3H_7$ | $CH_3$ | $(CH_3)_2CH-CH_2-$ | |
| 602 | Cl | Cl | H | i-$C_3H_7$ | $CH_3$ | $C_2H_5-CH(CH_3)-$ | |
| 603 | Cl | Cl | H | i-$C_3H_7$ | $CH_3$ | $(CH_3)_3C-$ | |
| 604 | Cl | Cl | H | i-$C_3H_7$ | $CH_3$ | $(CH_3)_3C-CH_2-$ | |
| 605 1) | Cl | Cl | H | i-$C_3H_7$ | $CH_3$ | 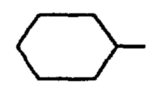 | |
| 606 | Cl | Cl | H | i-$C_3H_7$ | $CH_3$ | 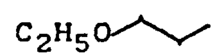 | |

Tabelle 3 (Fortsetzung)

| Bsp.-Nr. | X | Y | $Z_n$ | A | B | $R^2$ | Fp° C |
|---|---|---|---|---|---|---|---|
| 607 | Cl | Cl | H | i-$C_3H_7$ | $CH_3$ | 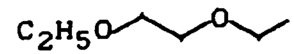 | |
| 608 | Cl | Cl | H | i-$C_3H_7$ | $CH_3$ | 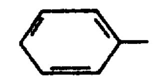 | |
| 609 | Cl | Cl | H | i-$C_3H_7$ | $CH_3$ | 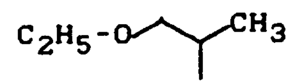 | |
| 610 | Cl | Cl | H | i-$C_3H_7$ | $CH_3$ | 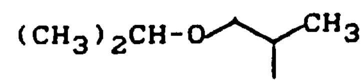 | |
| 611 | Cl | Cl | H | i-$C_3H_7$ | $CH_3$ | 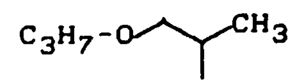 | |
| 612 | Cl | Cl | H | i-$C_3H_7$ | $CH_3$ | 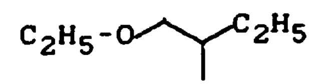 | |
| 613 | Cl | Cl | H | $-(CH_2)_4-$ | | $CH_3$ | |
| 614 | Cl | Cl | H | $-(CH_2)_4-$ | | $C_2H_5$ | |
| 615 | Cl | Cl | H | $-(CH_2)_4-$ | | $(CH_3)_2CH-$ | |
| 616 | Cl | Cl | H | $-(CH_2)_4-$ | | $(CH_3)_2CH-CH_2-$ | |
| 617 | Cl | Cl | H | $-(CH_2)_4-$ | | $C_2H_5-CH(CH_3)-$ | |

Tabelle 3 (Fortsetzung)

| Bsp.-Nr. | X | Y | $Z_n$ | A | B | $R^2$ | Fp° C |
|---|---|---|---|---|---|---|---|
| 618 | Cl | Cl | H | | $-(CH_2)_4-$ | $(CH_3)_3C-$ | |
| 619 | Cl | Cl | H | | $-(CH_2)_4-$ | $(CH_3)_3C-CH_2-$ | |
| 620 1) | Cl | Cl | H | | $-(CH_2)_4-$ | 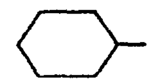 | |
| 621 | Cl | Cl | H | | $-(CH_2)_4-$ | $C_2H_5O$ 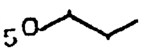 | |
| 622 | Cl | Cl | H | | $-(CH_2)_4-$ | $C_2H_5O$ 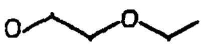 | |
| 623 | Cl | Cl | H | | $-(CH_2)_4-$ | 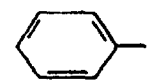 | |
| 624 | Cl | Cl | H | | $-(CH_2)_4-$ | $C_2H_5-O$ 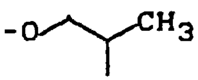 | |
| 625 | Cl | Cl | H | | $-(CH_2)_4-$ | $(CH_3)_2CH-O$ 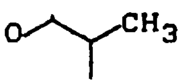 | |
| 626 | Cl | Cl | H | | $-(CH_2)_4-$ | $C_3H_7-O$ 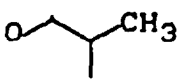 | |
| 627 | Cl | Cl | H | | $-(CH_2)_4-$ | $C_2H_5-O$ 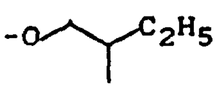 | |

<u>Tabelle 3</u> (Fortsetzung)

| Bsp.-Nr. | X | Y | $Z_n$ | A | B | $R^2$ | Fp° C |
|---|---|---|---|---|---|---|---|
| 628 | Cl | Cl | H | $-(CH_2)_5-$ | | $CH_3$ | |
| 629 | Cl | Cl | H | $-(CH_2)_5-$ | | $C_2H_5$ | |
| 630 | Cl | Cl | H | $-(CH_2)_5-$ | | $(CH_3)_2CH-$ | |
| 631 | Cl | Cl | H | $-(CH_2)_5-$ | | $(CH_3)_2CH-CH_2-$ | |
| 632 | Cl | Cl | H | $-(CH_2)_5-$ | | $C_2H_5$-CH-<br>$CH_3$ | |
| 633 | Cl | Cl | H | $-(CH_2)_5-$ | | $(CH_3)_3C-$ | |
| 634 | Cl | Cl | H | $-(CH_2)_5-$ | | $(CH_3)_3C-CH_2-$ | |
| 635 1) | Cl | Cl | H | $-(CH_2)_5-$ | | 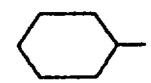 | |
| 636 | Cl | Cl | H | $-(CH_2)_5-$ | | 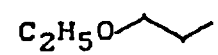 | |
| 637 | Cl | Cl | H | $-(CH_2)_5-$ | | 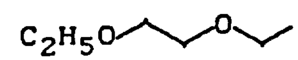 | |
| 638 | Cl | Cl | H | $-(CH_2)_5-$ | | 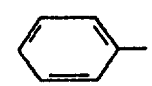 | |
| 639 | Cl | Cl | H | $-(CH_2)_5-$ | | 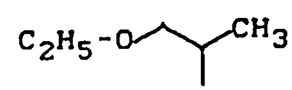 | |

Tabelle 3 (Fortsetzung)

| Bsp.-Nr. | X | Y | $Z_n$ | A | B | $R^2$ | Fp° C |
|---|---|---|---|---|---|---|---|
| 640 | Cl | Cl | H | $-(CH_2)_5-$ | | 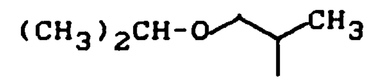 | |
| 641 | Cl | Cl | H | $-(CH_2)_5-$ | | 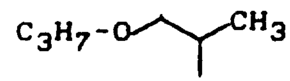 | |
| 642 | Cl | Cl | H | $-(CH_2)_5-$ | | 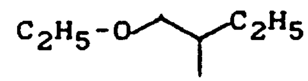 | |
| 643 | Cl | Cl | 6-Cl | H | H | $CH_3$ | |
| 644 | Cl | Cl | 6-Cl | $CH_3$ | H | $CH_3$ | |
| 645 | Cl | Cl | 6-Cl | $CH_3$ | H | $CH(CH_3)_2$ | |
| 646 | Cl | Cl | 6-Cl | $CH_3$ | H | $CH_2C(CH_3)_3$ | |
| 647 | $CH_3$ | $CH_3$ | H | H | H | $CH_3$ | |
| 648 | $CH_3$ | $CH_3$ | H | $CH_3$ | H | $CH_3$ | |
| 649 | $CH_3$ | $CH_3$ | H | $CH_3$ | H | $CH(CH_3)_2$ | |
| 650 | $CH_3$ | $CH_3$ | H | $CH_3$ | H | $CH_2C(CH_3)_3$ | |
| 651 | $CH_3$ | $CH_3$ | 6-$CH_3$ | H | H | $CH_3$ | |
| 652 | $CH_3$ | $CH_3$ | 6-$CH_3$ | H | H | $C_2H_5$ | |
| 653 | $CH_3$ | $CH_3$ | 6-$CH_3$ | H | H | $CH(CH_3)_2$ | |
| 654 | $CH_3$ | $CH_3$ | 6-$CH_3$ | H | H | 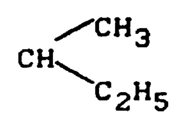 | |

<u>Tabelle 3</u> (Fortsetzung)

| Bsp.-Nr. | X | Y | $Z_n$ | A | B | $R^2$ | Fp° C |
|---|---|---|---|---|---|---|---|
| 655 | $CH_3$ | $CH_3$ | $6-CH_3$ | H | H | $CH_2-C(CH_3)_3$ | |
| 656 | $CH_3$ | CH | $6-CH_3$ | H | H | $(CH_2)_2O-C_2H_5$ | |
| 657[1)] | $CH_3$ | $CH_3$ | $6-CH_3$ | H | H | 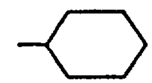 | |
| 658 | $CH_3$ | $CH_3$ | $6-CH_3$ | H | H | 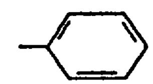 | |
| 659 | $CH_3$ | $CH_3$ | $6-CH_3$ | $CH_3$ | H | $CH_3$ | |
| 660 | $CH_3$ | $CH_3$ | $6-CH_3$ | $CH_3$ | H | $C_2H_5$ | |
| 661 | $CH_3$ | $CH_3$ | $6-CH_3$ | $CH_3$ | H | $CH(CH_3)_2$ | |
| 662 | $CH_3$ | $CH_3$ | $6-CH_3$ | $CH_3$ | H | 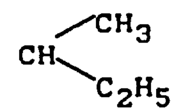 | |
| 663 | $CH_3$ | $CH_3$ | $6-CH_3$ | $CH_3$ | H | $CH_2C(CH_3)_3$ | |
| 664 | $CH_3$ | $CH_3$ | $6-CH_3$ | $CH_3$ | H | $(CH_2)_2O-C_2H_5$ | |
| 665[1)] | $CH_3$ | $CH_3$ | $6-CH_3$ | $CH_3$ | H | 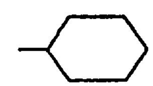 | |

<u>Tabelle 3</u> (Fortsetzung)

| Bsp.-Nr. | X | Y | $Z_n$ | A | B | $R^2$ | Fp° C |
|---|---|---|---|---|---|---|---|
| 666 | $CH_3$ | $CH_3$ | 6-$CH_3$ | $CH_3$ | H | 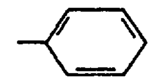 | |
| 667 | $CH_3$ | $CH_3$ | 6-$CH_3$ | $CH(CH_3)_2$ | H | $CH_3$ | |
| 668 | $CH_3$ | $CH_3$ | 6-$CH_3$ | $CH(CH_3)_2$ | H | $C_2H_5$ | |
| 669 | $CH_3$ | $CH_3$ | 6-$CH_3$ | $CH(CH_3)_2$ | H | $CH(CH_3)_2$ | |
| 670 | $CH_3$ | $CH_3$ | 6-$CH_3$ | $CH(CH_3)_2$ | H | 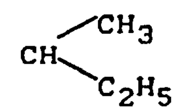 | |
| 671 | $CH_3$ | $CH_3$ | 6-$CH_3$ | $CH(CH_3)_2$ | H | $CH_2C(CH_3)_3$ | |
| 672 | $CH_3$ | $CH_3$ | 6-$CH_3$ | $CH(CH_3)_2$ | H | $(CH_2)_2O-C_2H_5$ | |
| 673 1) | $CH_3$ | $CH_3$ | 6-$CH_3$ | $CH(CH_3)_2$ | H | 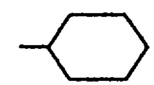 | |
| 674 | $CH_3$ | $CH_3$ | 6-$CH_3$ | $CH(CH)_2$ | H | 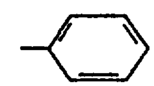 | |
| 675 | $CH_3$ | $CH_3$ | 6-$CH_3$ | $CH_2CH(CH_3)_2$ | H | $CH_3$ | |
| 676 | $CH_3$ | $CH_3$ | 6-$CH_3$ | $CH_2CH(CH_3)_2$ | H | $C_2H_5$ | |

Tabelle 3 (Fortsetzung)

| Bsp.-Nr. | X | Y | $Z_n$ | A | B | $R^2$ | Fp° C |
|---|---|---|---|---|---|---|---|
| 677 | $CH_3$ | $CH_3$ | 6-$CH_3$ | $CH_2CH(CH_3)_2$ | H | $CH(CH_3)_2$ | |
| 678 | $CH_3$ | $CH_3$ | 6-$CH_3$ | $CH_2CH(CH_3)_2$ | H | $CH_2CH(CH_3)_2$ | |
| 679 | $CH_3$ | $CH_3$ | 6-$CH_3$ | $CH_2CH(CH_3)_2$ | H | 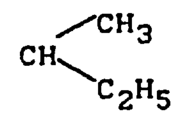 | |
| 680 | $CH_3$ | $CH_3$ | 6-$CH_3$ | $CH_2CH(CH_3)_2$ | H | $CH_2C(CH_3)_3$ | |
| 681 | $CH_3$ | $CH_3$ | 6-$CH_3$ | $CH_2CH(CH_3)_2$ | H | $(CH_2)_2O-C_2H_5$ | |
| 682 1) | $CH_3$ | $CH_3$ | 6-$CH_3$ | $CH_2CH(CH_3)_2$ | H | 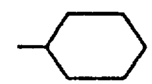 | |
| 683 | $CH_3$ | $CH_3$ | 6-$CH_3$ | $CH_2CH(CH_3)_2$ | H | 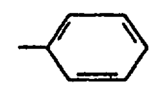 | |
| 684 | $CH_3$ | $CH_3$ | 6-$CH_3$ | $(CH_2)_2-SCH_3$ | H | $CH_3$ | |
| 685 | $CH_3$ | $CH_3$ | 6-$CH_3$ | $(CH_2)_2-SCH_3$ | H | $C_2H_5$ | |

Tabelle 3 (Fortsetzung)

| Bsp.-Nr. | X | Y | $Z_n$ | A | B | $R^2$ | Fp° C |
|---|---|---|---|---|---|---|---|
| 686 | $CH_3$ | $CH_3$ | $6-CH_3$ | $(CH_2)_2-SCH_3$ | H | $CH(CH_3)_2$ | |
| 687 | $CH_3$ | $CH_3$ | $6-CH_3$ | $(CH_2)_2-SCH_3$ | H | 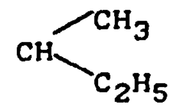 | |
| 688 | $CH_3$ | $CH_3$ | $6-CH_3$ | $(CH_2)_2-SCH_3$ | H | $CH_2C(CH_3)_3$ | |
| 689 | $CH_3$ | $CH_3$ | $6-CH_3$ | $(CH_2)_2-SCH_3$ | H | $(CH_2)_2O-C_2H_5$ | |
| 690 1) | $CH_3$ | $CH_3$ | $6-CH_3$ | $(CH_2)_2-SCH_3$ | H | 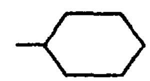 | |
| 691 | $CH_3$ | $CH_3$ | $6-CH_3$ | $(CH_2)_2-SCH_3$ | H | 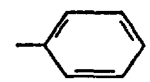 | |
| 692 | $CH_3$ | H | $6-CH_3$ | $CH_3$ | $CH_3$ | $CH_3$ | |
| 693 | $CH_3$ | $CH_3$ | $6-CH_3$ | $CH_3$ | $CH_3$ | $C_2H_5$ | 140 |
| 694 | $CH_3$ | $CH_3$ | $6-CH_3$ | $CH_3$ | $CH_3$ | $(CH_3)_2CH-$ | 161-163 |
| 695 | $CH_3$ | $CH_3$ | $6-CH_3$ | $CH_3$ | $CH_3$ | $(CH_3)_2CH-CH_2-$ | |
| 696 | $CH_3$ | $CH_3$ | $6-CH_3$ | $CH_3$ | $CH_3$ | 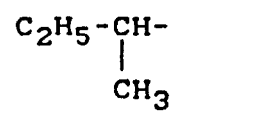 | 98 |

Tabelle 3 (Fortsetzung)

| Bsp.-Nr. | X | Y | $Z_n$ | A | B | $R^2$ | Fp° C |
|---|---|---|---|---|---|---|---|
| 697 | $CH_3$ | $CH_3$ | 6-$CH_3$ | $CH_3$ | $CH_3$ | $(CH_3)_3C-$ | |
| 698 | $CH_3$ | $CH_3$ | 6-$CH_3$ | $CH_3$ | $CH_3$ | $(CH_3)_3C-CH_2-$ | |
| 699 1) | $CH_3$ | $CH_3$ | 6-$CH_3$ | $CH_3$ | $CH_3$ | 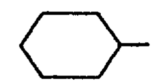 | |
| 700 | $CH_3$ | $CH_3$ | 6-$CH_3$ | $CH_3$ | $CH_3$ | 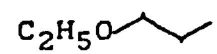 | |
| 701 | $CH_3$ | $CH_3$ | 6-$CH_3$ | $CH_3$ | $CH_3$ | 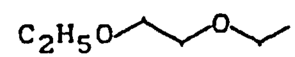 | |
| 702 | $CH_3$ | $CH_3$ | 6-$CH_3$ | $CH_3$ | $CH_3$ | 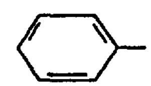 | |
| 703 | $CH_3$ | $CH_3$ | 6-$CH_3$ | $CH_3$ | $CH_3$ | 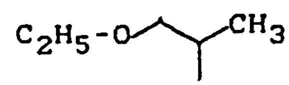 | |
| 704 | $CH_3$ | $CH_3$ | 6-$CH_3$ | $CH_3$ | $CH_3$ | 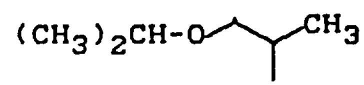 | |
| 705 | $CH_3$ | $CH_3$ | 6-$CH_3$ | $CH_3$ | $CH_3$ | 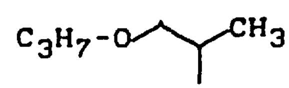 | |
| 706 | $CH_3$ | $CH_3$ | 6-$CH_3$ | $CH_3$ | $CH_3$ | 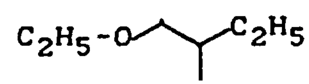 | |

Tabelle 3 (Fortsetzung)

| Bsp.-Nr. | X | Y | $Z_n$ | A | B | $R^2$ | Fp° C |
|---|---|---|---|---|---|---|---|
| 707 | $CH_3$ | H | 6-$CH_3$ | $C_2H_5$ | $CH_3$ | $CH_3$ | |
| 708 | $CH_3$ | $CH_3$ | 6-$CH_3$ | $C_2H_5$ | $CH_3$ | $C_2H_5$ | |
| 709 | $CH_3$ | $CH_3$ | 6-$CH_3$ | $C_2H_5$ | $CH_3$ | $(CH_3)_2CH-$ | |
| 710 | $CH_3$ | $CH_3$ | 6-$CH_3$ | $C_2H_5$ | $CH_3$ | $(CH_3)_2CH-CH_2-$ | |
| 711 | $CH_3$ | $CH_3$ | 6-$CH_3$ | $C_2H_5$ | $CH_3$ | $C_2H_5$-CH-<br>\|<br>$CH_3$ | |
| 712 | $CH_3$ | $CH_3$ | 6-$CH_3$ | $C_2H_5$ | $CH_3$ | $(CH_3)_3C-$ | |
| 713 | $CH_3$ | $CH_3$ | 6-$CH_3$ | $C_2H_5$ | $CH_3$ | $(CH_3)_3C-CH_2-$ | |
| 714 1) | $CH_3$ | $CH_3$ | 6-$CH_3$ | $C_2H_5$ | $CH_3$ | 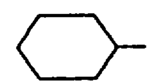 | |
| 715 | $CH_3$ | $CH_3$ | 6-$CH_3$ | $C_2H_5$ | $CH_3$ | $C_2H_5O$ 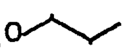 | |
| 716 | $CH_3$ | $CH_3$ | 6-$CH_3$ | $C_2H_5$ | $CH_3$ | $C_2H_5O$ 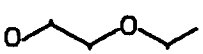 | |
| 717 | $CH_3$ | $CH_3$ | 6-$CH_3$ | $C_2H_5$ | $CH_3$ | 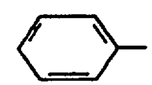 | |
| 718 | $CH_3$ | $CH_3$ | 6-$CH_3$ | $C_2H_5$ | $CH_3$ | $C_2H_5$-O 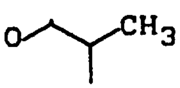 | |

<u>Tabelle 3</u> (Fortsetzung)

| Bsp.-Nr. | X | Y | $Z_n$ | A | B | $R^2$ | Fp° C |
|---|---|---|---|---|---|---|---|
| 719 | $CH_3$ | $CH_3$ | 6-$CH_3$ | $C_2H_5$ | $CH_3$ | 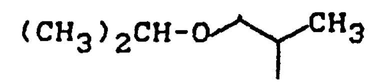 | |
| 720 | $CH_3$ | $CH_3$ | 6-$CH_3$ | $C_2H_5$ | $CH_3$ | 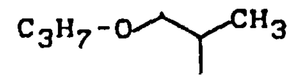 | |
| 721 | $CH_3$ | $CH_3$ | 6-$CH_3$ | $C_2H_5$ | $CH_3$ | 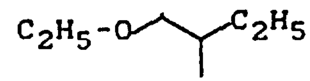 | |
| 722 | $CH_3$ | H | 6-$CH_3$ | $C_2H_5$ | $C_2H_5$ | $CH_3$ | |
| 723 | $CH_3$ | $CH_3$ | 6-$CH_3$ | $C_2H_5$ | $C_2H_5$ | $C_2H_5$ | |
| 724 | $CH_3$ | $CH_3$ | 6-$CH_3$ | $C_2H_5$ | $C_2H_5$ | $(CH_3)_2CH-$ | |
| 725 | $CH_3$ | $CH_3$ | 6-$CH_3$ | $C_2H_5$ | $C_2H_5$ | $(CH_3)_2CH-CH_2-$ | |
| 726 | $CH_3$ | $CH_3$ | 6-$CH_3$ | $C_2H_5$ | $C_2H_5$ | $C_2H_5-CH(CH_3)-$ | |
| 727 | $CH_3$ | $CH_3$ | 6-$CH_3$ | $C_2H_5$ | $C_2H_5$ | $(CH_3)_3C-$ | |
| 728 | $CH_3$ | $CH_3$ | 6-$CH_3$ | $C_2H_5$ | $C_2H_5$ | $(CH_3)_3C-CH_2-$ | |
| 729 1) | $CH_3$ | $CH_3$ | 6-$CH_3$ | $C_2H_5$ | $C_2H_5$ | 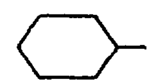 | |

Tabelle 3 (Fortsetzung)

| Bsp.-Nr. | X | Y | $Z_n$ | A | B | $R^2$ | Fp° C |
|---|---|---|---|---|---|---|---|
| 730 | $CH_3$ | $CH_3$ | 6-$CH_3$ | $C_2H_5$ | $C_2H_5$ | 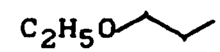 | |
| 731 | $CH_3$ | $CH_3$ | 6-$CH_3$ | $C_2H_5$ | $C_2H_5$ | 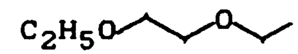 | |
| 732 | $CH_3$ | $CH_3$ | 6-$CH_3$ | $C_2H_5$ | $C_2H_5$ | 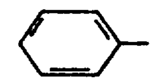 | |
| 733 | $CH_3$ | $CH_3$ | 6-$CH_3$ | $C_2H_5$ | $C_2H_5$ | 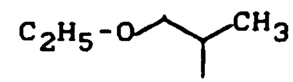 | |
| 734 | $CH_3$ | $CH_3$ | 6-$CH_3$ | $C_2H_5$ | $C_2H_5$ | 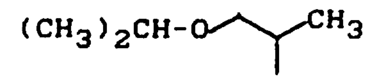 | |
| 735 | $CH_3$ | $CH_3$ | 6-$CH_3$ | $C_2H_5$ | $C_2H_5$ | 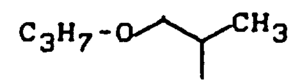 | |
| 736 | $CH_3$ | $CH_3$ | 6-$CH_3$ | $C_2H_5$ | $C_2H_5$ | 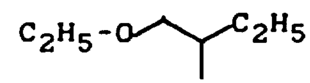 | |
| 737 | $CH_3$ | H | 6-$CH_3$ | $C_3H_7$ | $CH_3$ | $CH_3$ | |
| 738 | $CH_3$ | $CH_3$ | 6-$CH_3$ | $C_3H_7$ | $CH_3$ | $C_2H_5$ | |
| 739 | $CH_3$ | $CH_3$ | 6-$CH_3$ | $C_3H_7$ | $CH_3$ | $(CH_3)_2CH-$ | |
| 740 | $CH_3$ | $CH_3$ | 6-$CH_3$ | $C_3H_7$ | $CH_3$ | $(CH_3)_2CH-CH_2-$ | |
| 741 | $CH_3$ | $CH_3$ | 6-$CH_3$ | $C_3H_7$ | $CH_3$ | 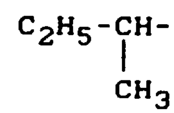 | |

Tabelle 3 (Fortsetzung)

| Bsp.-Nr. | X | Y | $Z_n$ | A | B | $R^2$ | Fp° C |
|---|---|---|---|---|---|---|---|
| 742 | $CH_3$ | $CH_3$ | 6-$CH_3$ | $C_3H_7$ | $CH_3$ | $(CH_3)_3C-$ | |
| 743 | $CH_3$ | $CH_3$ | 6-$CH_3$ | $C_3H_7$ | $CH_3$ | $(CH_3)_3C-CH_2-$ | |
| 744[1)] | $CH_3$ | $CH_3$ | 6-$CH_3$ | $C_3H_7$ | $CH_3$ | 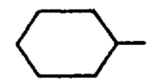 | |
| 745 | $CH_3$ | $CH_3$ | 6-$CH_3$ | $C_3H_7$ | $C_2H_5$ | 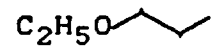 | |
| 746 | $CH_3$ | $CH_3$ | 6-$CH_3$ | $C_3H_7$ | $CH_3$ | 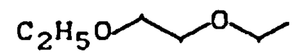 | |
| 747 | $CH_3$ | $CH_3$ | 6-$CH_3$ | $C_3H_7$ | $CH_3$ | 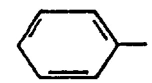 | |
| 748 | $CH_3$ | $CH_3$ | 6-$CH_3$ | $C_3H_7$ | $CH_3$ | 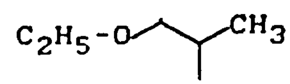 | |
| 749 | $CH_3$ | $CH_3$ | 6-$CH_3$ | $C_3H_7$ | $CH_3$ | 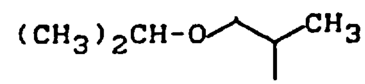 | |
| 750 | $CH_3$ | $CH_3$ | 6-$CH_3$ | $C_3H_7$ | $CH_3$ | 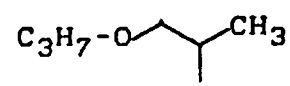 | |
| 751 | $CH_3$ | $CH_3$ | 6-$CH_3$ | $C_3H_7$ | $CH_3$ | 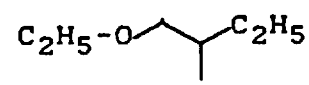 | |
| 752 | $CH_3$ | H | 6-$CH_3$ | i-$C_3H_7$ | $CH_3$ | $CH_3$ | |

Tabelle 3 (Fortsetzung)

| Bsp.-Nr. | X | Y | $Z_n$ | A | B | $R^2$ | Fp° C |
|---|---|---|---|---|---|---|---|
| 753 | $CH_3$ | $CH_3$ | 6-$CH_3$ | i-$C_3H_7$ | $CH_3$ | $C_2H_5$ | |
| 754 | $CH_3$ | $CH_3$ | 6-$CH_3$ | i-$C_3H_7$ | $CH_3$ | $(CH_3)_2CH-$ | |
| 755 | $CH_3$ | $CH_3$ | 6-$CH_3$ | i-$C_3H_7$ | $CH_3$ | $(CH_3)_2CH-CH_2-$ | |
| 756 | $CH_3$ | $CH_3$ | 6-$CH_3$ | i-$C_3H_7$ | $CH_3$ | $C_2H_5-CH-$<br>\|<br>$CH_3$ | |
| 757 | $CH_3$ | $CH_3$ | 6-$CH_3$ | i-$C_3H_7$ | $CH_3$ | $(CH_3)_3C-$ | |
| 758 | $CH_3$ | $CH_3$ | 6-$CH_3$ | i-$C_3H_7$ | $CH_3$ | $(CH_3)_3C-CH_2-$ | |
| 759 1) | $CH_3$ | $CH_3$ | 6-$CH_3$ | i-$C_3H_7$ | $CH_3$ | 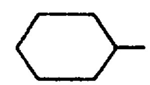 | |
| 760 | $CH_3$ | $CH_3$ | 6-$CH_3$ | i-$C_3H_7$ | $CH_3$ | 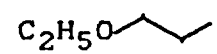 | |
| 761 | $CH_3$ | $CH_3$ | 6-$CH_3$ | i-$C_3H_7$ | $CH_3$ | 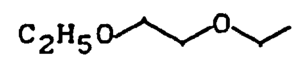 | |
| 762 | $CH_3$ | $CH_3$ | 6-$CH_3$ | i-$C_3H_7$ | $CH_3$ | 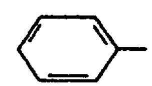 | |
| 763 | $CH_3$ | $CH_3$ | 6-$CH_3$ | i-$C_3H_7$ | $CH_3$ | 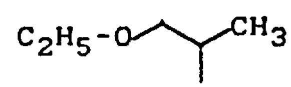 | |

Tabelle 3 (Fortsetzung)

| Bsp.-Nr. | X | Y | $Z_n$ | A | B | $R^2$ | Fp° C |
|---|---|---|---|---|---|---|---|
| 764 | $CH_3$ | $CH_3$ | 6-$CH_3$ | i-$C_3H_7$ | $CH_3$ | 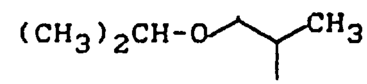 | |
| 765 | $CH_3$ | $CH_3$ | 6-$CH_3$ | i-$C_3H_7$ | $CH_3$ | 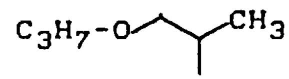 | |
| 766 | $CH_3$ | $CH_3$ | 6-$CH_3$ | i-$C_3H_7$ | $CH_3$ | 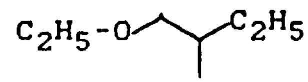 | |
| 767 | $CH_3$ | H | 6-$CH_3$ | -$(CH_2)_4$- | | $CH_3$ | |
| 768 | $CH_3$ | $CH_3$ | 6-$CH_3$ | -$(CH_2)_4$- | | $C_2H_5$ | |
| 769 | $CH_3$ | $CH_3$ | 6-$CH_3$ | -$(CH_2)_4$- | | $(CH_3)_2CH$- | |
| 770 | $CH_3$ | $CH_3$ | 6-$CH_3$ | -$(CH_2)_4$- | | $(CH_3)_2CH-CH_2$- | |
| 771 | $CH_3$ | $CH_3$ | 6-$CH_3$ | -$(CH_2)_4$- | | 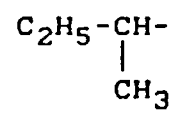 | |
| 772 | $CH_3$ | $CH_3$ | 6-$CH_3$ | -$(CH_2)_4$- | | $(CH_3)_3C$- | |
| 773 | $CH_3$ | $CH_3$ | 6-$CH_3$ | -$(CH_2)_4$- | | $(CH_3)_3C-CH_2$- | |
| 774 1) | $CH_3$ | $CH_3$ | 6-$CH_3$ | -$(CH_2)_4$- | | 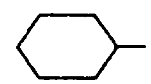 | |

Tabelle 3 (Fortsetzung)

| Bsp.-Nr. | X | Y | $Z_n$ | A | B | $R^2$ | Fp° C |
|---|---|---|---|---|---|---|---|
| 775 | $CH_3$ | $CH_3$ | 6-$CH_3$ | | $-(CH_2)_4-$ | 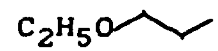 | |
| 776 | $CH_3$ | $CH_3$ | 6-$CH_3$ | | $-(CH_2)_4-$ | 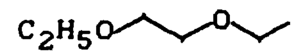 | |
| 777 | $CH_3$ | $CH_3$ | 6-$CH_3$ | | $-(CH_2)_4-$ | 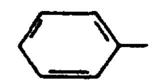 | |
| 778 | $CH_3$ | $CH_3$ | 6-$CH_3$ | | $-(CH_2)_4-$ | 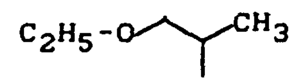 | |
| 779 | $CH_3$ | $CH_3$ | 6-$CH_3$ | | $-(CH_2)_4-$ | 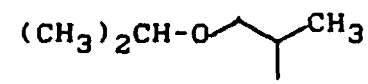 | |
| 780 | $CH_3$ | $CH_3$ | 6-$CH_3$ | | $-(CH_2)_4-$ | 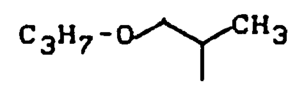 | |
| 781 | $CH_3$ | $CH_3$ | 6-$CH_3$ | | $-(CH_2)_4-$ | 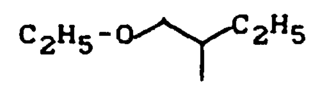 | |
| 782 | $CH_3$ | H | 6-$CH_3$ | | $-(CH_2)_5-$ | $CH_3$ | |
| 783 | $CH_3$ | $CH_3$ | 6-$CH_3$ | | $-(CH_2)_5-$ | $C_2H_5$ | |
| 784 | $CH_3$ | $CH_3$ | 6-$CH_3$ | | $-(CH_2)_5-$ | $(CH_3)_2CH-$ | |
| 785 | $CH_3$ | $CH_3$ | 6-$CH_3$ | | $-(CH_2)_5-$ | $(CH_3)_2CH-CH_2-$ | |
| 786 | $CH_3$ | $CH_3$ | 6-$CH_3$ | | $-(CH_2)_5-$ | $C_2H_5-CH-$<br>\|<br>$CH_3$ | |

Tabelle 3 (Fortsetzung)

| Bsp.-Nr. | X | Y | $Z_n$ | A | B | $R^2$ | Fp° C |
|---|---|---|---|---|---|---|---|
| 787 | $CH_3$ | $CH_3$ | $6\text{-}CH_3$ | $-(CH_2)_5-$ | | $(CH_3)_3C-$ | |
| 788 | $CH_3$ | $CH_3$ | $6\text{-}CH_3$ | $-(CH_2)_5-$ | | $(CH_3)_3C-CH_2-$ | |
| 789 [1] | $CH_3$ | $CH_3$ | $6\text{-}CH_3$ | $-(CH_2)_5-$ | | 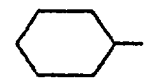 | |
| 790 | $CH_3$ | $CH_3$ | $6\text{-}CH_3$ | $-(CH_2)_5-$ | | 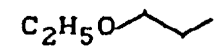 | |
| 791 | $CH_3$ | $CH_3$ | $6\text{-}CH_3$ | $-(CH_2)_5-$ | | 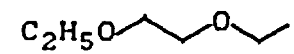 | |
| 792 | $CH_3$ | $CH_3$ | $6\text{-}CH_3$ | $-(CH_2)_5-$ | | 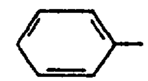 | |
| 793 | $CH_3$ | $CH_3$ | $6\text{-}CH_3$ | $-(CH_2)_5-$ | | 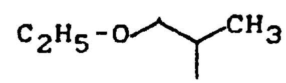 | |
| 794 | $CH_3$ | $CH_3$ | $6\text{-}CH_3$ | $-(CH_2)_5-$ | | 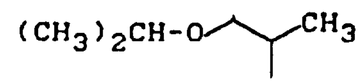 | |
| 795 | $CH_3$ | $CH_3$ | $6\text{-}CH_3$ | $-(CH_2)_5-$ | | 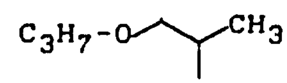 | |
| 796 | $CH_3$ | $CH_3$ | $6\text{-}CH_3$ | $-(CH_2)_5-$ | | 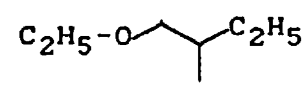 | |

[1] Diese Verbindungen gehören nicht zum beanspruchten Anmeldungsgegenstand

Beispiel (II1)

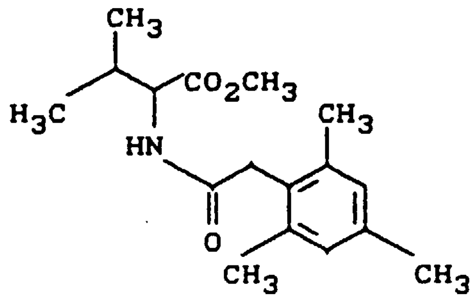

138 g (0,5 Mol) N-(2,4,6-Trimethylphenyl-acetyl)-valin werden in 500 ml Methanol suspendiert, mit 73 ml (0,55 Mol) Dimethoxypropan versetzt und nach Zugabe von 4,75 g (25 mmol) p-Toluolsulfonsäure-monohydrat und Dünnschicht-Chromatographie (DC)-Kontrolle unter Rückfluß erhitzt.

Nach Abrotieren des Lösungsmittels nimmt man den Rückstand in Methylenchlorid auf, wäscht mit Natriumhydrogencarbonat-Lösung, trocknet und rotiert ein.

Ausbeute: 127,6 g (= 88 % d.Th.)

Beispiel (IIa1)

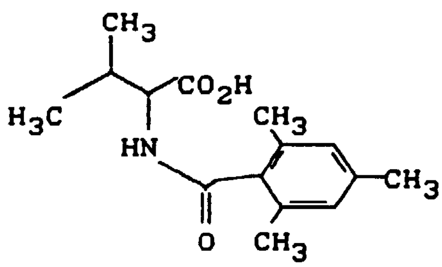

58,8 g (0,5 Mol) L-Valin in 720 ml Wasser werden mit 10 g (0,25 Mol) NaOH-Plätzchen versetzt. Anschließend werden synchron 30 g (0,75 Mol) NaOh-Plätzchen in 150 ml Wasser und 98,2 g (0,5 Mol) Mesitylenessigsäurechlorid so zugetropft, daß die Temperatur 40°C, nicht überschreitet. Nach 1 h wird bei 0-20°C mit konz. Salzsäure angesäuert, das Produkt abgesaugt und i.Vak. bei 70°C über Diphosphorpentoxid getrocknet.

Ausbeute: 138 g (= 100 % d.Th.) Fp. 140°C.

Die erfindungsgemäßen Wirkstoffe der Formel (I) eignen sich bei guter Pflanzenverträglichkeit und günstiger Warmblütertoxizität zur Bekämpfung von tierischen Schädlingen, insbesondere der Klasse Arachnida und der Ordnung Milben (Acarina), die in der Landwirtschaft, in Forsten, im Vorrats- und Materialschutz sowie auf dem Hygienesektor vorkommen. Sie sind gegen normal sensible und resistente Artn sowie gegen alle oder einzelne Entwicklungsstadien wirksam. Zu den oben erwähnten Schädlingen gehören:

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzekken, Räudemilben, Laufmilben.

Sie sind gegen normal sensible und resistente Arten und Stämme, sowie gegen alle parasitierenden und nicht parasitierenden Entwicklungsstadien der Ektoparasiten wirksam.

Die erfindungsgemäßen Wirkstoffe zeichnen sich durch eine hohe akarizide Wirksamkeit aus. Sie lassen sich mit besonders gutem Erfolg gegen pflanzenschädigende Milben, wie wie beispielsweise gegen die gemeine Spinnmilbe

(Tetranychus urticae) einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Charakteristisch für die erfindungsgemäßen Verbindungen ist, daß sie eine selektive Wirksamkeit gegen monokotyle Unkräuter im Vor- und Nachlaufverfahren (Pre- und Post-emergence) bei guter Kulturpflanzenverträglichkeit aufweisen.

Die erfindungsgemäßen Wirkstoffe können z.B. bei den folgenden Pflanzen verwendet werden:

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe neben einer hervorragenden Wirkung gegen Schadpflanzen gute Verträglichkeit gegenüber wichtigen Kulturpflanzen, wie z. B. Weizen, Baumwolle, Sojabohnen, Citrusfrüchten und Zuckerrüben, und können daher als selektive Unkrautbekämpfungsmittel eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole,

Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden Herbiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Bei den im folgenden aufgeführten biologischen Beispielen wurden folgende Verbindungen als Vergleichssubstanzen eingesetzt:

A)

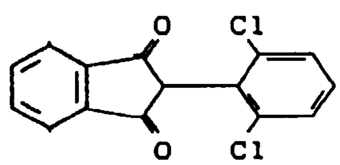

bekannt aus DE-A 2 361 084 und US-A 4 632 698

B)

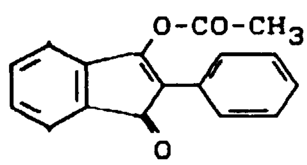

bekannt aus DE-A 2 361 084 und US-A 4 632 698

C)

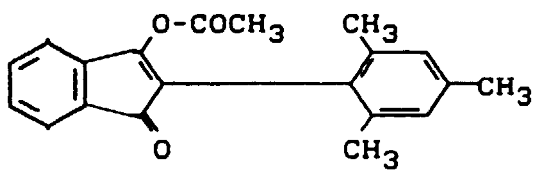

bekannt aus DE-A 2 361 084 und US-A 4 632 698

Beispiel A

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik:
(1), (2), (32), (40), (278), (280), (290), (299).

Beispiel B

Plutella-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt, Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: (1), (32), (283), (299).

Beispiel C

Nephotettix-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Neophotettix cincticepa) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik: (1), (32), (43), (290), (292), (299), (301).

Beispiel D

Pre-emergence-Test

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrollen. Es bedeuten:

0 % = keine Wirkung (wie unbehandelte Kontrolle

100 % = totale Vernichtung

Bei diesem Test zeigen z.B. die folgenden Verbindungen der Herstellungsbeispiele überlegene Wirksamkeit gegenüber dem Stand der Technik: (32), (281), (283).

Beispiel E

Post-emergence-Test

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle

100 % = totale Vernichtung

Bei diesem Test zeigen z.B. die folgenden Verbindungen der Herstellungsbeispiele überlegene Wirksamkeit gegenüber dem Stand der Technik: (32), (281), (283).

Beispiel F

Tetranychus-Test (OP-resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe oder Bohnenspinnmilbe (Tetranychus urticae) befallen sind, werden mit einer Wirkstoffzubereitung der gewünschten Konzentration tropfnaß gespritzt.

Nach der gewünschten Zeit wird die Wirkung in X bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: (281), (283).

## Patentansprüche

**1.** 3-Aryl-pyrrolidin-2,4-dion-Derivate der Formel (I)

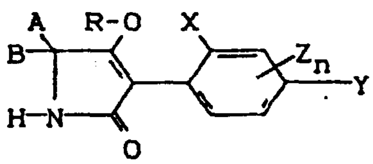

(I)

in welcher

X für $C_1$-$C_6$-Alkyl, Halogen, $C_1$-$C_6$-Alkoxy steht,

Y für Wasserstoff, $C_1$-$C_6$-Alkyl, Halogen, $C_1$-$C_6$-Alkoxy, $C_1$-$C_3$-Halogenalkyl steht,

Z für $C_1$-$C_6$-Alkyl, Halogen, $C_1$-$C_6$-Alkoxy steht,

n für eine Zahl von 0-3 steht,

R für Wasserstoff (Ia) oder für die Gruppen der Formel

$-CO-R^1$ (Ib) oder $-CO-O-R^2$ (Ic) oder $E^{\oplus}$ (Id)

steht, in welchen

$R^1$ für gegebenenfalls durch Halogen substituiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxy-$C_2$-$C_8$-alkyl und Cycloalkyl mit 3-8 Ringatomen,
das durch Sauerstoff und/oder Schwefel unterbrochen sein kann, steht,
für gegebenenfalls durch Halogen, Nitro, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl;
für gegebenenfalls durch Halogen, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl-$C_1$-$C_6$-alkyl steht,
für gegebenenfalls durch Halogen und $C_1$-$C_6$-Alkyl substituiertes Phenoxy-$C_1$-$C_6$-alkyl steht,

$R^2$ für gegebenenfalls durch Halogen substituiertes $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,
für gegebenenfalls durch Halogen, Nitro, $C_1$-$C_6$-Alkyl, $C_1$-$C_6$-Alkoxy, $C_1$-$C_6$-Halogenalkyl substituiertes Phenyl
steht,

A für Wasserstoff oder gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen steht, das durch Sauerstoff und/oder Schwefel unterbrochen sein kann,

B für Wasserstoff, geradkettiges oder verzweigtes $C_1$-$C_{12}$-Alkyl, $C_1$-$C_8$-Alkoxyalkyl steht, oder

A und Bgemeinsam mit dem Kohlenstoffatom an das sie gebunden sind einen 3-8 gliedrigen Ring bilden oder

$E^{\oplus}$ für einen Metallionenäquivalent oder ein Ammoniumion steht
sowie die enantiomerenreinen Formen von Verbindungen der Formel (I).

**2.** 3-Aryl-pyrrolidin-2,4-dion-Derivat der Formel (I) gemäß Anspruch 1, in welcher

X für $C_1$-$C_4$-Alkyl, Halogen, $C_1$-$C_4$-Alkoxy steht,

Y für Wasserstoff, $C_1$-$C_6$-Alkyl, Halogen, $C_1$-$C_4$-Alkoxy, $C_1$-$C_2$-Halogenalkyl steht,

Z für $C_1$-$C_4$-Alkyl, Halogen, $C_1$-$C_4$-Alkoxy steht,

n für eine Zahl von 0-3 steht,

R für Wasserstoff (Ia) oder für die Gruppen der Formel

$$-CO-R^1 \text{ (Ib)}, -CO-O-R^2 \text{ (Ic) oder } E^{\oplus} \text{ (Id)}$$

steht, in welchen

$R^1$ für gegebenenfalls durch Halogen substituiertes $C_1$-$C_{16}$-Alkyl, $C_2$-$C_{16}$-Alkenyl, $C_1$-$C_6$-Alkoxy-$C_2$-$C_6$-alkyl, $C_1$-$C_6$-Alkylthio-$C_2$-$C_6$-alkyl, $C_1$-$C_6$-Polyalkoxy-$C_2$-$C_6$-alkyl und Cycloalkyl mit 3-7 Ringatomen, das durch 1-2 Sauerstoff-und/oder Schwefelatome unterbrochen sein kann steht,
für gegebenenfalls durch Halogen, Nitro, $C_1$-$C_4$-Alkyl, $C_1$-$C_4$-Alkoxy, $C_1$-$C_3$-Halogenalkyl, $C_1$-$C_3$-Halogenalkoxy substituiertes Phenyl steht,
für gegebenenfalls durch Halogen, $C_1$-$C_4$-Alkyl, $C_1$-$C_4$-Alkoxy, $C_1$-$C_3$-Halogenalkyl, $C_1$-$C_3$-Halogenalkoxy substituiertes Phenyl-$C_1$-$C_4$-alkyl steht,
gegebenenfalls für durch Halogen und $C_1$-$C_4$-Alkyl substituiertes Phenoxy-$C_1$-$C_5$-alkyl steht,

$R^2$ für gegebenenfalls durch Halogen substituiertes $C_1$-$C_{16}$-Alkyl, $C_2$-$C_{16}$-Alkenyl, $C_1$-$C_{16}$-Alkoxy-$C_2$-$C_6$-alkyl, $C_1$-$C_6$-Polyalkoxy-$C_2$-$C_6$-alkyl steht,
für gegebenenfalls durch Halogen, Nitro, $C_1$-$C_4$-Alkyl, $C_1$-$C_3$-Alkoxy, $C_1$-$C_3$-Halogenalkyl substituiertes Phenyl steht,

A für Wasserstoff, gegebenenfalls durch Halogen substituiertes geradkettiges oder verzweigtes $C_1$-$C_{10}$-Alkyl, $C_3$-$C_6$-Alkenyl, $C_3$-$C_6$-Alkinyl, $C_1$-$C_8$-Alkoxy-$C_2$-$C_6$-alkyl, $C_1$-$C_6$-Polyalkoxy-$C_2$-$C_6$-alkyl, $C_1$-$C_8$-Alkylthio-$C_2$-$C_6$-alkyl, Cycloalkyl mit 3-7 Ringatomen steht, das durch 1-2 Sauerstoff-und/oder Schwefelatome unterbrochen sein kann,

B für Wasserstoff, geradkettiges oder verzweigtes $C_1$-$C_{10}$-Alkyl, $C_1$-C Alkoxyalkyl steht oder

A und Bgemeinsam mit dem Kohlenstoffatom an das sie gebunden sind einen 3-7-gliedrigen Ring bilden und

$E^{\oplus}$ für ein Metallionenäquivalent oder ein Ammoniumion steht,
sowie die enantiomerenreinen Formen von Verbindungen der Formel (I).

**3.** 3-Aryl-pyrrolidin-2,4-dion-Derivate der Formel (I) gemäß Anspruch 1, in welcher

X für Methyl, Ethyl, Propyl, i-Propyl, Fluor, Chlor, Brom, Methoxy und Ethoxy steht,

Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,

Z für Methyl, Ethyl, i-Propyl, Butyl, i-Butyl, tert.-Butyl, Fluor, Chlor, Brom, Methoxy und Ethoxy steht,

n für eine Zahl von 0-3 steht,

R für Wasserstoff (Ia) oder für die Gruppen der Formel

$$-CO-R^1 \text{ (Ib)}, -CO-O-R^2 \text{ (Ic) oder } E^{\oplus} \text{ (Id)}$$

steht, in welcher

$R^1$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Alkylthio-$C_2$-$C_6$-alkyl, $C_1$-$C_4$-Polyalkoxyl-$C_2$-$C_4$-alkyl und Cycloalkyl mit 3-6 Ringatomen, das durch 1-2 Sauerstoff-und/oder Schwefelatome unterbrochen sein kann steht,
für gegebenenfalls durch Fluor, Chlor, Brom, Methyl, Ethyl, Propyl, i-Propyl, Methoxy, Ethoxy, Trifluormethyl-, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-$C_1$-$C_3$-alkyl steht,
für gegebenenfalls durch Fluor, Chlor, Methyl, Ethyl substituiertes Phenoxy-$C_1$-$C_4$-alkyl steht,

$R^2$ für gegebenenfalls durch Fluor oder Chlor substituiertes $C_1$-$C_{14}$-Alkyl, $C_2$-$C_{14}$-Alkenyl, $C_1$-$C_4$-Alkoxy-$C_2$-$C_6$-alkyl, $C_1$-$C_4$-Polyalkoxy-$C_2$-$C_6$-alkyl steht
oder
für gegebenenfalls durch Fluor, Chlor, Nitro, Methyl, Ethyl, Propyl, i-Propyl, Methoxy, Ethoxy, Trifluormethyl substituiertes Phenyl steht,

A für Wasserstoff, gegebenenfalls durch Halogen substituiertes geradkettiges oder verzweigtes $C_1$-$C_8$-Alkyl, $C_3$-$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$-$C_4$-alkyl, Cycloalkyl mit 3-6 Ringatomen steht, das durch 1-2 Sauerstoff-und/oder Schwefelatomen unterbrochen sein kann,

B für Wasserstoff, geradkettiges oder verzweigtes $C_1$-$C_8$-Alkyl, $C_1$-$C_4$-Alkoxyalkyl steht, oder

A und Bgemeinsam mit dem Kohlenstoffatom, an das sie gebunden sind ein 3-6 gliedrigen Ring bilden, und

$E^{\oplus}$ für ein Metallionenäquivalent oder ein Ammoniumion steht

sowie die enantiomerenreinen Formen von Verbindungen der Formel I.

**4.** Verfahren zur Herstellung von 3-Aryl-pyrrolidin-2,4-dion-Derivaten der Formel (I) gemäß Anspruch 1,
dadurch gekennzeichnet,
daß man zum Erhalt von 3-Aryl-pyrrolidin-2,4-dionen bzw. deren Enolen der Formel (Ia)

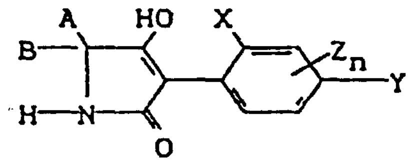

(Ia)

in welcher A, B, C, X, Y, Z und n die in Anspruch 1 angegebene Bedeutung haben,
(A)
N-Acylaminosäureester der Formel (II)

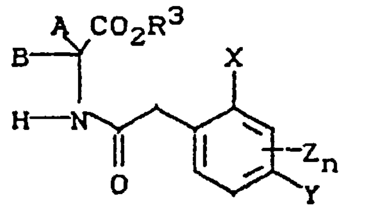

(II)

in welcher
A, B, X, Y, Z und n die oben angegebene Bedeutung haben
und

$R^3$ für Alkyl steht,

in Gegenwart eines Verdünnungsmittels und in Gegenwart einer Base intramolekular kondensiert,
(B)
oder daß man zum Erhalt von Verbindungen der Formel (Ib)

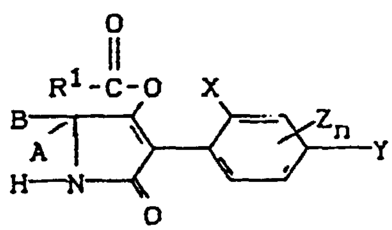

(Ib)

in welcher A, B, X, Y, Z, $R^1$ und n die in Anpruch 1 angegebene Bedeutung haben,
Verbindungen der Formel (Ia),

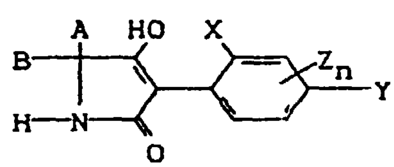

(Ia)

in welcher
A, B, X, Y, Z und n die oben angegebene Bedeutung haben,

α) mit Säurehalogeniden der allgemeinen Formel (III)

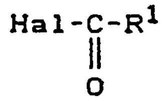

(III)

in welcher

$R^1$ die oben angegebene Bedeutung hat

und

Hal für Halogen, insbesondere Chlor und Brom steht,

gegebenenfalls in Gegenwart eines Verdünnungsmittels und gegebenenfalls in Gegenwart eines Säurebindemittels,
oder

β) mit Carbonsäureanhydriden der allgemeinen Formel (IV)

$$R^1\text{-CO-O-CO-}R^1 \qquad \text{(IV)}$$

in welcher

$R^1$ die oben angegebene Bedeutung hat,

gegebenenfalls in Gegenwart eines Verdünnungsmittels und gegebenenfalls in Gegenwart eines Säurebindemittels,

umsetzt,
(C)
oder daß man zum Erhalt von Verbindungen der Formel (Ic)

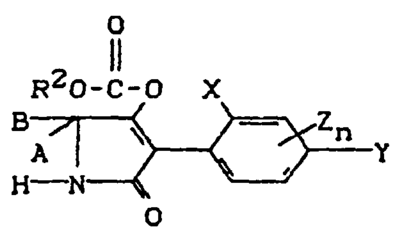

(Ic)

in welcher
A, B, C, X, Y, Z, $R^2$ und n die in Anspruch 1 angegebene Bedeutung haben,
Verbindungen der Formel (Ia)

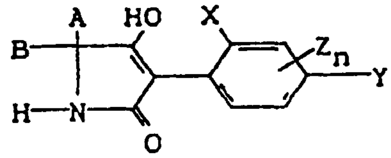

(Ia)

in welcher

A, B, X, Y, Z und n die oben angegebene Bedeutung haben, mit Chlorameisensäureester der allgemeinen Formel (V)

$$R^2\text{-O-CO-Cl} \qquad (V)$$

in welcher.

$R^2$ die oben angegebene Bedeutung hat,

gegebenenfals in Gegenwart eines Verdünnungsmittels und gegebenenfalls in Gegenwart eines Säurebindemittels umsetzt,
D)
oder daß man zum Erhalt von Verbindungen der Formel (Id)

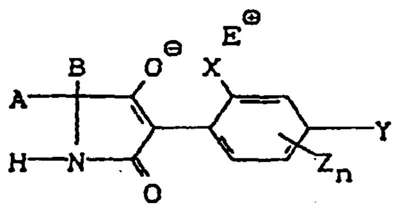

(Id)

in welcher X, Y, Z, A, B und n die in Anspruch 1 angegebene Bedeutung haben, Verbindungen der Formel (Ia)

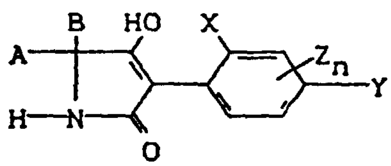

(Ia)

in welcher X, Y, Z, A, B und n die oben angegebene Bedeutung haben, mit Metallhydroxiden oder Aminen der allgemeinen Formeln (VI) und (VII)

$Me_sOH_t$ (VI)

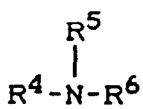

(VII)

in welchen

Me für ein- oder zweiwertige Metallionen,

s und t für die Zahl 1 und 2 und
$R^4$, $R^5$ und $R^6$ unabhängig voneinander für Wasserstoff und
Alkyl
stehen,
gegebenenfalls in Gegenwart eines Verdünnungsmittels, umsetzt.

**5.** Insektizide, akarizide und herbizide Mittel, gekennzeichnet durch einen Gehalt an mindestens einem 3-Aryl-pyrrolidin-2,4-dion-Derivat der Formel (I).

**6.** Verfahren zur Bekämpfung von Insekten und/oder Spinnentieren und/oder Unkräutern, dadurch gekennzeichnet, daß man 3-Aryl-pyrrolidin-2,4-dion-Derivate der Formel (I) auf Insekten und/oder Spinnentieren und/oder Unkräutern und/oder deren Lebensraum einwirken läßt.

**7.** Verwendung von 3-Aryl-pyrrolidin-2,4-dion-Derivaten der Formel (I) zur Bekämpfung von Insekten und/oder Spinnentieren und/oder Unkräutern.

**8.** Verfahren zur Herstellung von insektiziden und/oder akariziden und/oder herbiziden Mitteln, dadurch gekennzeichnet, daß man 3-Aryl-pyrrolidin-2,4-dion-Derivate der Formel (I) mit Streckmitteln und/oder oberflächenaktiven Mitteln vermischt.

## Claims

**1.** 3-Aryl-pyrrolidine-2,4-dione derivatives of the formula (I)

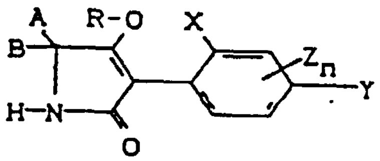

(I)

in which

X represents $C_1$-$C_6$-alkyl, halogen or $C_1$-$C_6$-alkoxy,

Y represents hydrogen, $C_1$-$C_6$-alkyl, halogen or $C_1$-$C_6$-alkoxy or $C_1$-$C_3$-halogenoalkyl,

Z represents $C_1$-$C_6$-alkyl, halogen or $C_1$-$C_6$-alkoxy,

n represents a number from 0-3,

R represents hydrogen (Ia), or represents the groups of the formula

$$-CO-R^1 \text{ (Ib) or } -CO-O-R^2 \text{ (Ic) or } E^{\oplus} \text{ (Id)}$$

in which

$R^1$ represents $C_1$-$C_{20}$-alkyl, $C_2$-$C_{20}$-alkenyl, $C_1$-$C_8$-alkoxy-$C_2$-$C_8$-alkyl,$C_1$-$C_8$-alkylthio-$C_2$-$C_8$-alkyl, $C_1$-$C_8$-polyalkoxy-$C_2$-$C_8$-alkyl and cycloalkyl which has 3-8 ring atoms and which can be interrupted by oxygen and/or sulphur, each of these substituents being optionally substituted by halogen,
or represents phenyl which is optionally substituted by halogen, nitro, $C_1$-$C_6$-alkyl, $C_1$-$C_6$-alkoxy, $C_1$-$C_6$-halogenoalkyl or $C_1$-$C_6$-halogenoalkoxy;
or represents phenyl-$C_1$-$C_6$-alkyl which is optionally substituted by halogen, $C_1$-$C_6$-alkyl, $C_1$-$C_6$-alkoxy, $C_1$-$C_6$-halogenoalkyl or $C_1$-$C_6$-halogenoalkoxy,
or represents phenoxy-$C_1$-$C_6$-alkyl which is optionally substituted by halogen and $C_1$-$C_6$-alkyl,

$R^2$ represents $C_1$-$C_{20}$-alkyl, $C_2$-$C_{20}$-alkenyl, $C_1$-$C_8$-alkoxy-$C_2$-$C_8$-alkyl or $C_1$-$C_8$-polyalkoxy-$C_2$-$C_8$-alkyl each of which is optionally substituted by halogen,
or represents phenyl which is optionally substituted by halogen, nitro, $C_1$-$C_6$-alkyl, $C_1$-$C_6$-alkoxy or $C_1$-$C_6$-halogenoalkyl,

A represents hydrogen or represents straight-chain or branched $C_1$-$C_{12}$-alkyl, $C_3$-$C_8$-alkenyl, $C_3$-$C_8$-alkinyl, $C_1$-

$C_{10}$-alkoxy-$C_2$-$C_8$-alkyl, $C_1$-$C_8$-polyalkoxy-$C_2$-$C_8$-alkyl, $C_1$-$C_{10}$-alkylthio-$C_2$-$C_8$-alkyl, cycloalkyl which has 3-8 ring atoms and which can be interrupted by oxygen and/or sulphur, each of these substituents being optionally substituted by halogen,

B represents hydrogen or straight-chain or branched $C_1$-$C_{12}$-alkyl or $C_1$-$C_8$-alkoxyalkyl, or

A and Btogether with the carbon atom to which they are bonded form a 3-8 membered ring, or

$E^{\oplus}$ represents a metal ion equivalent or an ammonium ion, and the pure enantiomeric forms of compounds of the formula (I).

**2.** 3-Aryl-pyrrolidine-2,4-dione derivative of the formula (I) according to Claim 1, in which

X represents $C_1$-$C_4$-alkyl, halogen, or $C_1$-$C_4$-alkoxy,

Y represents hydrogen, $C_1$-$C_6$-alkyl, halogen, $C_1$-$C_4$-alkoxy or $C_1$-$C_2$-halogenoalkyl,

Z represents $C_1$-$C_4$-alkyl, halogen or $C_1$-$C_4$-alkoxy,

n represents a number from 0-3,

R represents hydrogen (Ia) or represents the groups of the formula

$$-CO-R^1 \text{ (Ib)}, -CO-O-R^2 \text{ (Ic) or } E^{\oplus} \text{ (Id)}$$

in which

$R^1$ represents $C_1$-$C_{16}$-alkyl, $C_2$-$C_{16}$-alkenyl, $C_1$-$C_6$-alkoxy-$C_2$-$C_6$-alkyl, $C_1$-$C_6$-alkylthio-$C_2$-$C_6$-alkyl, $C_1$-$C_6$-polyalkoxy-$C_2$-$C_6$-alkyl and cycloalkyl which has 3-7 ring atoms and which can be interrupted by 1-2 oxygen and/or sulphur atoms, each of these substituents being optionally substituted by halogen,
or represents phenyl which is optionally substituted by halogen, nitro, $C_1$-$C_4$-alkyl, $C_1$-$C_4$-alkoxy, $C_1$-$C_3$-halogenoalkyl or $C_1$-$C_3$-halogenoalkoxy,
or represents phenyl-$C_1$-$C_4$-alkyl which is optionally substituted by halogen, $C_1$-$C_4$-alkyl, $C_1$-$C_4$-alkoxy, $C_1$-$C_3$-halogenoalkyl or $C_1$-$C_3$-halogenoalkoxy,
optionally represents phenoxy-$C_1$-$C_5$-alkyl which is substituted by halogen and $C_1$-$C_4$-alkyl,

$R^2$ represents $C_1$-$C_{16}$-alkyl, $C_2$-$C_{16}$-alkenyl, $C_1$-$C_{16}$-alkoxy-$C_2$-$C_6$-alkyl or $C_1$-$C_6$-polyalkoxy-$C_2$-$C_6$-alkyl each of which is optionally substituted by halogen,
or represents phenyl which is optionally substituted by halogen, nitro, $C_1$-$C_4$-alkyl, $C_1$-$C_3$-alkoxy or $C_1$-$C_3$-halogenoalkyl,

A represents hydrogen or straight-chain or branched $C_1$-$C_{10}$-alkyl, $C_3$-$C_6$-alkenyl, $C_3$-$C_6$-alkinyl, $C_1$-$C_8$-alkoxy-$C_2$-$C_6$-alkyl, $C_1$-$C_6$-polyalkoxy-$C_2$-$C_6$-alkyl, $C_1$-$C_8$-alkylthio-$C_2$-$C_6$-alkyl, cycloalkyl which has 3-7 ring atoms and which can be interrupted by 1-2 oxygen and/or sulphur atoms, each of these substituents being optionally substituted by halogen,

B represents hydrogen, straight-chain or branched $C_1$-$C_{10}$-alkyl, $C_1$-C alkoxyalkyl or

A and Btogether with the carbon atom to which they are bonded form a 3-7-membered ring and

$E^{\oplus}$ represents a metal ion equivalent or an ammonium ion, and the pure enantiomeric forms of compounds of the formula (I).

**3.** 3-Aryl-pyrrolidine-2,4-dione derivatives of the formula (I) according to Claim 1, in which

X represents methyl, ethyl, propyl, i-propyl, fluorine, chlorine, bromine, methoxy and ethoxy,

Y represents hydrogen, methyl, ethyl, propyl, i-propyl, butyl, i-butyl, tert.-butyl, fluorine, chlorine, bromine, methoxy, ethoxy and trifluoromethyl,

Z represents methyl, ethyl, i-propyl, butyl, i-butyl, tert.-butyl, fluorine, chlorine, bromine, methoxy and ethoxy,

n represents a number from 0-3,

R represents hydrogen (Ia) or represents the groups of the formula

$$-CO-R^1 \text{ (Ib)}, \qquad -CO-O-R^2 \text{ (Ic)} \qquad \text{or} \qquad E^{\oplus} \text{ (Id)}$$

in which

$R^1$ represents $C_1$-$C_{14}$-alkyl, $C_2$-$C_{14}$-alkenyl, $C_1$-$C_4$-alkoxy-$C_2$-$C_6$-alkyl,$C_1$-$C_4$-alkylthio-$C_2$-$C_6$-alkyl, $C_1$-$C_4$-polyalkoxyl-$C_2$-$C_4$-alkyl and cycloalkyl which has 3-6 ring atoms and which can be interrupted by 1-2 oxygen and/or sulphur atoms, each of these substituents being optionally substituted by fluorine or chlorine,
or represents phenyl which is optionally substituted by fluorine, chlorine, bromine, methyl, ethyl, propyl, i-propyl, methoxy, ethoxy, trifluoromethyl, trifluoromethoxy or nitro,
or represents phenyl-$C_1$-$C_3$-alkyl which is optionally substituted by fluorine, chlorine, bromine, methyl, ethyl, propyl, i-propyl, methoxy, ethoxy, trifluoromethyl or trifluoromethoxy,
or represents phenoxy-$C_1$-$C_4$-alkyl which is optionally substituted by fluorine, chlorine, methyl or ethyl,

$R^2$ represents $C_1$-$C_{14}$-alkyl, $C_2$-$C_{14}$-alkenyl, $C_1$-$C_4$-alkoxy-$C_2$-$C_6$-alkyl or $C_1$-$C_4$-polyalkoxy-$C_2$-$C_6$-alkyl each of which is optionally substituted by fluorine or chlorine,
or
represents phenyl which is optionally substituted by fluorine, chlorine, nitro, methyl, ethyl, propyl, i-propyl, methoxy, ethoxy or trifluoromethyl,

A represents hydrogen, or straight-chain or branched $C_1$-$C_8$-alkyl, $C_3$-$C_4$-alkenyl, $C_3$-$C_4$-alkinyl, $C_1$-$C_6$-alkoxy-$C_2$-$C_4$-alkyl, $C_1$-$C_4$-polyalkoxy-$C_2$-$C_4$-alkyl, $C_1$-$C_6$-alkylthio-$C_2$-$C_4$-alkyl, cycloalkyl which has 3-6 ring atoms and which can be interrupted by 1-2 oxygen and/or sulphur atoms, each of these substituents being optionally substituted by halogen,

B represents hydrogen or straight-chain or branched $C_1$-$C_8$-alkyl or $C_1$-$C_4$-alkoxyalkyl, or

A and Btogether with the carbon atom to which they are bonded form a 3-6 membered ring, and

$E^{\oplus}$ represents a metal ion equivalent or an ammonium ion,
and the pure enantiomeric forms of compounds of the formula (I).

**4.** Process for the preparation of 3-aryl-pyrrolidine-2,4-dione derivatives of the formula (I) according to Claim 1 characterized in that,
to obtain 3-aryl-pyrrolidine-2,4-diones or their enols of the formula (Ia)

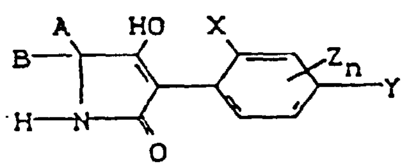

(Ia)

in which A, B, C, X, Y, Z and n have the meaning mentioned in Claim 1
(A)
N-acylamino acid esters of the formula (II)

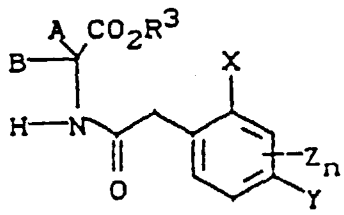

(II)

in which
A, B, X, Y, Z and n have the abovementioned meaning
and

$R^3$ represents alkyl

are subjected to intramolecular condensation in the presence of a diluent and in the presence of a base,
(B)
or in that, to obtain compounds of the formula (Ib)

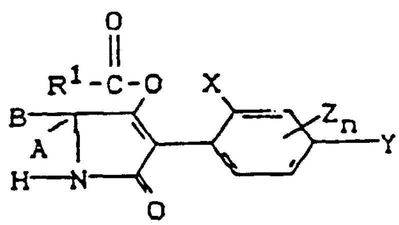

(Ib)

in which A, B, X, Y, Z, $R^1$ and n have the meaning mentioned in Claim 1,
compounds of the formula (Ia),

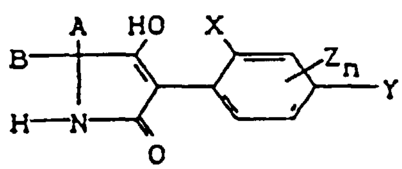

(Ia)

in which
A, B, X, Y, Z and n have the abovementioned meaning
are reacted

α) with acid halides of the general formula (III)

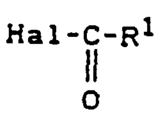

(III)

in which
$R^1$ has the abovementioned meaning
and

Hal represents halogen, in particular chlorine and bromine,

if appropriate in the presence of a diluent and if appropriate in the presence of an acid-binding agent, or

β) with carboxylic anhydrides of the general formula (IV)

$$R^1\text{-CO-O-CO-}R^1 \qquad (IV)$$

in which

$R^1$ has the abovementioned meaning,

if appropriate in the presence of a diluent and if appropriate in the presence of an acid-binding agent,

(C)
or in that, to obtain compounds of the formula (Ic)

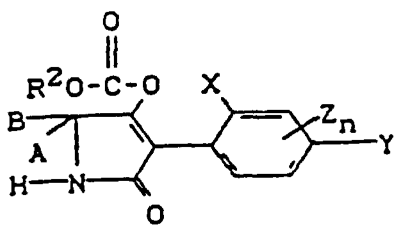

(Ic)

in which
A, B, C, X, Y, Z, $R^2$ and n have the meaning mentioned in Claim 1, compounds of the formula (Ia)

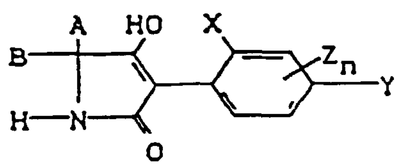

(Ia)

in which
A, B, X, Y, Z and n have the abovementioned meaning
are reacted with chloroformic ester of the general formula (V)

$$R^2\text{-O-CO-Cl} \qquad (V)$$

in which

$R^2$ has the abovementioned meaning,

if appropriate in the presence of a diluent and if appropriate in the presence of an acid-binding agent.
D)
or that, to obtain compounds of the formula (Id)

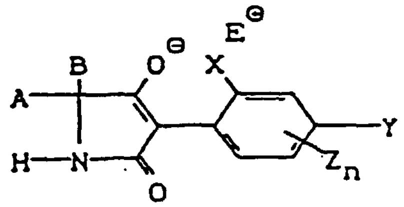

(Id)

in which X, Y, Z, A, B and n have the meaning mentioned in Claim 1,
compounds of the formula (Ia)

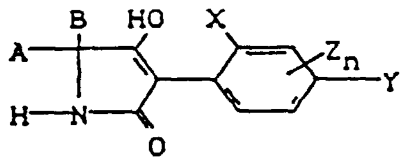

(Ia)

in which X, Y, Z, A, B and n have the abovementioned meaning
are reacted with metal hydroxides or amines of the general formulae (VI) and (VII)

$Me_sOH_t$ (VI)

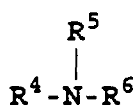

(VII)

in which
Me represents monovalent or divalent metal ions,
s and t represent the number 1 and 2 and

$R^4$, $R^5$ and $R^6$ independently of one another represent hydrogen and alkyl,

if appropriate in the presence of a diluent.

5. Insecticidal, acaricidal and herbicidal agents, characterized in that they contain at least one 3-aryl-pyrrolidine-2,4-dione derivative of the formula (I).

6. Method of combating insects and/or Arachnida and/or weeds, characterized in that 3-aryl-pyrrolidine-2,4-dione derivatives of the formula (I) are allowed to act on insects and/or Arachnida and/or weeds and/or their environment.

7. Use of 3-aryl-pyrrolidine-2,4-dione derivatives of the formula (I) for combating insects and/or Arachnida and/or weeds.

8. Process for the preparation of insecticidal and/or acaricidal and/or herbicidal agents, characterized in that 3-aryl-pyrrolidine-2,4-dione derivatives of the formula (I) are mixed with extenders and/or surface-active agents.

**Revendications**

1. Dérivés de 3-arylpyrrolidine-2,4-diones de formule (I)

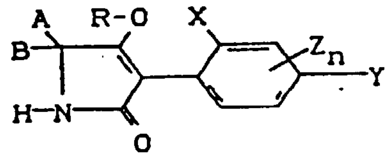

(I)

dans laquelle

X représente un groupe alkyle en $C_1$ à $C_6$, un halogène, un groupe alkoxy en $C_1$ à $C_6$,
Y représente de l'hydrogène, un groupe alkyle en $C_1$ à $C_6$, un halogène, un groupe alkoxy en $C_1$ à $C_6$, halogénalkyle en $C_1$ à $C_3$,
Z est un groupe alkyle en $C_1$ à $C_6$, un halogène, un groupe alkoxy en $C_1$ à $C_6$,
n est un nombre de 0 à 3,
R représente de l'hydrogène (Ia) ou les groupes de formule

$-CO-R^1$ (Ib) ou $-CO-O-R^2$ (Ic) ou bien $E^{\oplus}$ (Id)

où

$R^1$ est un groupe alkyle en $C_1$ à $C_{20}$ éventuellement substitué par un halogène, alcényle en $C_2$ à $C_{20}$, (alkoxy en $C_1$ à $C_8$)-(alkyle en $C_2$ à $C_8$), (alkyle en $C_1$ à $C_8$)-thio-(alkyle en $C_2$ à $C_8$), poly (alkoxy en $C_1$ à $C_8$)-(alkyle en $C_2$ à $C_8$) et cycloalkyle à noyau de 3 à 8 atomes, qui peut être interrompu par de l'oxygène et/ou du soufre,
un groupe phényle éventuellement substitué par un radical halogéno, nitro, alkyle en $C_1$ à $C_6$, alkoxy en $C_1$ à $C_6$, halogénalkyle en $C_1$ à $C_6$, halogénalkoxy en $C_1$ à $C_6$ ;
un groupe phényl-(alkyle en $C_1$ à $C_6$) éventuellement substitué par un halogène, un radical alkyle en $C_1$ à $C_6$, alkoxy en $C_1$ à $C_6$, halogénalkyle en $C_1$ à $C_6$, halogénalkoxy en $C_1$ à $C_6$,
un groupe phénoxy-(alkyle en $C_1$ à $C_6$) éventuellement substitué par un halogène et un radical alkyle en $C_1$ à $C_6$,
$R^2$ est un groupe alkyle en $C_1$ à $C_{20}$ éventuellement substitué par un halogène, un groupe alcényle en $C_2$ à $C_{20}$, (alkoxy en $C_1$ à $C_8$)-(alkyle en $C_2$ à $C_8$), poly(alkoxy en $C_1$ à $C_8$)-(alkyle en $C_2$ à $C_8$), un groupe phényle éventuellement substitué par un halogène, un radical nitro, alkyle en $C_1$ à $C_6$, alkoxy en $C_1$ à $C_6$, halogénalkyle en $C_1$ à $C_6$,

A représente de l'hydrogène ou un groupe alkyle en $C_1$ à $C_{12}$ linéaire ou ramifié éventuellement substitué par un halogène, un groupe alcényle en $C_3$ à $C_8$, alcynyle en $C_3$ à $C_8$, (alkoxy en $C_1$ à $C_{10}$)-(alkyle en $C_2$ à $C_8$), poly(alkoxy en $C_1$ à $C_8$)-(alkyle en $C_2$ à $C_8$), (alkylthio en $C_1$ à $C_{10}$)-(alkyle en $C_2$ à $C_8$), cycloalkyle à noyau de 3 à 8 atomes, qui peut être interrompu par de l'oxygène et/ou du soufre,
B est de l'hydrogène, un groupe alkyle en $C_1$ à $C_{12}$ linéaire ou ramifié, un groupe (alkoxy en $C_1$ à $C_8$)-alkyle, ou bien
A et Bforment un noyau de 3 à 8 chaînons conjointement avec l'atome d'azote auquel ils sont liés, ou bien
$E^{\oplus}$ est un équivalent d'ion métallique ou un ion ammonium,

ainsi que les formes énantiomères pures de composés de formule (I).

2. Dérivé de 3-arylpyrrolidine-2,4-diones de formule (I) suivant la revendication 1, dans laquelle

X est un groupe alkyle en $C_1$ à $C_4$, un halogène, un groupe alkoxy en $C_1$ à $C_4$,
Y est de l'hydrogène, un groupe alkyle en $C_1$ à $C_6$, un halogène, un groupe alkoxy en $C_1$ à $C_4$, halogénalkyle en $C_1$ ou $C_2$,
Z est un groupe alkyle en $C_1$ à $C_4$, un halogène, un groupe alkoxy en $C_1$ à $C_4$,
n est un nombre de 0 à 3,
R représente de l'hydrogène (Ia) ou les groupes de formule

$-CO-R^1$ (Ib), $-CO-O-R^2$ (Ic) ou $E^{\oplus}$ (Id)

où

$R^1$ est un groupe alkyle en $C_1$ à $C_{16}$ éventuellement substitué par un halogène, un groupe alcényle en $C_2$ à $C_{16}$, un groupe (alkoxy en $C_1$ à $C_6$)-(alkyle en $C_2$ à $C_6$), (alkylthio en $C_1$ à $C_6$)-(alkyle en $C_2$ à $C_6$), poly(alkoxy en $C_1$ à $C_6$)-(alkyle en $C_2$ à $C_6$) et un groupe cycloalkyle à noyau de 3 à 7 atomes, qui peut être interrompu par 1 ou 2 atomes d'oxygène et/ou de soufre,
un groupe phényle éventuellement substitué par un halogène, un radical nitro, alkyle en $C_1$ à $C_4$, alkoxy en $C_1$ à $C_4$, halogénalkyle en $C_1$ à $C_3$, halogénalkoxy en $C_1$ à $C_3$ ;
un groupe phényl-(alkyle en $C_1$ à $C_4$) éventuellement substitué par un halogène, un radical alkyle en $C_1$ à $C_4$, alkoxy en $C_1$ à $C_4$, halogénalkyle en $C_1$ à $C_3$, halogénalkoxy en $C_1$ à $C_3$,
un groupe phénoxy-(alkyle en $C_1$ à $C_5$) éventuellement substitué par un halogène et un radical alkyle en $C_1$ à $C_4$,

$R^2$ est un groupe alkyle en $C_1$ à $C_{16}$ éventuellement substitué par un halogène, un groupe alcényle en $C_2$ à $C_{16}$, (alkoxy en $C_1$ à $C_{16}$)-(alkyle en $C_2$ à $C_6$), poly(alkoxy en $C_1$ à $C_6$)-(alkyle en $C_2$ à $C_6$), un groupe phényle éventuellement substitué par un halogène, un radical nitro, alkyle en $C_1$ à $C_4$, alkoxy en $C_1$ à $C_3$, halogénalkyle en $C_1$ à $C_3$,

A représente de l'hydrogène, un groupe alkyle en $C_1$ à $C_{10}$ linéaire ou ramifié éventuellement substitué par un halogène, un groupe alcényle en $C_3$ à $C_6$, alcynyle en $C_3$ à $C_6$, (alkoxy en $C_1$ à $C_8$)-(alkyle en $C_2$ à $C_6$), poly (alkoxy en $C_1$ à $C_6$)-(alkyle en $C_2$ à $C_6$), (alkylthio en $C_1$ à $C_8$)-(alkyle en $C_2$ à $C_6$), cycloalkyle à noyau de 3 à 7 atomes, qui peut être interrompu par 1 ou 2 atomes d'oxygène et/ou de soufre,

B est de l'hydrogène, un groupe alkyle en $C_1$ à $C_{10}$ linéaire ou ramifié, un groupe (alkoxy en $C_1$ à $C_6$)-alkyle, ou bien

A et Bforment conjointement avec l'atome de carbone auquel ils sont liés un noyau de 3 à 7 chaînons et

$E^{\oplus}$ représente un équivalent d'ion métallique ou un ion ammonium,

ainsi que les formes énantiomères pures de composés de formule (I).

**3.** Dérivés de 3-arylpyrrolidine-2,4-diones de formule (I) suivant la revendication 1, dans laquelle

X représente les groupes méthyle, éthyle, propyle, isopropyle, du fluor, du chlore, du brome, des groupes méthoxy et éthoxy,

Y représente de l'hydrogène, les groupes méthyle, éthyle, propyle, isopropyle, butyle, isobutyle, tertiobutyle, du fluor, du chlore, du brome, les groupes méthoxy, éthoxy et trifluorométhyle,

Z représente les groupes méthyle, éthyle, isopropyle, butyle, isobutyle, tertiobutyle, du fluor, du chlore, du brome, les groupes méthoxy et éthoxy,

n est un nombre de 0 à 3,

R représente de l'hydrogène (Ia) ou les groupes de formule

$-CO-R^1$ (Ib), $-CO-O-R^2$ (Ic) ou $E^{\oplus}$ (Id)

dans laquelle

$R^1$ est un groupe alkyle en $C_1$ à $C_{14}$ éventuellement substitué par du fluor ou du chlore, un groupe alcényle en $C_2$ à $C_{14}$, (alkoxy en $C_1$ à $C_4$)-(alkyle en $C_2$ à $C_6$), (alkylthio en $C_1$ à $C_4$)-(alkyle en $C_2$ à $C_6$), poly(alkoxy en $C_1$ à $C_4$)-(alkyle en $C_2$ à $C_4$) et cycloalkyle à noyau de 3 à 6 atomes, qui peut être interrompu par 1 ou 2 atomes d'oxygène et/ou de soufre,
un groupe phényle éventuellement substitué par du fluor, du chlore, du brome, un radical méthyle, éthyle, propyle, isopropyle, méthoxy, éthoxy, trifluorométhyle, trifluorométhoxy, nitro,
un groupe phényl-(alkyle en $C_1$ à $C_3$) éventuellement substitué par du fluor, du chlore, du brome, un radical méthyle, éthyle, propyle, isopropyle, méthoxy, éthoxy, trifluorométhyle, trifluorométhoxy,
un groupe phénoxy-(alkyle en $C_1$ à $C_4$) éventuellement substitué par du fluor, du chlore, un radical méthyle, éthyle,

$R^2$ est un groupe alkyle en $C_1$ à $C_{14}$ éventuellement substitué par du fluor ou du chlore, un groupe

alcényle en $C_2$ à $C_{14}$, (alkoxy en $C_1$ à $C_4$)-(alkyle en $C_2$ à $C_6$), poly(alkoxy en $C_1$ à $C_4$)-(alkyle en $C_2$ à $C_6$),
ou bien
un groupe phényle éventuellement substitué par du fluor, du chlore, un radical nitro, méthyle, éthyle, propyle, isopropyle, méthoxy, éthoxy, trifluorométhyle,

A représente de l'hydrogène, un groupe alkyle en $C_1$ à $C_8$ linéaire ou ramifié éventuellement substitué par un halogène, un groupe alcényle en $C_3$ ou $C_4$, alcynyle en $C_3$ ou $C_4$, (alkoxy en $C_1$ à $C_6$)-(alkyle en $C_2$ à $C_4$), poly(alkoxy en $C_1$ à $C_4$)-(alkyle en $C_2$ à $C_4$), (alkylthio en $C_1$ à $C_6$)-(alkyle en $C_2$ à $C_4$), cycloalkyle à noyau de 3 à 6 atomes, qui peut être interrompu par 1 ou 2 atomes d'oxygène et/ou de soufre,
B est de l'hydrogène, un groupe alkyle linéaire ou ramifié en $C_1$ à $C_8$, un groupe (alkoxy en $C_1$ à $C_4$)-alkyle, ou bien
A et Bforment, conjointement avec l'atome de carbone auquel ils sont liés, un noyau de 3 à 6 chaînons, et
$E^{\oplus}$ représente un équivalent d'ion métallique ou un ion ammonium,

ainsi que les formes énantiomères pures de composés de formule (I).

**4.** Procédé de production de dérivés de 3-arylpyrrolidine-2,4-diones de formule (I) suivant la revendication 1, caractérisé en ce que
pour l'obtention de 3-arylpyrrolidine-2,4-diones ou de leurs énols de formule (Ia)

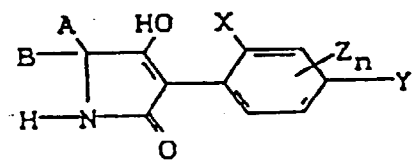

(Ia)

dans laquelle A, B, C, X, Y, Z et n ont la définition indiquée dans la revendication 1,
(A)
on effectue la condensation intramoléculaire d'un ester de N-acylamino-acide de formule (II)

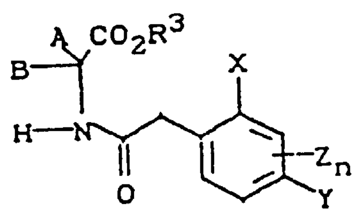

(II)

dans laquelle
A, B, X, Y, Z et n ont la définition indiquée ci-dessus,
et
$R^3$ est un groupe alkyle,
en présence d'un diluant et en présence d'une base,
(B)
ou bien pour obtenir des composés de formule (Ib)

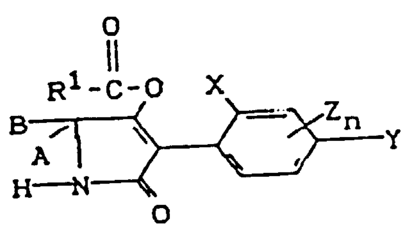

(Ib)

dans laquelle

A, B, X, Y, Z, $R^1$ et n ont la définition indiquée dans la revendication 1,

on fait réagir des composés de formule (Ia)

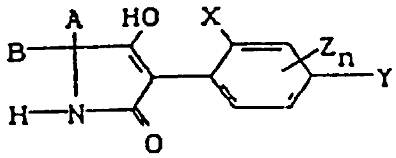

(Ia)

dans laquelle

A, B, X, Y, Z et n ont la définition indiquée ci-dessus,

α) avec des halogénures d'acides de formule générale (III)

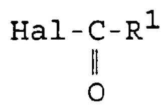

(III)

dans laquelle

$R^1$ a la définition indiquée ci-dessus,

et

Hal représente un halogène, en particulier le chlore et le brome,

le cas échéant en présence d'un diluant et en la présence éventuelle d'un accepteur d'acide,

ou bien

β) avec des anhydrides d'acides carboxyliques de formule générale (IV)

$$R^1\text{-CO-O-CO-}R^1 \qquad \text{(IV)}$$

dans laquelle

$R^1$ a la définition indiquée ci-dessus,

le cas échéant en présence d'un diluant et en la présence éventuelle d'un accepteur d'acide,

(C)

ou bien, pour l'obtention de composés de formule (Ic)

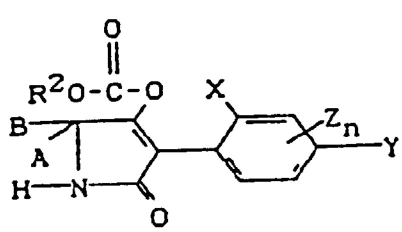

(Ic)

dans laquelle

A, B, C, X, Y, Z, $R^2$ et n ont la définition indiquée dans la revendication 1,

on fait réagir des composés de formule (Ia)

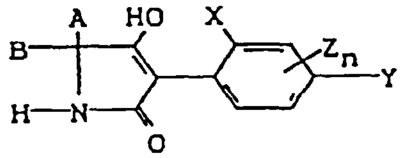 (Ia)

dans laquelle

A, B, X, Y, Z et n ont la définition indiquée ci-dessus,

avec des esters d'acide chloroformique de formule générale (V)

$R^2$-O-CO-Cl (V)

dans laquelle

$R^2$ a la définition indiquée ci-dessus,

le cas échéant en présence d'un diluant et en la présence éventuelle d'un accepteur d'acide,

(D)

ou bien pour l'obtention de composés de formule (Id)

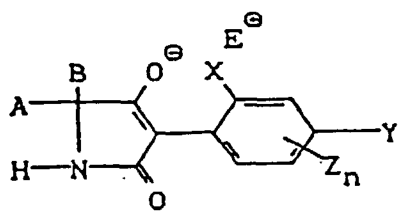 (Id)

dans laquelle X, Y, Z, A, B et n ont la définition indiquée dans la revendication 1,

on fait réagir des composés de formule (Ia)

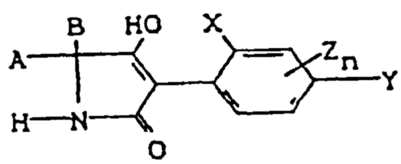 (Ia)

dans laquelle X, Y, Z, A, B et n ont la définition indiquée ci-dessus,

avec des hydroxydes métalliques ou des amines de formules générales (VI) et (VII)

$Me_sOH_t$ (VI) 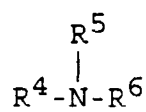 (VII)

dans lesquelles

| | |
|---|---|
| Me | est un ion de métal monovalent ou divalent, |
| s et t | représentent les nombres 1 et 2 et |
| $R^4$, $R^5$ et $R^6$ | représentent, indépendamment les uns des autres, de l'hydrogène et un groupe alkyle, |

le cas échéant en présence d'un diluant.

5. Compositions insecticides, acaricides et herbicides, caractérisées par une teneur en au moins un dérivé de 3-aryl-pyrrolidine-2,4-dione de formule (I).

6. Procédé pour combattre des insectes et/ou des acariens et/ou des mauvaises herbes, caractérisé en ce qu'on fait agir sur les insectes et/ou sur les acariens et/ou sur les mauvaises herbes et/ou sur leur milieu des dérivés de 3-arylpyrrolidine-2,4-diones de formule (I).

7. Utilisation de dérivés de 3-arylpyrrolidine-2,4-diones de formule (I) pour combattre des insectes et/ou des acariens et/ou des mauvaises herbes.

8. Procédé de préparation de compositions insecticides et/ou acaricides et/ou herbicides, caractérisé en ce qu'on mélange des dérivés de 3-arylpyrrolidine-2,4-diones de formule (I) avec des diluants et/ou des agents tensio-actifs.